# EUROPEAN PATENT APPLICATION

(11) **EP 4 850 761 A1**
(43) Date of publication of application: **09.12.2026**
(21) Application number: 25181311.9
(22) Date of filing: 06.06.2025
(51) Int. Cl.: A61P 29/00, C07D 233/64, C07D 233/84, C07D 233/90, C07D 401/12, C07D 401/14, C07D 403/06, C07D 403/12, C07D 471/04, C07D 487/04, A61K 31/4178, A61K 31/4439, A61K 31/55, A61K 31/437, A61K 31/496

(54) **IMIDAZOLES AS INHIBITORS OF NAV1.8**

(71) Applicant: Grünenthal GmbH, 52078 Aachen (DE)
(72) Inventor: KRÜGER, Sebastian, 52078 Aachen (DE); NORDHOFF, Sonja, 52078 Aachen (DE); MARIGO, Mauro, 52078 Aachen (DE); DIALER, Clemens, 52078 Aachen (DE); WAGENER, Markus, 52078 Aachen (DE); MÜLBAIER, Marcel, 52078 Aachen (DE)
(74) Representative: Grünenthal GmbH

(57) **Abstract**

The invention relates to compounds according to general formula (I) 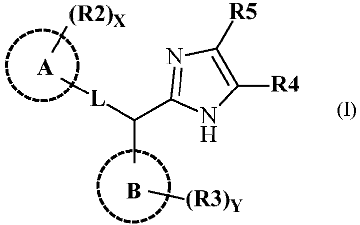 which act as inhibitors of Naᵥ1.8 and can be used in the treatment of pain.

## Description

The invention relates to compounds according to general formula (I) 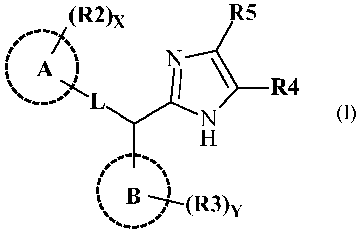 which act as inhibitors of Na_{V}1.8 and can be used in the treatment of pain.

Normal pain sensation (nociception) serves primarily as a survival mechanism, the body's way of self-protection, alerting against (further) tissue damage and disease from noxious stimuli. For instance, acute pain can arise when the external environment (temperature, pressure, chemicals) activates modality specific receptors (no-ciceptors) and ion channels within the skin. The peripheral terminals of pain-signaling neurons - whose cell bodies are found in the dorsal root ganglia [DRG] and trigeminal ganglia [TG] - convert the external stimuli into electrochemical generator potentials. Specific voltage-gated sodium channels (Na_{V}s) integrate and amplify these generator signals until the threshold for an action potential [AP] is reached. Thus, what starts as a noxious stimuli in the periphery eventually leads to action potential firing that travels towards the central nervous system, synapsing first onto neurons in the spinal cord and then towards the brain. Na_{V}s also function to support propagation of action potentials to the central terminals within the spinal cord. At the end of its travels along the somatosensory pathway, the action potential signal is interpreted as pain by the brain (Lumpkin and Caterina, Nature (2007), Vol.445 pp 858-865; and Crawford and Caterina Toxicologic Pathology (2020) 48(1)-174; Goodwin G and McMahon S.B. Nature Reviews Neuroscience (2021) Vol 22 pp 263-274; Bennett D.L. et al. Physiol Rev 99 (2019) Vol 99 pp 1079-1151).

Abnormal persistent neuropathic pain arises as a consequence of a lesion or disease of this somatosensory pathway. In response to nerve injury or inflammation, abnormal changes in ion channel expression can cause hyper-excitability of pain-signaling neurons and their nerves/axons, thus resulting in pathological pain.

The voltage-gated Na_{V}1.8 sodium channel is a therapeutic target for analgesia because of its restricted expression profile (almost exclusive to peripheral sensory tissues), its placement along the pain pathway (free nerve endings, sciatic nerve, and DRG), prominent physiological role in pain signaling (supports upstroke of AP and facilitates repetitive AP firing), and supporting genetic/pharmaco-phenotypic evidence (human/animal studies showing changes in Na_{V}1.8 function cause parallel changes in pain sensitivity).

Regarding its expression profile along the pain pathway, because Na_{V}1.8 was first found predominantly in peripheral sensory neurons of the dorsal root ganglia (DRG) and trigeminal ganglia (TG), it was originally termed SNS (sensory neuron specific) (Akopian A.N. et al Nature (1996) Vol 379 pp 257-261) or PN3 (peripheral nerve 3) (Sangameswaran L. et al J. Biol. Chem. (1996) Vol 271 pp 5953-5956). As well, Na_{V}1.8 is localized at free nerve endings, where pain signaling is initiated in the skin (Persson A.K. et al Mol. Pain. (2010) 6:84) and is diffusely localized along the entire length of non-myelinated axons of sciatic nerve (Rush A.M. et al. Eur.J.Neurosci (2005) Vol 22 pp 39-49).

In contrast, Na_{V}1.8 has minimal expression in nonneuronal tissue, such as heart and skeletal muscle, and in the CNS, including brain and spinal cord (9, 10, 338, 406) (Akopian A.N. op. cit.; Akopian A.N. et al. Nat. Neurosci (1999) Vol 2 pp 541-548; Novakovic S.D. et al. J. Neurosci. (1998) Vol 18, pp 2174-2187; and Sagameswaran L. op. cit.).

Regarding its physiological role, Na_{V}1.8 contributes the majority of the inward current during the rising phase of an all-or-none action potential in nociceptive sensory neurons (Blair N.T. et al. J. Neurosci. (2003) Vol 23 pp 10338-10350 and Renganathan M et al. J. Neurophysiol (2001) Vol 86 pp 629-640) - and also contributes most of the current in subsequent spikes during repetitive firing in DRG neurons (Choi J.S. J. Neurophysiol (2011) Vol 106 pp 3173-3184; and Tan Z.Y. et al. J. Neurosci. (2014) Vol 34 pp 7190-7197).

Regarding genetic and pharmacology studies, gain-of-function mutations in Na_{V}1.8 were found in patients with chronic neuropathic pain such as small fiber neuropathy (Faber C.G. et al. (2012) Ann. Neurol Vol 71 pp 26-39; Han C et al. J. Neurol Neurosurg Psychiatry (2014) Vol 85 pp 499-505; and, Kist A.M. et al. PLoS One (2016) Vol 11 e0161789); Eijkenboom I. et al J. Neurol Neurosurg Psychiatry (2019) 90 (3) pp 342-352); loss-of-function (gene knockout) studies in mice reduced pain sensitivity, notably in nociception (Laird J.M. et al. J. Neurosci

(2002) J. Neurosci Vol 22 pp 8352-8356; Jarvis M.F. et al Proc Natl Acad Sci USA (2007) Vol 104 pp 8520-8525; Joshi S.K. et al Pain (2006) Vol 123 pp 75-82) and in neuropathic models (Roza C. et al J Physiol (2003) Vol 550 pp 921-926); Na_{V}1.8-selective small molecule inhibitors reduced pain in rodents, specifically in inflammatory and neuropathic models (Jarvis et al. op. cit.; Kort M.E. et al Bioorg Med Chem Lett (2010) Vol 20 pp 6812-6815; Scanio M.J. et al Bioorg Med Chem (2010) Vol 18 pp 7816-7825; Payne C.E. et al Br J Pharmacol (2015) Vol 172 pp 2654-2670).

Currently, non-selective Na_{V} channel inhibitors are used to treat epilepsy, cardiac arrhythmia, and chronic pain (Hille, B. J. Gen. Physiol. (1977) Vol. 69 pp. 497-515; Hille. B, Ion Channels of Excitable Membranes (1992) pp. 391-421; Sunderland, Mass., Sinauer Associates, Inc. 3rd ed.; Hondeghem L.M. and Katzung B.G. Annu. Rev. Pharmacol. Toxicol. (1984) Vol. 24. Pp. 387-423; Catterall W.A. Trends Pharmacol. Sci. (1987) Vol. 8 pp.57-65) - however, all of these analgesics have limited efficacy owing to dose-limiting adverse side-effects related to inhibiting Na_{V}1.1/Na_{V}1.2/1.6 (seizure liability), inhibiting Na_{V}1.4 (muscle weakness/paralysis), inhibiting Na_{V}1.5 (arrhythmia risk).

There is a need to develop a Na_{V}1.8-selective small molecule inhibitor as an effective and safe analgesic.

It was an object of the invention to provide novel compounds which are inhibitors, preferably selective inhibitors, of Na_{V}1.8, and which preferably have advantages over the compounds of the prior art. The novel compounds should in particular be suitable for use in the treatment of pain.

This object has been achieved by the subject-matter of the patent claims.

It was surprisingly found that the compounds according to the invention are highly potent and selective inhibitors of the Na_{V}1.8 channel. Further it was surprisingly found that the compounds according to the invention have advantageous properties compared to the Na_{V}1.8 inhibitors of the prior art.

A first aspect of the invention relates to a compound according to general formula (I) 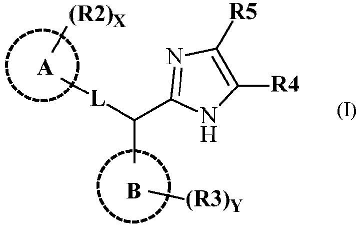 wherein
- **A** and **B**: independently from one another represent phenyl, 5 to 10-membered heteroaryl, C₃₋₇-cycloalkyl, or 4- to 10-membered heterocycloalkyl;
- **R2** and **R3**: independently from one another represent F, Cl, CN, C₁₋₄-alkyl, C₃₋₇-cycloalkyl, OH, OC₁₋₄-alkyl, NH₂, NHC₁₋₄-alkyl or N(C₁₋₄-alkyl)₂;
- **X** and **Y**: independently from one another represent 0, 1, 2, 3 or 4;
- **L**: represents bond, O, NH or C₁₋₃-alkylene;
- **R4**: represents H, Cl, CN, C₁₋₄-alkyl or C₃₋₇-cycloalkyl;
- **R5**: represents S(O)₂N**R6R7,** C(O)N**R6R7**, C(O)O**R6**, C(O)OH, C(O)NH₂, C(O)C₁₋₄-alkyl, S(O)₂-(4- to 7-membered heterocycloalkyl) or S(O)₂C₁₋₃-alkylene-(4- to 7-membered heterocycloalkyl);
- **R6**: represents C₁₋₄-alkyl, C₃₋₇-cycloalkyl, 4- to 7-membered heterocycloalkyl or C₁₋₃-alkylene-(4- to 7-membered heterocycloalkyl); and
- **R7**: represents H or C₁₋₄-alkyl; or
- **R4** and **R6**: together with the atoms to which they are attached form 4- to 7-membered heterocycloalkyl or 5 to 10-membered heteroaryl; or
- **R6** and **R7**: together with the nitrogen atom to which they are attached form 4- to 7-membered heterocycloalkyl;
with the proviso that when **R5** represents S(O)₂N**R6R7**, **R4** represents C₁₋₄-alkyl and/or **L** represents O or NH;
wherein C₁₋₄-alkyl and C₁₋₃-alkylene in each case independently from one another is linear or branched, saturated or unsaturated;
wherein C₁₋₄-alkyl, C₁₋₃-alkylene, C₃₋₇-cycloalkyl, 4- to 7-membered heterocycloalkyl and 4- to 10-membered heterocycloalkyl in each case independently from one another are unsubstituted or mono- or polysubstituted with one or more substituents selected from F, Cl, CN, C₁₋₄-alkyl, C₁₋₃-alkylene-NH₂, CH₂F, CHF₂, CF₃, C(O)C₁₋₄-alkyl, C(O)OH, C(O)OC₁₋₄-alkyl, C(O)NH₂, C(O)NHC₁₋₄-alkyl, C(O)N(C₁₋₄-alkyl)₂, OH, =O, OC₁₋₄-alkyl, OCH₂F, OCHF₂, OCF₃, NH₂, NHC₁₋₄-alkyl, N(C₁₋₄-alkyl)₂ and NHC(O)C₁₋₄-alkyl;
wherein phenyl and 5 to 10-membered heteroaryl in each case independently from one another are unsubstituted or mono- or polysubstituted with one or more substituents selected from F, Cl, CN, C₁₋₄-alkyl, CH₂F, CHF₂, CF₃, OH, OCH₂F, OCHF₂, OCF₃, OC₁₋₄-alkyl, NH₂, NHC₁₋₄-alkyl and N(C₁₋₄-alkyl)₂;
in the form of the free compound or a physiologically acceptable salt thereof.

In a preferred embodiment, the compound according to the invention is present in form of the free compound. For the purpose of specification, "free compound" preferably means that the compound according to the invention is not present in form of a salt. Methods to determine whether a chemical substance is present as the free compound or as a salt are known to the skilled artisan such as ¹⁴N or ¹⁵N solid state NMR, x-ray diffraction, x-ray powder diffraction, IR, Raman, XPS. ¹H-NMR recorded in solution may also be used to consider the presence of protonation.

In another preferred embodiment, the compound according to the invention is present in form of a physiologically acceptable salt. For the purposes of this specification, the term "physiologically acceptable salt" preferably refers to a salt obtained from a compound according to the invention and a physiologically acceptable acid or base.

According to the invention, the compound according to the invention may be present in any possible form including solvates, cocrystals and polymorphs. For the purposes of this specification, the term "solvate" preferably refers to an adduct of (i) a compound according to the invention and/or a physiologically acceptable salt thereof with (ii) distinct molecular equivalents of one or more solvents.

Further, the compound according to the invention may be present in form of the racemate, enantiomers, diastereomers, tautomers or any mixtures thereof. The person skilled in the art knows that imidazoles show annular tautomerism.

The compounds according to the invention may have one or more stereocenter. The person skilled in art knows by looking at a chemical structure whether the depicted compound has one or more stereocenters or not.

For some compounds according to the invention that have one or more stereocenters and which chemical structures are disclosed in the examples of the present application, the chemical structure includes bold bonds and/or hashed bonds to indicate the relative structural orientation of those substituents connected by the bold bonds and/or hashed bonds to the superior structure. If the bold bonds and/or hashed bonds are depicted in form of a wedge, the absolute stereochemical configuration of the compound is known and thereby indicated. If the bold bonds and/or hashed bonds are depicted as a straight bond (i.e. no wedge), the absolute stereochemical configuration of the compound has not been determined. In that case, the bold bonds and/or hashed bonds merely serve to indicate that this particular compound is present as one enantiomer or one diastereomer (e.g. cis-diastereomer (i.e. mixture of two cis-enantiomers) or trans-diastereomer (i.e. mixture of two trans-enantiomers)). All compounds according to the invention that have one or more stereocenters but which chemical structures disclosed in the examples of the present application do not include bold bonds and/or hashed bonds, are present as a mixture of the respective stereoisomers.

The invention also includes isotopic isomers of a compound of the invention, wherein at least one atom of the compound is replaced by an isotope of the respective atom which is different from the naturally predominantly occurring isotope, as well as any mixtures of isotopic isomers of such a compound. Preferred isotopes are ²H (deuterium), ³H (tritium), ¹³C and ¹⁴C. Isotopic isomers of a compound of the invention can generally be prepared by conventional procedures known to a person skilled in the art.

According to the invention, the term "C₁₋₄-alkyl" preferably means acyclic and preferably saturated hydrocarbon residues, which can be linear (i.e. unbranched) or branched and which can be unsubstituted or mono- or polysubstituted (e.g. di- or trisubstituted), and which contain 1 to 4 (i.e. 1, 2, 3 or 4) carbon atoms. Preferably, C₁₋₄-alkyl is saturated. Preferred C₁₋₄-alkyl groups are selected from the group consisting of methyl, ethyl, n-propyl, 2-propyl, n-butyl, isobutyl, sec-butyl and tert-butyl.

Still further according to the invention, the term "C₃₋₇-cycloalkyl" preferably means monocyclic or polycyclic, preferably monocyclic or bicyclic, aliphatic hydrocarbons containing 3 to 7 (i.e. 3, 4, 5, 6 or 7) carbon atoms, wherein the hydrocarbons in each case can be saturated or unsaturated (but not aromatic), unsubstituted or mono- or polysubstituted.

For the purpose of this specification, "polycyclic" and "bicyclic" shall preferably mean fused, bridged or spiro cyclic systems.

Preferably, C₃₋₇-cycloalkyl is saturated. C₃₋₇-cycloalkyl can be bound to the respective superordinate general structure via any desired and possible ring member of the cycloalkyl group. C₃₋₇-cycloalkyl can also be fused with further saturated or (partially) unsaturated heterocycloalkyl, aromatic or heteroaromatic ring systems, preferably aromatic ring system, which can in turn be unsubstituted or mono- or polysubstituted, thereby being part of a bi- or polycyclic system having up to 14 ring members. Accordingly, the term "C₃₋₇-cycloalkyl" preferably includes monocyclic or polycyclic, preferably monocyclic or bicyclic, aliphatic hydrocarbons containing 3 to 7 carbon atoms; which are fused with a phenyl moiety. Preferred C₃₋₇-cycloalkyl groups are selected from the group consisting of cyclopropyl, cyclobutyl, cyclopentyl, cyclohexyl, cycloheptyl, spiro[2.2]pentyl, spiro[2.3]hexyl, spiro[3.3]heptyl, bicyclo[1.1.0]butyl, bicyclo[2.1.0]pentyl, bicyclo[2.1.1]hexyl, bicyclo[3.1.1]heptyl, bicyclo[2.2.1]heptyl, bicyclo[3.1.0]hexyl, bicyclo[3.2.0]heptyl, and bicyclo[4.1.0]heptyl.

Further according to the present invention, the term "C₁₋₃-alkylene" relates to linear or branched, preferably linear, and preferably saturated aliphatic residues which are preferably selected from the group consisting of methylene (-CH₂-), ethylene (-CH₂CH₂-), propylene (-CH₂CH₂CH₂- or -C(CH₃)₂-), more preferably methylene (-CH₂-) and ethylene (-CH₂CH₂-).

According to the invention, C₁₋₄-alkyl and C₁₋₃-alkylene in each case independently from one another is linear or branched, saturated or unsaturated. In a preferred embodiment, C₁₋₄-alkyl and C₁₋₃-alkylene in each case independently from one another is linear or branched, and saturated.

According to the present invention, the terms "4 to 7-membered heterocycloalkyl" and "4 to 10-membered heterocycloalkyl" preferably mean monocyclic or polycyclic, preferably monocyclic or bicyclic, heterocycloaliphatic saturated or unsaturated (but not aromatic) residues having 4 to 7 (i.e. 4, 5, 6 or 7) or 4 to 10 (i.e. 4, 5, 6, 7, 8, 9 or 10) ring memberswherein in each case at least one, if appropriate also two or three carbon atoms are replaced by a heteroatom or a heteroatom group each selected independently of one another from the group consisting of O, S, S(=O), S(=O)₂, N, NH and N(C₁₋₄-alkyl) such as N(CH₃), wherein the carbon atoms of the ring can be unsubstituted or mono- or polysubstituted. Preferably, 4 to 7-membered heterocycloalkyl and 4 to 10-membered heterocycloalkyl are saturated. The 4 to 7-membered heterocycloalkyl and the 4 to 10-membered heterocycloalkyl groups can also be fused with further saturated or (partially) unsaturated cycloalkyl, aromatic or heteroaromatic ring systems, preferably aromatic ring system, which can in turn be unsubstituted or mono- or polysubstituted, if not indicated otherwise, thereby being being part of a bi- or polycyclic system having up to 14 ring members.. In a preferred embodiment, 4 to 7-membered heterocycloalkyl and 4 to 10-membered heterocycloalkyl are not fused with further ring systems. Still more preferably, 4 to 7-membered heterocycloalkyl and 4 to 10-membered heterocycloalkyl are not fused with further ring systems and are saturated. In another preferred embodiment, 4 to 7-membered heterocycloalkyl and 4 to 10-membered heterocycloalkyl are condensed with further ring systems, preferably phenyl. The 4 to 7-membered heterocycloalkyl and 4 to 10-membered heterocycloalkyl group can be bound to the superordinate general structure via any desired and possible ring member of the heterocycloaliphatic residue if not indicated otherwise. In a preferred embodiment, 4 to 7-membered heterocycloalkyl and 4 to 10-membered heterocycloalkyl are bound to the superordinate general structure via a carbon atom. In another preferred embodiment, 4 to 7-membered heterocycloalkyl and 4 to 10-membered heterocycloalkyl are bound to the superordinate general structure via a heteroatom, in particular N.

Preferred 4 to 7-membered heterocycloalkyl groups are selected from the group consisting of 1,1-dioxo tetrahydrothiophenyl, 1-oxo thiomorpholinyl, tetrahydropyranyl, oxetanyl, tetrahydrofuranyl, morpholinyl, pyrrolidinyl, pyrrolidinonyl, azetidinyl, piperazinyl, piperazinonyl, piperidinyl, thietanyl, 1,1-dioxothietanyl, 2,6-diazaspiro[3.3]heptyl, 2,5-diazabicyclo[2.2.1]heptyl, 3,6-diazabicyclo[3.3.1]heptyl, azepanyl, dioxepanyl, oxazepanyl, diazepanyl, thiazolidinyl, tetrahydrothiophenyl, tetrahydropyridinyl, thiomorpholinyl, 4-methylpiperazinyl, morpholinonyl, dithiolanyl, dihydropyrrolyl, dioxanyl, dioxolanyl, dihydropyridinyl, dihydrofuranyl, dihydroisox-azolyl, dihydrooxazolyl, imidazolidinyl, isoxazolidinyl, oxazolidinyl, pyrazolidinyl, and cromanyl.

Preferred 4 to 10-membered heterocycloalkyl groups are selected from the group consisting of tetrahydro-2H-thiopyranyl 1,1-dioxide, 1-oxo thiomorpholinyl, tetrahydropyranyl, oxetanyl, tetrahydrofuranyl, morpholinyl, pyrrolidinyl, pyrrolidinonyl, azetidinyl, piperazinyl, piperazinonyl, piperidinyl, thietanyl, 1,1-dioxothietanyl, 2,6-diazaspiro[3.3]heptyl, 2,5-diazabicyclo[2.2.1]heptyl, 3,6-diazabicyclo[3.3.1]heptyl, cromanyl, thiepanyl 1,1-dioxide, 1,2-thiazepanyl 1,1-dioxide, 2,5,6,7-tetrahydro-1,2-thiazepinyl 1,1-dioxide, 5,6-dihydro-2H-1,2-thiazinyl 1,1-dioxide, 1,1-dioxo tetrahydrothiophenyl, 2-oxaspiro[3.4]octyl, 5-oxaspiro[2.4]heptyl, 2-oxabicyclo[2.1.1]hexyl, 1,1-dioxo thietanyl, 2,3-dihydro-[1,4]dioxino[2,3-b]pyridyl, tetrahydro-1,8-naphthyridinyl, azepanyl, dioxepanyl, oxazepanyl, diazepanyl, thiazolidinyl, tetrahydrothiophenyl, tetrahydropyridinyl, thiomorpholinyl, 4-methylpiperazinyl, morpholinonyl, dithiolanyl, dihydropyrrolyl, dioxanyl, dioxolanyl, dihydropyridinyl, dihydrofuranyl, di-hydroisoxazolyl, dihydrooxazolyl, imidazolidinyl, isoxazolidinyl, oxazolidinyl, pyrazolidinyl, 5,7-dihydro-furo[3,4-b]pyridinyl, and 5,7-dihydrofuro[3,4-d]pyrimidinyl; more preferably tetrahydro-2H-thiopyranyl 1,1-dioxide, 1-oxo thiomorpholinyl, tetrahydropyranyl, oxetanyl, tetrahydrofuranyl, pyrrolidinyl, pyrrolidinonyl, azetidinyl, piperazinyl, piperazinonyl, piperidinyl, 1,1-dioxothietanyl, 2,6-diazaspiro[3.3]heptyl, 2,5-diazabicy-clo[2.2.1]heptyl, 3,6-diazabicyclo[3.3.1]heptyl, cromanyl, thiepanyl 1,1-dioxide, 1,2-thiazepanyl 1,1-dioxide, 2,5,6,7-tetrahydro-1,2-thiazepinyl 1,1-dioxide and 5,6-dihydro-2H-1,2-thiazinyl 1,1-dioxide.

According to the present invention, the term "5 to 10-membered heteroaryl" preferably means a 5, 6, 7, 8, 9 or 10-membered monocyclic or bicyclic aromatic residue, respectively, which contains at least 1, if appropriate also 2, 3, 4 or 5 heteroatoms, wherein the heteroatoms are each selected independently of one another from the group S, N and O and the heteroaryl residue can be unsubstituted or mono- or polysubstituted, if not indicated otherwise. In the case of substitution on the heteroaryl, the substituents can be the same or different and be in any desired and possible position of the heteroaryl. The binding to the superordinate general structure can be carried out via any desired and possible ring member of the heteroaryl residue if not indicated otherwise. Preferably, the 5 to 10-membered heteroaryl is bound to the supraordinate general structure via a carbon atom of the heterocycle. The heteroaryl can also be fused with further saturated or (partially) unsaturated cycloalkyl, or heterocycloalkyl ring systems, which can in turn be unsubstituted or mono- or polysubstituted, thereby being part of a bi- or polycyclic system having up to 14 ring members. In a preferred embodiment, the 5 to 10-membered heteroaryl is not fused with further saturated or (partially) unsaturated cycloalkyl, or heterocycloalkyl ring systems. Preferably, the 5 to 10-membered heteroaryl is selected from the group consisting of pyridyl (i.e. 2-pyridyl, 3-pyridyl, 4-pyridyl), pyrimidinyl, pyridazinyl, pyrazinyl, pyrrolyl, imidazolyl, pyrazolyl, oxazolyl, isoxazolyl, thiazolyl, isothiazolyl, furanyl, thienyl (thiophenyl), triazolyl, thiadiazolyl, 4,5,6,7-tetrahydro-2H-indazolyl, 2,4,5,6-tetrahydrocyclo-penta[c]pyrazolyl, benzofuranyl, benzoimidazolyl, benzothienyl, benzothiadiazolyl, benzothiazolyl, benzotriazolyl, benzooxazolyl, benzooxadiazolyl, quinazolinyl, quinoxalinyl, carbazolyl, quinolinyl, dibenzofuranyl, diben-zothienyl, imidazothiazolyl, indazolyl, indolizinyl, indolyl, isoquinolinyl, naphthyridinyl, oxazolyl, oxadiazolyl, phenazinyl, phenothiazinyl, phthalazinyl, purinyl, phenazinyl, tetrazolyl and triazinyl. Particularly preferred is pyridyl (i.e. 2-pyridyl, 3-pyridyl, 4-pyridyl).

In connection with the terms "C₁₋₄-alkyl", "C₁₋₃-alkylene", "C₃₋₇-cycloalkyl", "4- to 7- membered heterocycloalkyl" and "4- to 10- membered heterocycloalkyl" the term "substituted" refers in the sense of the invention, with respect to the corresponding residues or groups, to the single substitution (monosubstitution) or multiple substitution (poly substitution), e.g. disubstitution, trisubstitution or tetrasubstitution; more preferably to monosubstitution, disubstitution or trisubstitution; of one or more hydrogen atoms each independently of one another by at least one substituent. In case of a multiple substitution, i.e. in case of polysubstituted residues, such as di- or trisubstituted residues, these residues may be polysubstituted either on different or on the same atoms, for example trisubstituted on the same carbon atom, as in the case of CF₃, CH₂CF₃ or disubstituted as in the case of 1,1-difluo-rocyclopropyl, or at various points, as in the case of 1-chloro-3-fluorocyclopropyl. The multiple substitution can be carried out using the same or using different substituents.

If a residue occurs multiply within a molecule, then this residue can have respectively different meanings for various substituents.

According to the invention, C₁₋₄-alkyl, C₁₋₃-alkylene, C₃₋₇-cycloalkyl, 4- to 7- membered heterocycloalkyl and 4- to 10- membered heterocycloalkyl in each case independently from one another are unsubstituted or mono- or polysubstituted with one or more substituents selected from F, Cl, CN, C₁₋₄-alkyl, C₁₋₃-alkylene-NH₂, CH₂F, CHF₂, CF₃, C(O)C₁₋₄-alkyl, C(O)OH, C(O)OC₁₋₄-alkyl, C(O)NH₂, C(O)NHC₁₋₄-alkyl, C(O)N(C₁₋₄-alkyl)₂, OH, =O, OC₁₋₄-alkyl, OCH₂F, OCHF₂, OCF₃, NH₂, NHC₁₋₄-alkyl, N(C₁₋₄-alkyl)₂ and NHC(O)C₁₋₄-alkyl.

Further according to the invention, phenyl and 5 to 10-membered heteroaryl in each case independently from one another are unsubstituted or mono- or polysubstituted with one or more substituents selected from F, Cl, CN, C₁₋₄-alkyl, CH₂F, CHF₂, CF₃, OH, OCH₂F, OCHF₂, OCF₃, OC₁₋₄-alkyl, NH₂, NHC₁₋₄-alkyl and N(C₁₋₄-alkyl)₂.

In a preferred embodiment, **A** represents phenyl, **L** represents bond and **B** represents phenyl.

In a preferred embodiment, **A** represents C₃₋₇-cycloalkyl, **L** represents **O** and **B** represents phenyl.

In a preferred embodiment, **A** represents pyridyl, **L** represents NH and **B** represents phenyl.

In a preferred embodiment, **R5** represents C(O)N**R6R7**, C(O)NH₂, or S(O)₂N**R6R7**.

In a preferred embodiment, **R5** represents C(O)C₁₋₄-alkyl, C(O)NH₂, C(O)OH C(O)OR6, or C(O)N**R6R7**.

In a preferred embodiment, **R5** represents C(O)C₁₋₄-alkyl; preferably C(O)CH₃.

In a preferred embodiment, **R5** represents C(O)NH₂.

In a preferred embodiment, **R5** represents C(O)OH or C(O)OR6.

In a preferred embodiment, **R5** represents C(O)OH.

In a preferred embodiment, **R5** represents C(O)OR6; preferably C(O)OCH₃.

In a preferred embodiment, **R5** represents S(O)₂-(4- to 7-membered heterocycloalkyl) or S(O)₂C₁₋₃-alkylene-(4- to 7-membered heterocycloalkyl); preferably CH₂-azetidinyl.

In a preferred embodiment, **R5** represents C(O)N**R6R7** or S(O)₂N**R6R7**; preferably C(O)NHR6 or S(O)₂NH**R6**.

In a preferred embodiment, **R5** represents S(O)₂N**R6R7** and **R4** represents C₁₋₄-alkyl:

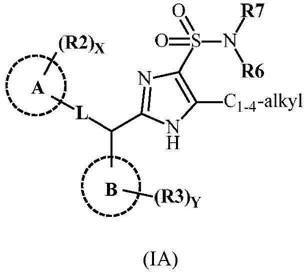

In a preferred embodiment, **R5** represents S(O)₂N**R6R7** and L represents O or NH:

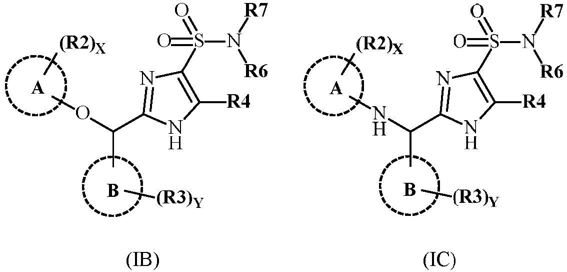

In a preferred embodiment, **R6** represents C₁₋₄-alkyl; preferably CH₃, CH₂C(O)NH₂, CH₂CH₂NH₂, CH₂CH₂NHCH₃, CH₂CH₂N(CH₃)₂, CH₂CH₂CH₂NH₂, CH₂CF₂CH₂NH₂, CH₂CH₂CH₂NHCH₃ or CH₂CH₂CH₂N(CH₃)₂.

In a preferred embodiment, **R6** represents C₃₋₇-cycloalkyl; preferably cyclopropyl or cyclobutyl; in each case unsubstituted or monosubstituted with NH₂;

In a preferred embodiment, **R6** represents 4- to 7-membered heterocycloalkyl; preferably azetidinyl, pyrrolidinyl or piperidinyl; in each case unsubstituted or with 1 or 2 substituents independently from one another selected from F, CH₃, CH₂OH, CH₂OCH₃, NH₂, OH and =O;

In a preferred embodiment, **R6** represents C₁₋₃-alkylene-(4- to 7-membered heterocycloalkyl); preferably -CH₂-azetidinyl or -CH₂-pyrrolidinyl; in each case unsubstituted or monosubstituted with F, CH₃ or CH₂CH₃;

In a preferred embodiment, **R6** and **R7** together with the nitrogen atom to which they are attached form azetidinyl, pyrrolidinyl, morpholinyl or piperazinyl; in each case unsubstituted or substituted with 1 or 2 substituents independently from one another selected from F, CH₂OH, CH₂OCH₃, NH₂, NHCH₃, N(CH₃)₂ and =O;

In a preferred embodiment, **R5** represents C(O)N**R6R7** and wherein **R4** and **R6** together with the nitrogen atom to which they are attached form a ring and mean CH₂CH₂ or CH₂CH₂CH₂.

In a preferred embodiment, **R7** represents H or CH₃.

In preferred embodiments, the compound according to the invention is according to general formula (II), (III) or (IV) 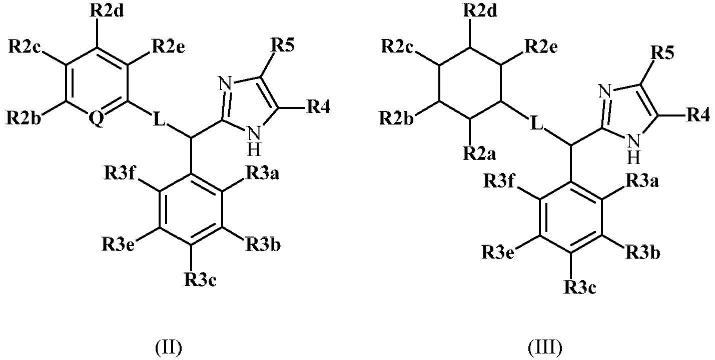 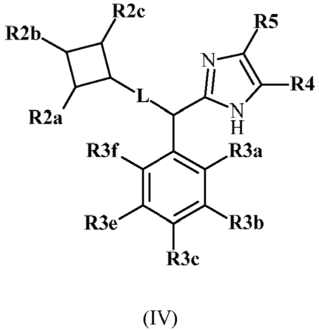 wherein
**L, R4** and **R5** are defined as above;
**Q** represents N or C**R2a**;
**R2a, R2b, R2c, R2d and R2e** independently from one another represent H, F, Cl, CH₃, CH₂F, CHF₂, CF₃ or OCH₃; with the proviso that not more than 4 of **R2a, R2b, R2c, R2d,** and **R2e** are other than H;
**R3a, R3b, R3c, R3d and R3e** independently from one another represent H, F, Cl, CH₃, CH₂F, CHF₂, CF₃ or OCH₃; with the proviso that not more than 4 of **R3a, R3b, R3c, R3d** and **R3e** are other than H.

In preferred embodiments, the compound according to the invention is selected from the group consisting of
1 1-(2-(bis(3-chloro-4-fluorophenyl)methyl)-1H-imidazol-4-yl)ethan-1-one
2 2-(bis(3-chloro-4-fluorophenyl)methyl)-1H-imidazole-4-carboxamide
3 2-(bis(3-chloro-4-fluorophenyl)methyl)-N-methyl-1H-imidazole-4-carboxamide
4 2-(bis(3-chloro-4-fluorophenyl)methyl)-N,N-dimethyl-1H-imidazole-5-carboxamide
5 2-(bis(3-chloro-4-fluorophenyl)methyl)-N-cyclopropyl-1H-imidazole-5-carboxamide
6 (2-(bis(3-chloro-4-fluorophenyl)methyl)-1H-imidazol-5-yl)(pyrrolidin-1-yl)methanone
7 (2-(bis(3-chloro-4-fluorophenyl)methyl)-1H-imidazol-5-yl)(3,3-difluoropyrrolidin-1-yl)methanone
8 (2-(bis(3-chloro-4-fluorophenyl)methyl)-1H-imidazol-5-yl)(morpholino)methanone
9 4-(2-(bis(3-chloro-4-fluorophenyl)methyl)-1H-imidazole-5-carbonyl)piperazin-2-one
10 2-(bis(3-chloro-4-fluorophenyl)methyl)-N-(5-oxopyrrolidin-3-yl)-1H-imidazole-5-carboxamide
11 (2-(bis(3-chloro-4-fluorophenyl)methyl)-1H-imidazol-5-yl)(4-methylpiperazin-1-yl)methanone
12 N-(2-amino-2-oxoethyl)-2-(bis(3-chloro-4-fluorophenyl)methyl)-1H-imidazole-5-carboxamide
13 2-(bis(3-chloro-4-fluorophenyl)methyl)-1,5,6,7-tetrahydro-4H-imidazo[4,5-c]pyridin-4-one
14 2-(bis(3-chloro-4-fluorophenyl)methyl)-5,6,7,8-tetrahydroimidazo[4,5-c]azepin-4(1H)-one
15 2-(bis(3-chloro-4-fluorophenyl)methyl)-1,5-dihydro-4H-imidazo[4,5-c]pyridin-4-one
16 2-(bis(3-chloro-4-fluorophenyl)methyl)-5-methyl-1H-imidazole-4-carboxamide
17 2-((3-chloro-4-fluorophenyl)((5-fluoro-6-methylpyridin-2-yl)amino)methyl)-N-(4-hydroxypyrrolidin-3-yl)-5-methyl-1H-imidazole-4-carboxamide
18 2-((3-chloro-4-fluorophenyl)((5-fluoro-6-methylpyridin-2-yl)amino)methyl)-5-methyl-1H-imidazole-4-carboxylic acid
19 methyl 2-(bis(4-chloro-2-methoxyphenyl)methyl)-5-methyl-1H-imidazole-4-carboxylate
20 2-(bis(4-chloro-2-methoxyphenyl)methyl)-5-methyl-1H-imidazole-4-carboxylic acid
21 2-(bis(4-chloro-2-methoxyphenyl)methyl)-N-(4-hydroxypyrrolidin-3-yl)-5-methyl-1H-imidazole-4-carboxamide
22 (3-aminoazetidin-1-yl)(2-((3-chloro-4-fluorophenyl)((6-(difluoromethyl)-5-fluoropyridin-2-yl)amino)methyl)-5-(methoxymethyl)-1H-imidazol-4-yl)methanone
23 (3-aminoazetidin-1-yl)(2-((3-chloro-4-fluorophenyl)((5-fluoro-6-methylpyridin-2-yl)amino)methyl)-5-(methoxymethyl)-1H-imidazol-4-yl)methanone
24 (3-aminoazetidin-1-yl)(2-((3-chlorophenyl)((6-(difluoromethyl)-5-fluoropyridin-2-yl)amino)methyl)-5-(methoxymethyl)-1H-imidazol-4-yl)methanone
25 2-((3-chlorophenyl)((6-(difluoromethyl)-5-fluoropyridin-2-yl)amino)methyl)-N-(4-hydroxypyrrolidin-3-yl)-5-(methoxymethyl)-1H-imidazole-4-carboxamide
26 N-(3-aminopropyl)-2-((3-chloro-4-fluorophenyl)((5-fluoro-6-methylpyridin-2-yl)amino)methyl)-5-me-thyl-1H-imidazole-4-sulfonamide
27 2-((3-chloro-4-fluorophenyl)((5-fluoro-6-methylpyridin-2-yl)amino)methyl)-N-((3-fluoroazetidin-3-yl)methyl)-5-methyl-1H-imidazole-4-sulfonamide
28 2-((3-chloro-4-fluorophenyl)((5-fluoro-6-methylpyridin-2-yl)amino)methyl)-N-(2-(dimethyla-mino)ethyl)-5-methyl-1H-imidazole-4-sulfonamide
29 N-(azetidin-3-ylmethyl)-2-((3-chloro-4-fluorophenyl)((trans)-3-(trifluoromethyl)cyclobutoxy)methyl)-5-methyl-1H-imidazole-4-sulfonamide
30 2-((3-chloro-4-fluorophenyl)((5-fluoro-6-methoxypyridin-2-yl)amino)methyl)-5-methyl-1H-imidazole-4-carboxylic acid
31 2-((3-chloro-4-fluorophenyl)(((trans)-4-(trifluoromethyl)cyclohexyl)oxy)methyl)-5-methyl-1H-imidazole-4-carboxylic acid
32 2-((3-chloro-4-fluorophenyl)(((trans)-4-(trifluoromethyl)cyclohexyl)oxy)methyl)-N-(4-hydroxypyrrolidin-3-yl)-5-methyl-1H-imidazole-4-carboxamide
33 N-((4-((azetidin-3-ylmethyl)sulfonyl)-5-methyl-1H-imidazol-2-yl)(3-chloro-4-fluorophenyl)methyl)-5-fluoro-6-methylpyridin-2-amine
34 2-(bis(4-chloro-2-methylphenyl)methyl)-5-methyl-1H-imidazole-4-carboxylic acid
35 2-((3-chloro-4-fluorophenyl)((5-fluoro-6-methylpyridin-2-yl)amino)methyl)-5-(methoxymethyl)-1H-imidazole-4-carboxylic acid
36 2-((3-chloro-4-fluorophenyl)((6-(difluoromethyl)-5-fluoropyridin-2-yl)amino)methyl)-5-(methoxyme-thyl)-1H-imidazole-4-carboxylic acid
37 methyl 2-((3-chloro-4-fluorophenyl)((5-fluoro-6-methylpyridin-2-yl)amino)methyl)-4-methyl-1H-imidazole-5-carboxylate
38 2-((3-chloro-4-fluorophenyl)((5-fluoro-6-methoxypyridin-2-yl)amino)methyl)-5-(methoxymethyl)-1H-imidazole-4-carboxylic acid
39 2-(((6-(difluoromethyl)-5-fluoropyridin-2-yl)amino)(3,4-difluorophenyl)methyl)-5-(methoxymethyl)-1H-imidazole-4-carboxylic acid
40 2-(bis(3-chloro-4-fluorophenyl)methyl)-5-methyl-1H-imidazole-4-carboxylic acid
41 methyl 2-(bis(3-chloro-4-fluorophenyl)methyl)-4-methyl-1H-imidazole-5-carboxylate
42 2-((3,4-difluorophenyl)((5-fluoro-6-methylpyridin-2-yl)amino)methyl)-5-methyl-1H-imidazole-4-car-boxylic acid
43 methyl 2-((3-chlorophenyl)((6-(difluoromethyl)-5-fluoropyridin-2-yl)amino)methyl)-5-(methoxymethyl)-1H-imidazole-4-carboxylate
44 2-((3,4-dichlorophenyl)((6-(difluoromethyl)-5-fluoropyridin-2-yl)amino)methyl)-5-methyl-1H-imidazole-4-carboxylic acid
45 2-((3-chlorophenyl)((5-fluoro-6-methylpyridin-2-yl)amino)methyl)-5-methyl-1H-imidazole-4-carboxylic acid
46 2-((3-chloro-4-fluorophenyl)((6-(difluoromethyl)-5-fluoropyridin-2-yl)amino)methyl)-5-methyl-1H-imidazole-4-carboxylic acid
47 2-((3-chlorophenyl)((6-(difluoromethyl)-5-fluoropyridin-2-yl)amino)methyl)-5-(methoxymethyl)-1H-imidazole-4-carboxylic acid
48 methyl2-(bis(4-chloro-2-methoxyphenyl)methyl)-5-(methoxymethyl)-1H-imidazole-4-caiboxylate
492-((3-chloro-4-fluorophenyl)((4-(trifluoromethyl)cyclohexyl)oxy)methyl)-5-(methoxymethyl)-1H-im-idazole-4-carboxylic acid
50 2-(bis(4-chloro-2-methoxyphenyl)methyl)-5-(methoxymethyl)-1H-imidazole-4-carboxylic acid
51 2-(bis(4,5-difluoro-2-methoxyphenyl)methyl)-5-methyl-1H-imidazole-4-carboxylic acid
52 2-(bis(4,5-difluoro-2-methylphenyl)methyl)-5-methyl-1H-imidazole-4-carboxylic acid
53 2-(bis(4-fluoro-2-methylphenyl)methyl)-5-methyl-1H-imidazole-4-carboxylic acid
54 2-((3-chloro-4-fluorophenyl)((5-fluoro-6-methylpyridin-2-yl)amino)methyl)-4-(hydroxymethyl)-N-(1-methylazetidin-3-yl)-1H-imidazole-5-carboxamide
55 2-((3-chloro-4-fluorophenyl)((5-fluoro-6-methylpyridin-2-yl)amino)methyl)-5-(methoxymethyl)-N-(1-methylazetidin-3-yl)-1H-imidazole-4-carboxamide
56 (2-((3-chloro-4-fluorophenyl)((5-fluoro-6-methylpyridin-2-yl)amino)methyl)-5-methyl-1H-imidazol-4-yl)(2-(hydroxymethyl)piperazin-1-yl)methanone
57 (3,6-diazabicyclo[3.1.1]heptan-3-yl)(2-((3-chloro-4-fluorophenyl)((5-fluoro-6-methylpyridin-2-yl)amino)methyl)-5-methyl-1H-imidazol-4-yl)methanone
58 (2-((3-chloro-4-fluorophenyl)((5-fluoro-6-methylpyridin-2-yl)amino)methyl)-5-methyl-1H-imidazol-4-yl)(2,6-diazaspiro[3.3]heptan-2-yl)methanone
59 2-((3-chloro-4-fluorophenyl)((5-fluoro-6-methylpyridin-2-yl)amino)methyl)-N-(4-hydroxypyrrolidin-3-yl)-5-methyl-1H-imidazole-4-carboxamide
60 (3-aminoazetidin-1-yl)(2-((3-chloro-4-fluorophenyl)((5-fluoro-6-methylpyridin-2-yl)amino)methyl)-5-methyl-1H-imidazol-4-yl)methanone
61 (3-aminopyrrolidin-1-yl)(2-((3-chloro-4-fluorophenyl)((5-fluoro-6-methylpyridin-2-yl)amino)methyl)-5-methyl-1H-imidazol-4-yl)methanone
62 (3-amino-3-(methoxymethyl)azetidin-1-yl)(2-((3-chloro-4-fluorophenyl)((5-fluoro-6-methylpyridin-2-yl)amino)methyl)-4-methyl-1H-imidazol-5-yl)methanone
63 (2-((3-chloro-4-fluorophenyl)((5-fluoro-6-methylpyridin-2-yl)amino)methyl)-5-methyl-1H-imidazol-4-yl)(3-(hydroxymethyl)piperazin-1-yl)methanone
64 (2-((3-chloro-4-fluorophenyl)((5-fluoro-6-methylpyridin-2-yl)amino)methyl)-5-methyl-1H-imidazol-4-yl)(piperazin-1-yl)methanone
65 2-((3-chloro-4-fluorophenyl)((5-fluoro-6-methylpyridin-2-yl)amino)methyl)-N-(3-(hydroxymethyl)pyrrolidin-3-yl)-5-methyl-1H-imidazole-4-carboxamide
66 2-((3-chloro-4-fluorophenyl)((5-fluoro-6-methylpyridin-2-yl)amino)methyl)-N-(3-(methoxymethyl)azetidin-3-yl)-4-methyl-IH-imidazole-5-carboxaiuide
67 (3-amino-3-(hydroxymethyl)azetidin-1-yl)(2-((3-chloro-4-fluorophenyl)((5-fluoro-6-methylpyridin-2-yl)amino)methyl)-5-methyl-1H-imidazol-4-yl)methanone
68 (3-amino-3-(hydroxymethyl)pyrrolidin-1-yl)(2-((3-chloro-4-fluorophenyl)((5-fluoro-6-methylpyridin-2-yl)amino)methyl)-5-methyl-1H-imidazol-4-yl)methanone
69 (3-aminopyrrolidin-1-yl)(2-((3-chloro-4-fluorophenyl)((5-fluoro-6-methylpyridin-2-yl)amino)methyl)-5-(methoxymethyl)-1H-imidazol-4-yl)methanone
70 (2,2-bis(hydroaηmethyl)piperazin-1yl)(2-((3-chloro-4-fluorophenyl)((5-fluoro-6-methylpyridin-2-yl)amino)methyl)-5-methyl-1H-imidazol-4-yl)methanone
71 (2-((3-chloro-4-fluorophenyl)((5-fluoro-6-methylpyridin-2-yl)amino)methyl)-5-(methoxymethyl)-1H-imidazol-4-yl)(3-(dimethylamino)azetidin-1-yl)methanone
72 (2-((3-chloro-4-fluorophenyl)((5-fluoro-6-methylpyridin-2-yl)amino)methyl)-4-(methoxymethyl)-1H-imidazol-5 yl)(3-(methylamino)azetidin-1-yl)methanone
73 2-((3-chloro-4-fluorophenyl)((5-fluoro-6-methylpyridin-2-yl)amino)methyl)-N-(4-hydroxypyrrolidin-3-yl)-5-(methoxymethyl)-1H-imidazole-4-carboxamide
74 (3-aminoazetidin-1-yl)(2-(((6-(difluoromethyl)-5-fluoropyridin-2-yl)amino)(3,4-difluorophenyl)methyl)-5-(methoxymethyl)-1H-imidazol-4-yl)methanone
76 N-(3-aminocyclobutyl)-2-((3-chloro-4-fluorophenyl)((5-fluoro-6-methylpyridin-2-yl)amino)methyl)-4-methyl-1H-imidazole-5-sulfonamide
77 N-((5-((3-aminoazetidin-1-yl)sulfonyl)-4-methyl-1H-imidazol-2-yl)(3-chloro-4-fluorophenyl)methyl)-5-fluoro-6-methylpyridin-2-amine
78 2-((3-chloro-4-fluorophenyl)((5-fluoro-6-methylpyridin-2-yl)amino)methyl)-N-(3-(hydroxymethyl)azetidin-3-yl)-5-methyl-1H-imidazole-4-carboxamide
79 N-(3-amino-2,2-difluoropropyl)-2-((3-chloro-4-fluorophenyl)((5-fluoro-6-methylpyridin-2-yl)amino)methyl)-4-methyl-1H-imidazole-5-sulfonamide
80 2-((3-chloro-4-fluorophenyl)((5-fluoro-6-methylpyridin-2-yl)amino)methyl)-4-methyl-N-(pyrrolidin-2-ylmethyl)-1H-imidazole-5-sulfonamide
81 2-((3-chloro-4-fluorophenyl)((5-fluoro-6-methylpyridin-2-yl)amino)methyl)-N-(3-(dimethylamino)propyl)-4-methyl-1H-imidazole-5-sulfonamide
82 N-(azetidin-3-ylmethyl)-2-((3-chloro-4-fluorophenyl)((5-fluoro-6-methylpyridin-2-yl)amino)methyl)-5-(methoxymethyl)-1H-imidazole-4-carboxamide
83 2-((3-chloro-4-fluorophenyl)((5-fluoro-6-methylpyridin-2-yl)amino)methyl)-4-methyl-N-(piperidin-3-yl)-1H-imidazole-5-sulfonamide
84 2-((3-chloro-4-fluorophenyl)((5-fluoro-6-methylpyridin-2-yl)amino)methyl)-4-methyl-N-(1-methylazetidin-3-yl)-1H-imidazole-5-sulfonamide
85 2-((3-chloro-4-fluorophenyl)((5-fluoro-6-methylpyridin-2-yl)amino)methyl)-4-methyl-N-(2-(1-methylpyrrolidin-2-yl)ethyl)-1H-imidazole-5-sulfonamide
86 2-((3-chloro-4-fluorophenyl)((5-fluoro-6-methylpyridin-2-yl)amino)methyl)-4-methyl-N-(2-(methylamino)ethyl)-1H-imidazole-5-sulfonamide
87 2-((3-chloro-4-fluorophenyl)((4-(trifluoromethyl)cyclohexyl)oxy)methyl)-N-(4-hydroxy-1-methylpyr-rolidin-3-yl)-5-(methoxymethyl)-1H-imidazole-4-carboxamide
88 2-((3-chloro-4-fluorophenyl)((5-fluoro-6-methylpyridin-2-yl)amino)methyl)-N-(4-hydroxypyrrolidin-3-yl)-4-methyl-1H-imidazole-5-sulfonamide
89 2-((3-chloro-4-fluorophenyl)((5-fluoro-6-methylpyridin-2-yl)amino)methyl)-4-methyl-N-(pyrrolidin-3-yl)-1H-imidazole-5-sulfonamide
91 N-(azetidin-2-ylmethyl)-2-((3-chloro-4-fluorophenyl)((5-fluoro-6-methylpyridin-2-yl)amino)methyl)-4-methyl-1H-imidazole-5-sulfonamide
92 N-(2-aminoethyl)-2-((3-chloro-4-fluorophenyl)((5-fluoro-6-methylpyridin-2-yl)amino)methyl)-4-me-thyl-1H-imidazole-5-sulfonamide
93 2-((3-chloro-4-fluorophenyl)((5-fluoro-6-methylpyridin-2-yl)amino)methyl)-4-methyl-N-((1-methylazetidin-3-yl)methyl)-1H-imidazole-5-sulfonamide
94 2-((3-chloro-4-fluorophenyl)((5-fluoro-6-methylpyridin-2-yl)amino)methyl)-N-(4-fluoropyrrolidin-3-yl)-5-(methoxymethyl)-1H-imidazole-4-carboxamide
95 (3-aminoazetidin-1-yl)(2-((3-chloro-4-fluorophenyl)((5-fluoro-6-methoxypyridin-2-yl)amino)methyl)-5-(methoxymethyl)-1H-imidazol-4-yl)methanone
96 N-(azetidin-3-yl)-2-((3-chloro-4-fluorophenyl)((5-fluoro-6-methylpyridin-2-yl)amino)methyl)-4-methyl-1H-imidazole-5-sulfonamide
97 2-((3-chloro-4-fluorophenyl)((5-fluoro-6-methylpyridin-2-yl)amino)methyl)-N-((1-ethylpyrrolidin-2-yl)methyl)-4-methyl-1H-imidazole-5-sulfonamide
98 2-((3-chloro-4-fluorophenyl)((4-(trifluoromethyl)cyclohexyl)oxy)methyl)-N-(4-hydroxypyrrolidin-3-yl)-5-(methoxymethyl)-1H-imidazole-4-carboxamide
99 N-(azetidin-3-ylmethyl)-2-((3-chloro-4-fluorophenyl)((4-(trifluoromethyl)cyclohexyl)oxy)methyl)-5-(methoxymethyl)-1H-imidazole-4-carboxamide
100 2-((3-chloro-4-fluorophenyl)((5-fluoro-6-methylpyridin-2-yl)amino)methyl)-4-methyl-N-((1-methylazetidin-2-yl)methyl)-1H-imidazole-5-sulfonamide
101 2-((3-chloro-4-fluorophenyl)((5-fluoro-6-methylpyridin-2-yl)amino)methyl)-N-(4-fluoro-1-methylpyrrolidin-3-yl)-5-(methoxymethyl)-1H-imidazole-4-carboxamide
102 N-(azetidin-3-ylmethyl)-2-((3-chloro-4-fluorophenyl)((5-fluoro-6-methylpyridin-2-yl)amino)methyl)-5-methyl-1H-imidazole-4-carboxamide
103 2-((3-chloro-4-fluorophenyl)((5-fluoro-6-methylpyridin-2-yl)amino)methyl)-N-(4-fluoropyrrolidin-3-yl)-5-methyl-1H-imidazole-4-carboxamide
and the physiologically acceptable salts thereof.

In a preferred embodiment, the compound according to the invention is an inhibitor of Na_{V}1.8. In the sense of the invention, the term "inhibitor of Na_{V}1.8" preferably means that the respective compound exhibits in a patch clamp assay an IC50 value on Na_{V}1.8 of at most 10 µM (10·10⁻⁶ mol/L); more preferably at most 1 µM; still more preferably at most 500 nM (10⁻⁹ mol/L); even more preferably at most 100 nM; and most preferably at most 10 nM.

A preferred assay for testing compounds for their potency and method for determining an IC50 on Na_{V}1.8 is described in the experimental part down below.

In a preferred embodiment, the compound according to the invention is a selective inhibitor of Na_{V}1.8. In the sense of the invention, the term "selective inhibitor of Na_{V}1.8" preferably means that the respective compound preferably does not exhibit any inhibitory activity on Na_{V}1.1, Na_{V}1.2, Na_{V}1.4, Na_{V}1.5 and Na_{V}1.6. The skilled artisan knows suitable ways to determine whether a compound exhibits inhibitory effects on any of Na_{V}1.1, Na_{V}1.2, Na_{V}1.4, Na_{V}1.5 and Na_{V}1.6.

The invention therefore relates to a compound according to the invention for use in the inhibition of Na_{V}1.8.

Therefore, another aspect of the invention relates to a compound according to the invention for use in the treatment of pain. Still another aspect of the invention relates to a method of treatment of pain; comprising the administration of a therapeutically effective amount of a compound according to the invention to a subject in need thereof, preferably a human.

A further aspect of the invention relates to a compound according to the invention as medicament.

Another aspect of the invention relates to a pharmaceutical dosage form comprising a compound according to the invention. Preferably, the pharmaceutical dosage form comprises a compound according to the invention and one or more pharmaceutical excipients such as physiologically acceptable carriers, additives and/or auxiliary substances; and optionally one or more further pharmacologically active ingredient. Examples of suitable physiologically acceptable carriers, additives and/or auxiliary substances are fillers, solvents, diluents, colorings and/or binders. These substances are known to the person skilled in the art (see H. P. Fiedler, Lexikon der Hilfsstoffe fur Pharmazie, Kosmetik und angrenzende Gebiete, Editio Cantor Aulendoff).

The pharmaceutical dosage form according to the invention is preferably for systemic, topical or local administration, preferably for oral administration. Therefore, the pharmaceutical dosage form can be in form of a liquid, semisolid or solid, e.g. in the form of injection solutions, drops, juices, syrups, sprays, suspensions, tablets, patches, films, capsules, plasters, suppositories, ointments, creams, lotions, gels, emulsions, aerosols or in multiparticulate form, for example in the form of pellets or granules, if appropriate pressed into tablets, decanted in capsules or suspended in a liquid, and can also be administered as such.

The pharmaceutical dosage form according to the invention is preferably prepared with the aid of conventional means, devices, methods and processes known in the art. The amount of the compound according to the invention to be administered to the patient may vary and is e.g. dependent on the patient's weight or age and also on the type of administration, the indication and the severity of the disorder. Preferably 0.001 to 100 mg/kg, more preferably 0.05 to 75 mg/kg, most preferably 0.05 to 50 mg of a compound according to the invention are administered per kg of the patient's body weight.

Therefore, another aspect of the invention relates to the pharmaceutical dosage form according to the invention for use in the treatment of pain. Still another aspect of the invention relates to a method of treatment of pain; comprising the administration of a pharmaceutical dosage form according to the invention to a subject in need thereof, preferably a human.

### General synthesis schemes

Examples of the invention can be synthesized preferentially according to the general synthetic schemes below or using chemistry known to the person skilled in the art.

Protection of imidazoles can lead to two different regioisomers. Both regioisomers can generally undergo similar transformation in similar set of condition. In the following, only one of the regiosiomers is indicated for clarity. Deprotection of either of the two regiosiomers will lead to the same final compounds.

As illustrated in Scheme 1 for compounds with L=bond, one equivalent of compounds of the general formula (A) can be treated with two equivalents of metal-organic compounds of the general formula (B) (wherein A=B, R2=R3 and x=y) to afford compounds of general formula (C). Preferred metal-organic compounds include Grignard and organolithium reagents. Compounds of the general formula (C) can be converted into compounds of the general formula (D) with reactions known to the person skilled in the art. Preferred transformations include reductions to get compounds of the general formula (D), preferred reagents include tin (II)chloride in acetic media.

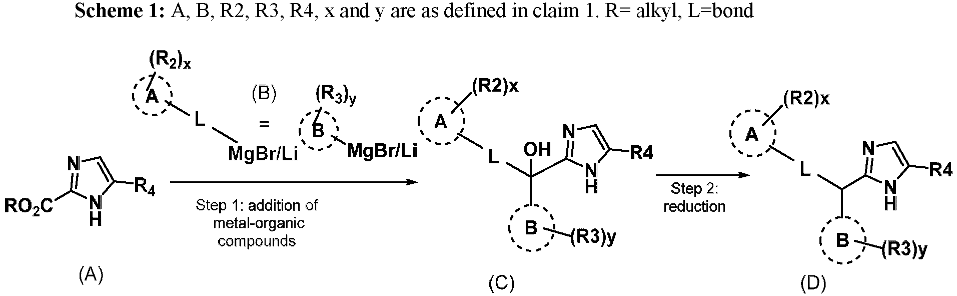

Aldehydes of the general formula (E), protected imidazoles of the general formula (F) and N,N-bis(pro-pan-2-yl)carbamoyl chloride (G) in the presence of base (preferred bases include DIPEA) can be reacted in a multicomponent reaction to get compounds of the general formula (H). Acid-catalyzed displacement between compounds of the general formula (H) and alcohols (L=O) of the general formula (J) or amines (L=NH) of the general formula (J) yields compounds of the general formula (D). Preferred conditions for this displacement include the use of BF₃·Et₂O in combination with TFA, which in some cases leads to concomitant deprotection. In some cases, intermediate protection and later deprotection of the imidazole may be warranted, preferred protecting groups include SEM.

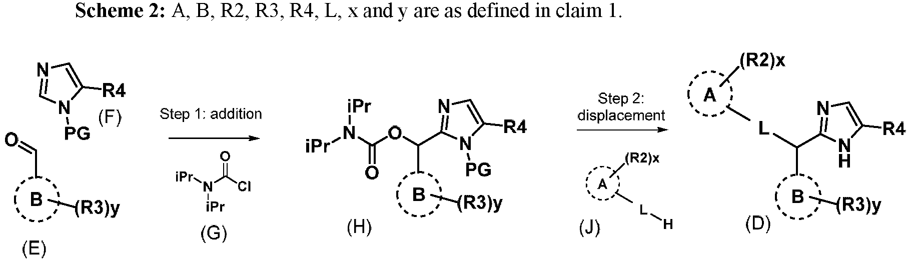

As illustrated in Scheme 3, compounds of the general formula (D) can be halogenated, preferentially iodinated using I₂ or NIS to obtain compounds of the general formula (K). These compounds can be used to install a variety of R5 moieties to yield compounds of the general formula (I). One step procedures include the formation of amides or esters via palladium catalyzed carbonylation (*Angew. Chem. Int. Ed.* **2009,** *48*, 4114-4133). Formation of amides can also be carried out via saponification of esters followed by amide coupling using standard reaction conditions known to the person skilled in the art. Multi-step procedures include thioether formation followed by oxidation to yield the corresponding sulfonylchlorides (preferred reagents include NCS) and then further conversion to the corresponding sulfonamides upon treatment with nitrogen nucleophiles. In the case of R₄=H, iodination using NIS yields bis-iodinated products (hal=I, R4=I) that are transformed into compounds of the general formula (K) with R4=H upon treatment with sodium thiosulfate. Certain transformations require the intermediate protection and later deprotection of the imidazole, preferred protecting groups include SEM. Certain transformation require the use of intermediate protecting groups on amines, such as Boc. Protection and deprotection of both SEM and Boc are known in the art.

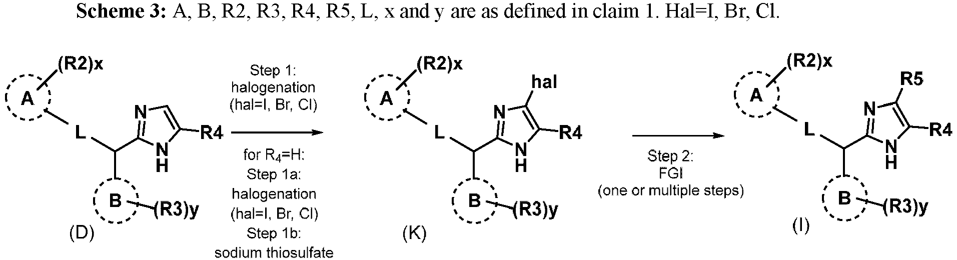

### List of abbreviations

ABPR= automated back pressure regulator, ACN= acetonitrile, BINAP= [2,2'-Bis(diphenylphosphino)-1,1'-binaphthyl], Boc= tert-butyloxycarbonyl, DAST= diethylaminosulfur trifluoride, dba= dibenzylideneacetone, DCM= dichloromethane, DIBAL= diisobutylaluminium hydride, DIPEA= N,N-diisopropylethylamine, , DMF= dimethylformamide, DMSO= dimethyl sulfoxide, dppf= 1,1'-bis(diphenylphosphino)ferrocene, EA=ethyl acetate, EDC/EDCI= 1-ethyl-3-(3-dimethylaminopropyl)carbodiimide, FGI=functional group interconversion, HATU= [O-(7-Azabenzotriazol-1-yl)-N,N,N',N'-tetramethyluronium-hexafluorphosphat, Hex=hexane/hexanes, HOBT= hydroxybenzotriazole, HPLC= high-performance liquid chromatography, LCMS= liquid chromatography-mass spectrometry, m-CPBA= meta-chloroperoxybenzoic acid, NCS= N-chlorosuccinimide, NIS= N-iodosuccinimide, NMR= nuclear magnetic resonance, PG=protecting group, rt= room temperature, Rt=retention time, SEM= 2-(trimethylsilyl)ethoxymethyl, SFC= supercritical fluid chromatography, T3P= propanephosphonic acid anhydride, TBAF= tetra-n-butylammonium fluoride, TEA= triethylamine, TFA= trifluoroacetic acid, TFAA= trifluoroacetic anhydride, THF=tetrahydrofurane, Xantphos= (9,9-dimethyl-9H-xanthene-4,5-diyl)bis(diphenylphosphane).

### Intermediate 1: bis(3-chloro-4-fluorophenyl)(1H-imidazol-2-yl)methanol

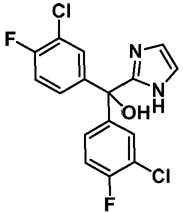

Mg (0.86 g, 35.71 mmol) was weighed in into a two-neck flask equipped with a reflux condenser and dried under an argon atmosphere. Then THF (7.0 mL), 4-bromo-2-chloro-1-fluorobenzene (7.5 g, 35.71 mmol) and DIBAL (1M in toluene, 0.4 mL) were added to the mixture sequentially at rt. The mixture was stirred at rt for 1 h. To the freshly prepared reagent was then added a solution of ethyl 1H-imidazole-2-carboxylate (1.0 g, 7.14 mmol) in THF (33.0 mL) at 0 °C and the reaction mixture was heated to 80 °C for 3 h. The reaction mixture was cooled to rt, quenched with ammonium chloride solution and extracted with ethyl acetate (3 x 100 mL). The combined organic layers were washed with brine, dried over Na₂SO₄ and concentrated under reduced pressure to get the crude product which purified by column chromatography (SiO₂, 0-60% EtOAc/Hex) to yield the title compound (1.5 g, 59%), LCMS: m/z [M+H]⁺ = 355.

### Intermediate 2: 2-(bis(3-chloro-4-fluorophenyl)methyl)-1H-imidazole

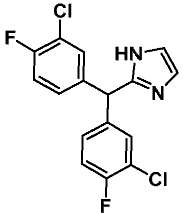

To a solution of bis(3-chloro-4-fluorophenyl)(1H-imidazol-2-yl)methanol (intermediate 1, 1.5 g, 4.22 mmol) in acetic acid (15.0 mL) were added SnCl₂ (4.8 g, 25.35 mmol) and HCl (12N, 3.0 mL) at rt. The reaction mixture was heated to 120 °C under reflux conditions for 16 h, cooled to rt and concentrated under reduced pressure. The residue was neutralized with NaHCO₃ solution and extracted with ethyl acetate (3 × 150 mL). The combined organic layers were washed with brine, dried over Na₂SO₄ and concentrated under reduced pressure. The resulting crude product was purified by column chromatography (SiO₂, 0-80% EtOAc/Hex) to yield the title compound (1.1 g, 77%), LCMS: m/z [M+H]⁺ = 339.

### Intermediate 3: 2-(bis(3-chloro-4-fluorophenyl)methyl)-4,5-diiodo-1H-imidazole

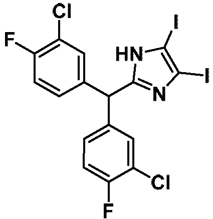

To a solution of 2-(bis(3-chloro-4-fluorophenyl)methyl)-1H-imidazole (intermediate 2, 1.5 g, 4.42 mmol) in DMF (20.0 mL) were added KOH (0.5 g, 8.85 mmol) and I₂ (1.7 g, 6.63 mmol) at 0 °C and the reaction mixture was stirred at rt for 2 h. The reaction mixture was quenched with sodium thiosulfate solution, the aqueous layer was extracted with ethyl acetate (3 × 100 mL). The combined organic layers were washed with brine, dried over Na₂SO₄ and concentrated under reduced pressure. The resulting crude product was purified by column chromatography (SiO₂, 0-40% EtOAc/Hex) to yield the title compound (1.8 g, 69%), LCMS: m/z [M+H]⁺ = 591.

### Intermediate 4: 2-(bis(3-chloro-4-fluorophenyl)methyl)-4-iodo-1H-imidazole

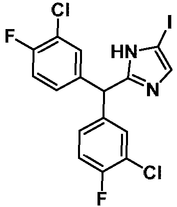

To a stirred solution of 2-[bis(3-chloro-4-fluorophenyl)methyl]-4,5-diiodo-1H-imidazole (intermediate 3, 2.2 g, 3.72 mmol) in ethanol (25.0 mL) and water (7.0 mL) was added sodium thiosulfate (18.5 g, 74.57 mmol) at rt and the mixture was heated to 110 °C under reflux conditions for 16 h. Sodium thiosulfate (18.5 g, 74.57 mmol) was then added again to the reaction mixture and the mixture was heated to 110 °C for 4 days. The reaction mixture was cooled to rt, diluted with water and extracted with ethyl acetate (2 x 100 mL). The combined organic layers were washed with brine, dried over Na₂SO₄ and concentrated under reduced pressure. The residue was purified by column chromatography (SiO₂, 0-40% EtOAc/Hex) to yield the title compound (1.5 g, 86%), LCMS: m/z [M+H]⁺ = 465.

### Intermediate 5: 2-(bis(3-chloro-4-fluorophenyl)methyl)-4-iodo-1-((2-(trimethylsilyl)ethoxy)methyl)-1H-imidazole

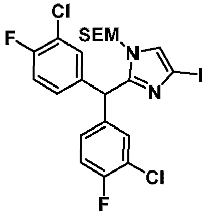

To a solution of 2-(bis(3-chloro-4-fluorophenyl) methyl)-4-iodo-1H-imidazole (intermediate 4, 2.0 g, 4.301 mmol) in DMF (15.0 mL) was added NaH (0.343 g, 8.602 mmol, 60% suspension) at 0 °C, the mixture was then stirred at rt for 30 minutes. Then SEM-Cl (1.14 mL, 6.452 mmol) was added after cooling the mixture to 0 °C and the mixture was then stirred at rt for 16 h. The reaction mixture was quenched with saturated ammonium chloride solution and extracted with ethyl acetate (3 x 100 mL). The combined organic layers were washed with water, brine, dried over anhydrous Na₂SO₄ and concentrated under reduced pressure. The residue was purified by column chromatography (SiO₂, 10% EtOAc/Hex) to yield the title compound (2.0 g, 78%), LCMS: m/z [M+H]⁺ = 595.

### Intermediate 9: methyl 2-(bis(3-chloro-4-fluorophenyl)methyl)-1H-imidazole-4-carboxylate

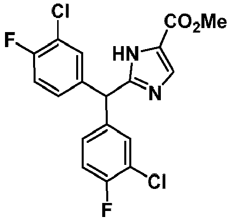

A solution of 2-(bis(3-chloro-4-fluorophenyl)methyl)-4-iodo-1H-imidazole (intermediate 4, 0.1 g, 0.216 mmol) and TEA (0.15 mL, 1.07 mmol) in MeOH (15.0 mL) was degassed with argon for 10 min followed by the addition of dppf (0.012 g, 0.022 mmol) and PdCl₂(dppf)·DCM (0.01 g, 0.011 mmol) at rt. The reaction mixture was heated to 70 °C under 8.0 bar pressure of CO gas for 16 h. The reaction mixture was cooled to rt, filtered through a celite bed and the filtrate was concentrated under reduced pressure. The residue was purified by column chromatography (SiO₂, 0-60% EtOAc/Hex) to yield the title compound (0.07 g, 81%), LCMS: m/z [M+H]⁺ = 397.

### Intermediate 10: methyl 2-(bis(3-chloro-4-fluorophenyl)methyl)-5-iodo-1H-imidazole-4-carboxylate

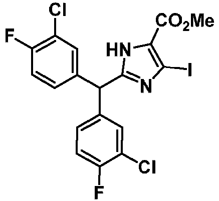

To a stirred solution of methyl 2-(bis(3-chloro-4-fluorophenyl)methyl)-1H-imidazole-4-carboxylate (intermediate 9, 1.0 g, 2.525 mmol) in DCM (50.0 mL) and DMF (1.0 mL) was added NIS (0.852 g, 3.788 mmol) at rt. The reaction was stirred at rt for 16 h and was then quenched with sodium thiosulfate solution. The aqueous layer was extracted with ethyl acetate (2 × 100 mL). The combined organic layers were washed with brine, dried over Na₂SO₄ and concentrated under reduced pressure to get the crude product which was purified by column chromatography (SiO₂, 0-60% EtOAc/Hex) to yield the title compound (1.0 g, 75%), LCMS: m/z [M+H]⁺ = 523.

### Intermediate 11: 2-(bis(3-chloro-4-fluorophenyl)methyl)-N-(tert-butyl)-1H-imidazole-4-carboxamide

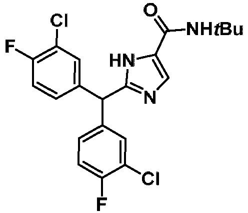

To a stirred solution of 2-(bis(3-chloro-4-fluorophenyl)methyl)-4-iodo-1H-imidazole (intermediate 4, 0.5 g, 1.08 mmol) in toluene (15 mL) was added TEA (0.35 mL, 3.25 mmol) and the mixture was degassed with argon for 10 min. After degassing, triphenyl phosphine (0.057 g, 0.216 mmol), tertbutyl amine (0.395 g, 5.389 mmol) and Pd(OAc)₂ (0.025 g, 0.108 mmol) were added to the reaction mixture at rt. The reaction mixture was heated to 100 °C under 8.0 bar CO gas pressure for 16 h in a Parr autoclave. The reaction mixture was cooled to rt, filtered through a celite bed and the filtrate was concentrated under reduced pressure. The residue was purified by column chromatography (SiO₂, 0-40% EtOAc/Hex) to yield the title compound (0.3 g, 63%), LCMS: m/z [M+H]⁺ = 438.

### Intermediate 12: 2-(bis(3-chloro-4-fluorophenyl)methyl)-N-(tert-butyl)-5-iodo-1H-imidazole-4-carboxamide

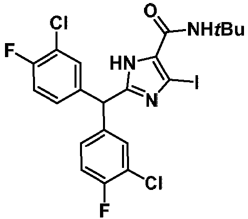

To a stirred solution of 2-(bis(3-chloro-4-fluorophenyl)methyl)-N-(tert-butyl)-1H-imidazole-4-carboxamide (intermediate 11, 0.3 g, 0.69 mmol) in DCM (10 mL) was added NIS (0.19 g, 0.82 mmol) at 0 °C. The reaction was then stirred at rt for 3 h. The reaction mixture was quenched with sodium thiosulfate solution. The aqueous layer was extracted with ethyl acetate (2×30 mL). The combined organic layers were washed with brine, dried over Na₂SO₄ and concentrated under reduced pressure to get the crude product, which was purified by column chromatography (SiO₂, 0-60% EtOAc/Hex) to yield the title compound (0.2 g, 51%), LCMS: m/z [M+H]⁺ = 564.

### Intermediate 14: bis(3-chloro-4-fluorophenyl)(5-methyl-1H-imidazol-2-yl)methanol

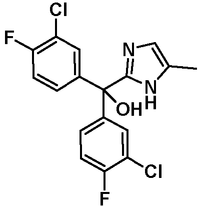

Mg (3.2 g, 129.727 mmol) was taken up in a 2-necked flask equipped with a reflux condenser and dried under argon atmosphere. THF (30.0 mL), 4-bromo-2-chloro-1-fluorobenzene (15.8 mL, 129.727 mmol) and DIBAL (1M in toluene) (2.0 mL) were added to the mixture sequentially at rt. The reaction mixture was stirred at rt for 1 h. To this mixture, a solution of ethyl 4-methyl-1H-imidazole-2-carboxylate (4.0 g, 25.95 mmol) in THF (170.0 mL) was added at 0 °C and the reaction mixture was heated to 80 °C for 5 h. The reaction mixture was cooled to rt and quenched with ammonium chloride solution. The aqueous layer was filtered through a celite bed and extracted with ethyl acetate (3 x 200 mL). The combined organic layers were washed with brine, dried over Na₂SO₄ and concentrated under reduced pressure to get the crude product which was purified by column chromatography (SiO₂, 0-60% EtOAc/Hex) to yield the title compound (7.1 g, 74%), LCMS: m/z [M+H]⁺ = 369.

### Intermediate 15

The title compound was prepared using an analogous method to that described for intermediate 14.

| | | |
|---|---|---|
| 15 | 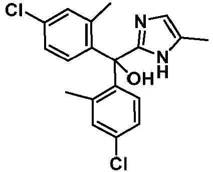 | bis(4-chloro-2-methylphenyl)(5-methyl-1H-imidazol-2-yl)methanol, |
| | | LCMS: m/z [M+H]⁺ = 491. |

### Intermediate 16: bis(4-chloro-2-methoxyphenyl)(5-methyl-1H-imidazol-2-yl)methanol

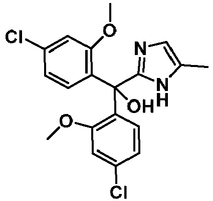

To a stirred solution of 1-bromo-4-chloro-2-methoxybenzene (31 g, 139.5 mmol) in THF (175 mL) was added i-PrMgBr (2 M in THF, 70 mL, 139.4 mmol) dropwise at 0 °C and the resulting solution was stirred at 40 °C for 16 h. To this freshly prepared Grignard reagent, a solution of ethyl 5-methyl-1H-imidazole-2-carboxylate (4.3 g, 27.9 mmol) in THF (25 mL) was added at 0 °C and the reaction mixture was heated to 80 °C for 3 h. The reaction mixture was quenched with ammonium chloride solution. The aqueous part was extracted with ethyl acetate (2 x 300 mL). The combined organic layers were washed with brine (150 mL), dried over anhydrous sodium sulfate and concentrated under reduced pressure to afford bis(4-chloro-2-methoxyphenyl)(5-methyl-1H-imidazol-2-yl)methanol. Yield: crude (10.96 g). LCMS: m/z [M+H]⁺ = 393.

### Intermediate 17: 2-(bis(3-chloro-4-fluorophenyl)methyl)-4-methyl-1H-imidazole

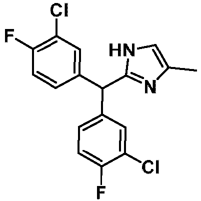

To a solution of bis(3-chloro-4-fluorophenyl)(5-methyl-1H-imidazol-2-yl)methanol (intermediate 14, 7.0 g, 19.02 mmol) in acetic acid (100.0 mL) were added SnCl₂ (21.6 g, 114.13 mmol) and conc. HCl (21.0 mL) at 0 °C. The reaction mixture was stirred at 120 °C under reflux conditions for 16 h. The reaction mixture was cooled to rt and concentrated under reduced pressure. The resulting crude residue was basified with Na₂CO₃ solution. The aqueous layer was filtered through a celite bed and the filtrate was extracted with ethyl acetate (3 × 200 mL). The combined organic layers were washed with brine, dried over Na₂SO₄ and concentrated under reduced pressure to get the crude product which was washed with 10% ethyl acetate in hexane to obtain the title compound (5.2 g, 77%), LCMS: m/z [M+H]⁺ = 353.

### Intermediates 18-19

The title compound was prepared using an analogous method to that described for intermediate 17.

| Intermediate | Structure | Name/Data |
|---|---|---|
| 18 | 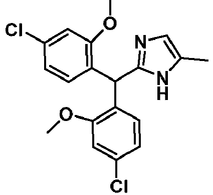 | 2-(bis(4-chloro-2-methoxyphenyl)methyl)-5-methyl-1H-imidazole. |
| | | Yield: 57% over 2 steps (6 g). |
| | | LCMS: m/z [M+H]⁺ = 377. |
| 19 | 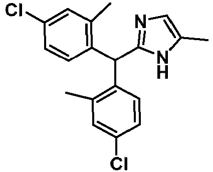 | 2-(bis(4-chloro-2-methylphenyl)methyl)-5-methyl-1H-imidazole. |
| | | LCMS: m/z [M+H]⁺ = 345. |

### Intermediate 20: 2-(bis(3-chloro-4-fluorophenyl)methyl)-5-iodo-4-methyl-1H-imidazole

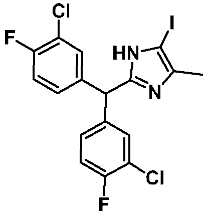

To a solution of 2-[bis(3-chloro-4-fluorophenyl)methyl]-5-methyl-1H-imidazole (intermediate 17, 2.5 g, 7.102 mmol) in DMF (30.0 mL) were added KOH (0.6 g, 10.653 mmol) and I₂ (2.4 g, 9.233 mmol) at 0 °C. The reaction mixture was then stirred at rt for 1 h. The reaction mixture was quenched with sodium thiosulfate solution, the aqueous layer was extracted with ethyl acetate (2 × 150 mL). The combined organic layers were washed with brine, dried over Na₂SO₄ and evaporated under reduced pressure to get the crude product which was purified by column chromatography (SiO₂, 0-40% EtOAc/Hex) to yield the title compound (2.2 g, 64%), LCMS: m/z [M+H]⁺ = 479.

### Intermediates 21-22

The title compound was prepared using an analogous method to that described for intermediate 20.

| Intermediate | Structure | Name/Data |
|---|---|---|
| 21 | 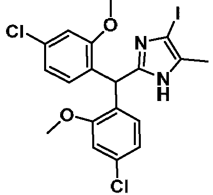 | 2-(bis(4-chloro-2-methoxyphenyl)methyl)-4-iodo-5-methyl-1H-imidazole. |
| | | Yield: 96% (4.63 g). |
| | | LCMS: m/z [M+H]⁺ = 503. |
| 22 | 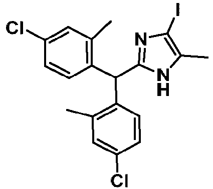 | 2-(bis(4-chloro-2-methylphenyl)methyl)-4-iodo-5-methyl-1H-imidazole. |
| | | LCMS: m/z [M+H]⁺ = 471. |

### Intermediate 23: methyl 2-(bis(4-chloro-2-methoxyphenyl)methyl)-5-methyl-1-((2-(trimethylsilyl)ethoxy)methyl)-1H-imidazole-4-carboxylate

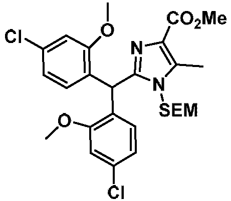

Step 1: To a solution of 2-(bis(4-chloro-2-methoxyphenyl)methyl)-4-iodo-5-methyl-1H-imidazole (intermediate 21, 4.63 g, 9.2 mmol) in DCM (60 mL) was added DIPEA (4.8 mL, 27.605 mmol) followed by SEM-Cl (2.4 mL, 13.803 mmol) at 0 °C. The resulting reaction mixture was stirred at rt for 16 h. The reaction mixture was poured into ice water and extracted with ethyl acetate (2 × 80 mL). The combined organic layers were washed with water (60 mL) followed by brine (60 mL), dried over anhydrous sodium sulfate and concentrated under reduced pressure to afford the crude product, which was purified by column chromatography (SiO₂, 10% EtOAc/Hex) to afford 2-(bis(4-chloro-2-methoxyphenyl)methyl)-4-iodo-5-methyl-1-((2-(trimethylsilyl)ethoxy)methyl)-1H-imidazole. Yield: 82% (4.8 g). LCMS: m/z [M+H]⁺ = 633.

Step 2: A solution of 2-(bis(4-chloro-2-methoxyphenyl)methyl)-4-iodo-5-methyl-1-((2-(trimethylsilyl)ethoxy)methyl)-1H-imidazole (1 g, 1.579 mmol) and TEA (0.66 mL, 4.736 mmol) in MeOH (50 mL) was degassed with argon for 10 min followed by the addition of PdC1₂(dppf)-DCM (0.13 g, 0.158 mmol) at rt. The resulting reaction mixture was heated to 90 °C under 10 bar pressure of CO gas for 16 h in a Parr autoclave. The reaction mixture was cooled to rt and filtered through a celite bed. The filtrate was concentrated under reduced pressure to afford the crude product, which was purified by column chromatography (SiO₂, 20% EtOAc/Hex) to afford the tile compound. Yield: 67% (600 mg). LCMS: m/z [M+H]⁺ = 565.

### Intermediate 24: 4-iodo-1-((2-(trimethylsilyl)ethoxy)methyl)-1H-imidazole

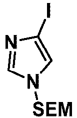

Sodium hydride (60% dispersion in mineral oil, 0.297 g, 12.372 mmol) was added to a solution of 4-iodo-1H-imidazole (2.0 g, 10.310 mmol) in DMF (20 mL) at 0 °C. The mixture was stirred at 0 °C for 1 h followed by the addition of SEM-Cl (1.829 mL, 10.310 mmol) at 0 °C. The reaction was stirred at rt for 18 h. The reaction mixture was diluted with ice water (10 mL) and extracted with ethyl acetate (20 mL × 2). The combined organic layers were dried over anhydrous Na₂SO₄, filtered and concentrated under reduced pressure to get the crude material which was purified by flash chromatography (SiO₂, 0-50% EtOAc/Hex) to afford the title compound (1.7 g, 51%).

### Intermediate 25: 2-(bis(3-chloro-4-fluorophenyl)methyl)-1H-imidazole-5-carboxylic acid

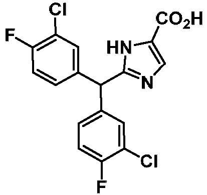

To a stirred solution of methyl 2-(bis(3-chloro-4-fluorophenyl)methyl)-1H-imidazole-4-carboxylate (intermediate 9, 1.2 g, 3.03 mmol) in 1,4-dioxane (40.0 mL) was added HCl (6N in water; 40.0 mL) at rt. The reaction mixture was heated to 90 °C for 24 h. The reaction mixture was concentrated under reduced pressure to get the crude product, which was triturated with 1% acetone in hexane to yield the title compound as the corresponding hydrochloride (1.0 g, 86%). LCMS: m/z [M+H]⁺ = 381.

**Intermediate 26:** methyl 2-((3-chloro-4-fluorophenyl)((5-fluoro-6-methylpyridin-2-yl)amino)methyl)-5-methyl-1-((2-(trimethylsilyl)ethoxy)methyl)-1H-imidazole-4-carboxylate

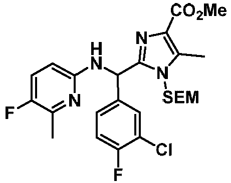

Step 1: To a stirred suspension of 4-iodo-5-methyl-1H-imidazole (40 g, 480.77 mmol) in toluene (400 mL) was added DIPEA (67 mL, 385 mmol) followed by SEM-Cl (41 mL, 230.769 mmol) at rt. The resulting solution was heated to 90 °C for 16 h. The reaction mixture was washed with H₂O (250 mL). The organic part was concentrated under reduced pressure, diluted with ethyl acetate (600 mL) and washed with H₂O (2 × 150 mL) and brine (150 mL). The organic layer was dried over Na₂SO₄ and concentrated under reduced pressure to afford 4-iodo-5-methyl-1-((2-(trimethylsilyl)ethoxy)methyl)-1H-imidazole (62 g, 95%). LCMS: m/z [M+H]⁺ = 339.

Step-2: To a stirred solution of 4-iodo-5-methyl-1-((2-(trimethylsilyl)ethoxy)methyl)-1H-imidazole (10.0 g, 29.475 mmol) in MeCN (150.0 mL) were added 3-chloro-4-fluorobenzaldehyde (5.41 g, 88.42 mmol), N,N-bis(propan-2-yl)carbamoyl chloride (5.788 g, 35.37 mmol) and DIPEA (15.78 mL, 88.42 mmol) at rt. The reaction mixture was stirred at 80 °C for 16 h. The reaction mixture was concentrated under reduced pressure to get the crude product which was diluted with EtOAc (200 mL) and washed with H₂O (100 mL) and brine (100 mL). The organic extract was dried over anhydrous Na₂SO₄, filtered and concentrated under reduced pressure to get the crude product which was purified by flash chromatography (SiO₂, 0-50% EtOAc/Hex) to afford (3-chloro-4-fluorophenyl)(4-iodo-5-methyl-1-((2-(trimethylsilyl)ethoxy)methyl)-1H-imidazol-2-yl)methyl diisopropylcarbamate (13.0 g, 71%). LCMS: m/z [M+H]⁺ = 624.

Step-3: To a stirred solution of (3-chloro-4-fluorophenyl)(5-iodo-4-methyl-1-{[2-(trimethylsilyl)ethoxy]methyl}-1H-imidazol-2-yl)methyl N,N-bis(propan-2-yl)carbamate (15.3 g, 24.52 mmol) in DCM (300 mL) were added 5-fluoro-6-methylpyridin-2-amine (7.73 g, 61.298 mmol), BF₃·Et₂O (4.54 mL, 36.779 mmol) and TFA (8.44 mL, 110.336 mmol). The resulting mixture was stirred at rt for 16 h. The reaction mixture was washed with NaHCO₃ solution (150 mL), brine (100 mL) and dried over anhydrous sodium sulfate. The solvent was concentrated under reduced pressure to afford the crude product which was purified by flash chromatography (SiO₂, 0-100% EtOAc/Hex) to afford N-[(3-chloro-4-fluorophenyl)(4-iodo-5-methyl-1-{[2-(trimethylsilyl)ethoxy]methyl}-1H-imidazol-2-yl)methyl]-5-fluoro-6-methylpyridin-2-amine (13.0 g, 88%). LCMS: m/z [M+H]⁺ = 605.

Step-4: N-[(3-chloro-4-fluorophenyl)(4-iodo-5-methyl-1-{[2-(trimethylsilyl)ethoxymethyl}-1H-imidazol-2-yl)methyl]-5-fluoro-6-methylpyridin-2-amine (5 g, 8.27 mmol) was added in a Parr autoclave vessel and dissolved with MeOH (150 mL) and degassed with argon. To the reaction mixture triethylamine (2.88 mL, 20.663 mmol) and PdCl₂(dppf)·CH₂Cl₂ (0.337 g, 0.413 mmol) were added and the vessel was put in the autoclave at 90 °C and 10.3 bar CO gas pressure for 16 h. The reaction mixture was concentrated to obtain a solid, which was then dissolved in ethyl acetate and the mixture was filtered through a celite bed. The filtrate was concentrated to get the crude product which was purified by column chromatography (SiO₂, 0-20% EtOAc/Hex) to obtain the title compound. (3.4 g, 77%). LCMS: m/z [M+H]⁺ = 537.

**Intermediate 27:** (3-chloro-4-fluorophenyl)(4-iodo-5-methyl-1-((2-(trimethylsilyl)ethoxy)methyl)-1H-imidazol-2-yl)methyl diisopropylcarbamate

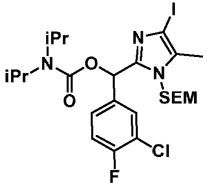

The compound was prepared as described in the synthesis of intermediate 26 (step 1 and step 2).

### Intermediate 28: 4-(methoxymethyl)-1-((2-(trimethylsilyl)ethoxy)methyl)-1H-imidazole

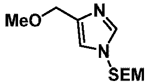

Step 1: To a solution of 1H-imidazol-4-ylmethanol hydrochloride (9 g, 66.88 mmol) in DMF (100 mL) were added DIPEA (29.1 mL, 167.2 mmol) and SEM-Cl (13.0 mL, 73.57 mmol) at 0 °C and the reaction mixture was stirred at rt for 16 h. The reaction mixture was quenched with ice cold water (100 mL) and extracted with DCM (2 × 100 mL). The combined organic layers were washed with brine (100 mL), dried over Na₂SO₄ and concentrated under reduced pressure to afford the crude product which was purified by column chromatography (SiO₂, 5% MeOH/DCM) to afford (1-((2-(trimethylsilyl)ethoxy)methyl)-1H-imidazol-4-yl)methanol. Yield: 43% (6.6 g). LCMS: m/z [M+H]⁺ = 229.

Step 2: To a solution of (1-((2-(trimethylsilyl)ethoxy)methyl)-1H-imidazol-4-yl)methanol (6.6 g, 28.9 mmol) in THF (120 mL) was added NaH (60%, 1.39 g, 57.8 mmol) portionwise at 0 °C and the mixture was then stirred at this temperature for 30 min followed by addition of iodomethane (2.7 mL, 43.35 mmol). The mixture was then stirred at rt for 3 h. The reaction was quenched with cold NH₄Cl aqueous solution (100 mL) and extracted with EtOAc (2 × 200 mL). The combined organic layers were washed with brine (300 mL), dried over Na₂SO₄ and concentrated under reduced pressure to afford the title compound (Yield: 10 g (crude). LCMS: m/z [M+H]⁺ = 243.

### Intermediate 29: methyl 2-((3-chloro-4-fluorophenyl)((5-fluoro-6-methylpyridin-2-yl)amino)methyl)-5-(methoxymethyl)-1H-imidazole-4-carboxylate

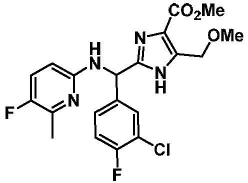

Step 1 was carried out in analogy to the synthesis of intermediate 26, step 2 using intermediate 28.

Step 2: To a solution of (3-chloro-4-fluorophenyl)(4-(methoxymethyl)-1-((2-(trimethylsilyl)ethoxy)methyl)-1H-imidazol-2-yl)methyl diisopropylcarbamate (10 g, 18.93 mmol) in MeCN (250 mL) was added NIS (12.78 g, 56.8 mmol) at 0 °C and the mixture was heated to 80 °C for 6 h. The reaction mixture was concentrated under reduce pressure, diluted with EtOAc (300 mL), washed with water (200 mL), saturated sodium thiosulphate solution (200 mL) and brine (200 mL). The organic layer was dried over Na₂SO₄ and concentrated under reduced pressure to afford the crude product which was purified by column chromatography (SiO₂, 10% EtOAc/Hex) to afford (3-chloro-4-fluorophenyl)(5-iodo-4-(methoxymethyl)-1-((2-(trimethylsilyl)ethoxy)methyl)-1H-imidazol-2-yl)methyl diisopropylcarbamate. Yield: 32% (4 g). LCMS: m/z [M+H]⁺

Step 3 was carried out in analogy to the synthesis of intermediate 26, step 3.

Step 4: A solution N-((3-chloro-4-fluorophenyl)(5-iodo-4-(methoxymethyl)-1H-imidazol-2-yl)methyl)-5-fluoro-6-methylpyridin-2-amine (2 g, 3.96 mmol) in MeOH (40 mL) was degassed with argon for 15 min followed by the addition of TEA ( 2.8 mL, 19.8 mmol), dppf (220 mg, 0.396 mmol) and PdCl₂(dppf)·DCM (162 mg, 0.198 mmol). The reaction mixture was stirred at 70 °C under 120 psi pressure of carbon monoxide for 16 h in an autoclave reactor. The reaction mixture was filtered through a celite bed and washed with MeOH (50 mL). The filtrate was concentrated under reduce pressure, diluted with EtOAc (200 mL) and washed with water (100 mL) and brine (100 mL). The organic layer was dried over Na₂SO₄ and concentrated under reduced pressure to afford the crude product which was purified by column chromatography (SiO₂, 30% EtOAc/Hex) to yield the title compound. Yield: 69% (1.2 g). LCMS: m/z [M+H]⁺ = 437.

### Intermediates 30-31

The title compound was prepared using an analogous method to that described for intermediate 29.

| Intermediate | Structure | Name/Data |
|---|---|---|
| 30 | 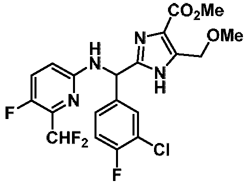 | methyl 2-((3-chloro-4-fluorophenyl)((6-(difluoromethyl)-5-fluoropyridin-2-yl)amino)methyl)-5-(methoxymethyl)-1H-imidazole-4-carboxylate. |
| | | Yield: 160 mg (impure). |
| | | LCMS: m/z [M+H]⁺ = 473. |
| 31 | 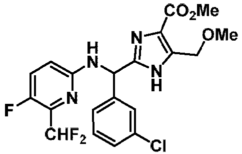 | methyl 2-((3-chlorophenyl)((6-(difluoromethyl)-5-fluoro-pyridin-2-yl)amino)methyl)-5-(methoxymethyl)-1H-imidazole-4-carboxylate. |
| | | Yield: 47% (75 mg). |
| | | LCMS: m/z [M+H]⁺ = 455. |

### Intermediate 32: 6-(difluoromethyl)-5-fluoropyridin-2-amine

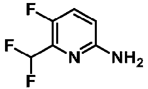

Step 1: To a solution of 6-chloro-3-fluoropyridine-2-carbaldehyde (3.0 g, 18.8 mmol) in DCM (50 mL) was added DAST (4.47 mL, 33.84 mmol) at - 30 °C dropwise and the reaction mixture was gradually warmed to rt. The reaction mixture was stirred at rt for 4 h. The reaction mixture was quenched with NaHCO₃ solution (100 mL) at 0 °C and diluted with DCM (200 mL). The organic layer was separated and washed with water (100 mL) and brine (100 mL). The organic layer was dried over Na₂SO₄ and concentrated under reduced pressure to afford the crude product which was purified by column chromatography (SiO₂, 5% EtOAc/Hex) to afford 6-chloro-2-(difluoromethyl)-3-fluoropyridine. Yield: 73% (2.5 g).

Step 2: A mixture of 6-chloro-2-(difluoromethyl)-3-fluoropyridine (3.0 g, 16.52 mmol), diphenylme-thanimine (5.5 mL, 33.05 mmol) and Cs₂CO₃ (10.8 g, 33.05 mmol) in 1,4-dioxane (60 mL) was degassed with argon for 15 minutes followed by the addition of BINAP (1029 mg, 1.65 mmol), Pd(OAc)₂ (371 mg, 1.65 mmol) and the reaction mixture was then heated to 100 °C for 16 h. The reaction mixture was filtered, the filter was washed with EtOAc (100 mL) and the filtrate was concentrated under reduced pressure, diluted with water (100 mL) and extracted with EtOAc (2 × 100 mL). The combined organic layers were washed with brine (100 mL), dried over Na₂SO₄ and concentrated under reduced pressure to afford the crude product which was purified by column chromatography (SiO₂, 2% EtOAc/Hex) to afford 6-(difluoromethyl)-N-(diphenylmethylidene)-5-fluoro-pyridin-2-amine. Yield: 78% (4.2 g). LCMS: m/z [M+H]⁺ = 327.

Step 3: To a solution of 6-(difluoromethyl)-N-(diphenylmethylidene)-5-fluoropyridin-2-amine (4.2 g, 12.87 mmol) in THF (60 mL) was added 1N HCl (60 mL) at 0 °C and the mixture was stirred at rt for 16 h. The reaction mixture was concentrated under reduced pressure to obtain a residue which was basified with saturated NaHCO₃ solution and extracted with EtOAc (2 × 100 mL). The combined organic layers were washed with brine (100 mL), dried over Na₂SO₄ and concentrated under reduced pressure to afford the crude product which was purified by column chromatography (SiO₂, 30% EtOAc/Hex) to afford the title compound. Yield: 76% (1.6 g). LCMS: m/z [M+H]⁺ = 163.

### Intermediate 33: 5-fluoro-6-methoxypyridin-2-amine

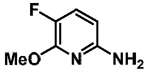

Step 1: To a solution of 2-chloro-5-fluoropyridine (10.0 g, 76.03 mmol) in DCM (50 mL) was added m-CPBA (77%, 20.5 g, 91.23 mmol) and the mixture was heated to 65 °C for 16 h. The reaction mixture was cooled to 0 °C and saturated sodium thiosulfate aqueous solution was added. The obtained mixture was stirred for 20 minutes. A saturated sodium bicarbonate solution was added to the reaction solution, and the mixture was extracted with chloroform. The obtained organic layer was dried over magnesium sulfate and concentrated under reduced pressure to get the crude product, which was purified by column chromatography (SiO₂, 0-80% EtOAc/Hex) to afford 2-chloro-5-fluoropyridine 1-oxide (4.0 g, 35%).

Step 2: To a stirred solution of 2-chloro-5-fluoropyridine 1-oxide (4.0 g, 27.11 mmol) in THF (30 mL) were added TEA (11.41 mL, 81.34 mmol) and TFAA (4.53 mL, 32.53 mmol) and the mixture was stirred at rt for 16 h. A small amount of water was added to the reaction solution. Thereafter, a 5 N sodium hydroxide aqueous solution was added to the mixture, while the solution was stirred under cooling. The obtained mixture was stirred at rt for 1 h. The mixture was acidified by the addition of acetic acid and was then extracted with EtOAc (100 mL). The organic layer was washed with brine (75 mL), then dried over magnesium sulfate and concentrated under reduced pressure to get the crude product which was purified by column chromatography (SiO₂, 0-20% EtOAc/Hex) to yield 6-chloro-3-fluoropyridin-2(1H)-one (2.0 g, 50%). LCMS: m/z [M+H]⁺ = 148.

Step 3: To a solution of 6-chloro-3-fluoropyridin-2(1H)-one (3.0 g, 20.33 mmol) in CHCl₃ (40 mL) were added Ag₂CO₃ (7.1 g, 30.5 mmol) and methyl iodide (2.6 ml, 40.67 mmol) and the mixture was stirred at rt for 16 h. The reaction mixture was filtered through celite, the celite was washed with EtOAc (100 mL) and concentrated to get the crude compound which was purified by column chromatography (SiO₂, 0-100% EtOAc/Hex) to afford 6-chloro-3-fluoro-2-methoxypyridine (1.4 g, 42%).

Step 3: To a solution of 6-chloro-3-fluoro-2-methoxypyridine (2.7 g, 14.24 mmol) in 1,4-dioxane (30 mL) were added tert-butyl carbamate (8.4 g, 71.18 mmol) and potassium triphosphate (3.9 g, 28.47 mmol) and the mixture was degassed with argon, followed by the addition of Xanthphos (820 mg, 1.42 mmol) and Pd₂dba₃ (0.65 g, 0.71 mmol). The mixture was heated to 110 °C for 16h. The reaction mixture was concentrated under reduced pressure to get a residue which was diluted with ethyl acetate (100 mL). The organic part was washed with water (100 mL) and brine (50 mL) and was dried over anhydrous sodium sulfate. The solvent was concentrated under reduced pressure to afford the crude product, which was purified by column chromatography (SiO₂, 0-20% EtOAc/Hex) to afford tert-butyl (5-fluoro-6-methoxypyridin-2-yl)carbamate (1.5 g, 43%). LCMS: m/z [M+H]⁺ = 243.

Step 4: A solution of tert-butyl (5-fluoro-6-methoxypyridin-2-yl)carbamate (3.0 g, 12.33 mmol) in 30% TFA in DCM (30 mL) and stirred at rt for 16 h. The reaction mixture was poured on crushed ice, neutralized by addition of saturated NaHCO₃ (50 mL) and extracted with DCM (2 × 75 mL). The organic layer was washed with brine (50 mL) and dried over sodium sulfate. The solvent was evaporated under reduced pressured to get the crude product, which was purified by column chromatography (SiO₂, 0-30% EtOAc/Hex) to obtain the title compound (1.6 g, 91%). LCMS: m/z [M+H]⁺ = 143.

### Intermediate 34: (4-(benzylthio)-5-methyl-1-((2-(trimethylsilyl)ethoxy)methyl)-1H-imidazol-2-yl)(3-chloro-4-fluorophenyl)methyl diisopropylcarbamate

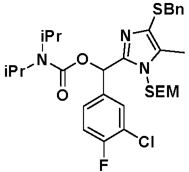

Step 1: A solution of 4-iodo-5-methyl-1-{[2-(trimethylsilyl)ethoxy]methyl}-1H-imidazole (intermediate 36, 10 g, 30.84 mmol) in 1,4-dioxane (150 mL) was degassed with argon for 10 minutes followed by the addition of benzyl mercaptan (7.23 mL, 61.68 mmol), Xanthphos (1.78 g, 3.08 mmol) and Pd₂(dba)₃ (1.7 g, 1.85 mmol) at rt. The reaction mixture was heated to 110 °C for 16 h. The reaction mixture was cooled to rt, filtered and concentrated. The obtained residue was diluted with water and extracted with EtOAc (2 × 200 mL). The combined organic layers were washed with brine (100 mL), dried over Na₂SO₄ and concentrated under reduced pressure to get the crude product which was purified by column chromatography (SiO₂, 40% EtOAc/Hex) to afford 4-(benzylsulfanyl)-5-methyl-1-{[2-(trimethylsilyl)ethoxy]methyl}-1H-imidazole (regeoisomeric mixture). Yield: 61% (6.0 g). LCMS: m/z [M+H]⁺ = 321.

Step 2 was carried out in analogy to the synthesis of intermediate 26, step 2.

Yield: 60% (1.7 g). LCMS: m/z [M+H]⁺ = 606.

**Intermediate 35:** methyl 2-((3-chloro-4-fluorophenyl)(((trans)-4-(trifluoromethyl)cyclohexyl)oxy)methyl)-5-methyl-1-((2-(trimethylsilyl)ethoxy)methyl)-1H-imidazole-4-carboxylate

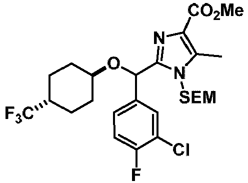

Step 1: To a solution of (3-chloro-4-fluorophenyl)(4-iodo-5-methyl-1-{[2-(trimethylsilyl)ethoxy]methyl}-1H-imidazol-2-yl)methyl N,N-bis(propan-2-yl)carbamate (intermediate 27, 1 g, 1.6 mmol) and (trans)-4-(trifluoromethyl)cyclohexan-1-ol (1.1 g, 6.41 mmol) in THF (20 mL) was added boron trifluoride etherate (0.3 mL, 2.4 mmol) followed by TFA (0.55 mL, 7.21 mmol) and the mixture was then stirred at 70 °C for 2 h. The reaction mixture was concentrated under reduced pressure, the residue was diluted with ice-cold water (50 mL) and extracted with EtOAc (2 × 40 mL). The combined organic layers were washed with sodium bicarbonate solution (50 ml) and brine (50 mL), dried over anhydrous Na₂SO₄, filtered and concentrated under reduced pressure to get the crude product which was purified by column chromatography (SiO₂, 10% EtOAc/Hex) to afford 2-((3-chloro-4-fluorophenyl)(((trans)-4-(trifluoromethyl)cyclohexyl)oxy)methyl)-4-iodo-5-methyl-1-((2-(trimethylsilyl)ethoxy)methyl)-1H-imidazole. Yield: 53% (550 mg, 0.856 mmol). LCMS: m/z [M+H]⁺ = 647.

Step 2: To a solution of 2-((3-chloro-4-fluorophenyl)(((trans)-4-(trifluoromethyl)cyclohexyl)oxy)methyl)-4-iodo-5-methyl-1-((2-(trimethylsilyl)ethoxy)methyl)-1H-imidazole (400 mg, 0.62 mmol) in MeOH (20 mL) were added TEA (0.5 mL, 3.09 mmol) and Pd(OAc)₂ (30 mg, 0.12 mmol) and the mixture was stirred at 80 °C under 120 psi CO pressure in an autoclave for 16 h. The reaction mixture was filtered through a celite pad, evaporated to dryness and the residue was purified by column chromatography (SiO₂, 10-20% EtOAc/Hex) to afford the title compound. Yield: 90% (325 mg). LCMS: m/z [M+H]⁺ = 579.

### Intermediate 36: 4-iodo-5-methyl-1-((2-(trimethylsilyl)ethoxy)methyl)-1H-imidazole

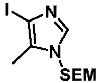

To a stirred suspension of 4-iodo-5-methyl-1H-imidazole (40 g, 480.77 mmol) in toluene (400 mL) was added DIPEA (67 mL, 385 mmol) followed by SEM-Cl (41 mL, 230.769 mmol) at rt. The resulting mixture was heated to 90 °C for 16 h. The reaction mixture was cooled and water was added (250 mL). The organic layer was concentrated under reduced pressure, the residue was diluted with ethyl acetate (600 mL) and washed with H₂O (2 × 150 mL) and brine (150 mL), dried over Na₂SO₄ and concentrated under reduced pressure to afford 4-iodo-5-methyl-1-{[2-(trimethylsilyl)ethoxy]methyl}-1H-imidazole (62 g, 95%). LCMS: m/z [M+H]⁺ = 339.

### Example 1: 1-(2-(bis(3-chloro-4-fluorophenyl)methyl)-1H-imidazol-4-yl)ethan-1-one

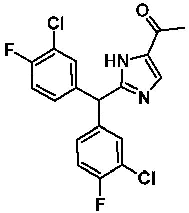

A solution of 2-(bis(3-chloro-4-fluorophenyl)methyl)-4-iodo-1H-imidazole (intermediate 4, 0.1 g, 0.215 mmol) in 1,4-dioxane (2.0 mL) was degassed with argon for 10 min followed by the addition of tributyl(1-ethoxyvinyl)tin (0.1 ml, 0.323 mmol) and Pd(PPh₃)₂Cl₂ (0.08 mg, 0.011 mmol) at rt. The reaction mixture was heated to 100 °C for 16 h in a sealed tube. The reaction mixture was diluted with water and extracted with ethyl acetate (2 x 50 mL). The combined organic layers were washed with brine and dried over sodium sulphate. The combined organic part was concentrated under reduced pressure. The resulting crude mixture was dissolved in THF (2 mL) and 1N HCl (2 mL) was added to the reaction mixture at rt. The reaction mixture was stirred at rt for 2 h. The reaction mixture was neutralized with saturated NaHCO₃ solution and extracted with ethyl acetate (2 x 50 mL). The combined organic layers were washed with brine, dried over sodium sulphate and concentrated under reduced pressure to get the crude product which was purified by reverse-phase prep HPLC to yield the title compound (0.022 g, 27%). LCMS m/z = 379 [M+H]⁺; ¹H-NMR (400 MHz, DMSO-d₆): 12.91 (s, 1H), 7.87 (s, 1H), 7.54 (d, 2H), 7.40-7.33 (m, 4H), 5.67 (s, 1H), 2.37 (s, 3H).

### Example 2: 2-(bis(3-chloro-4-fluorophenyl)methyl)-1H-imidazole-4-carboxamide

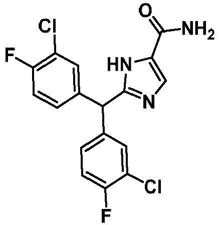

To a solution of methyl 2-(bis(3-chloro-4-fluorophenyl)methyl)-1H-imidazole-5-carboxylate (intermediate 9, 0.07 g, 0.176 mmol) in THF (1.0 mL) was added NH₃ solution (7M in MeOH, 8.0 mL) at rt. Th reaction mixture was heated to 110 °C for 24 h in a sealed tube. The reaction mixture was cooled to rt and the solvent was evaporated under reduced pressure to get the crude product, which was purified by reverse-phase prep HPLC to yield the title compound (0.033 g, 49%). LCMS m/z = 382 [M+H]⁺; ¹H-NMR (400 MHz, DMSO-d₆): 12.15 (s, 1H), 7.53-7.50 (m, 3H), 7.34-7.31 (m, 4H), 7.12-6.90 (m, 1H), 6.81-6.73 (m, 1H), 5.64 (s, 1H).

### Example 3: 2-(bis(3-chloro-4-fluorophenyl)methyl)-N-methyl-1H-imidazole-4-carboxamide

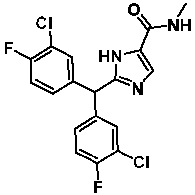

To a stirred solution of 2-(bis(3-chloro-4-fluorophenyl)methyl)-1H-imidazole-4-carboxylic acid (intermediate 25, 50 mg, 0.13 mmol) in DMF were added methylamine (1.5 eq.), T3P (3.0 eq.) and DIPEA (3.5 eq.) and the reaction was heated to 60 °C for 16 hours. The mixture was purified by preparative-HPLC to yield the title compound. LCMS m/z = 396 [M+H]⁺; retention time: 2.91 min, method B.

### Examples 4-12.

The title compounds were prepared using an analogous method to that described for Example 3.

| Example | Structure | Name | Data |
|---|---|---|---|
| **4** | 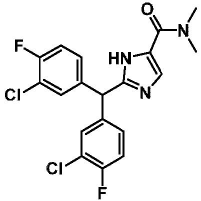 | 2-(bis(3-chloro-4-fluorophenyl)methyl)-N,N-dimethyl-1H-imidazole-5-carboxamide | LCMS m/z = 410 [M+H]⁺; retention time: 7.52 min, method C |
| **5** | 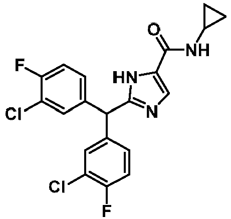 | 2-(bis(3-chloro-4-fluorophenyl)methyl)-N-cyclopropyl-1H-imidazole-5-carboxamide | LCMS m/z = 422 [M+H]⁺; retention time: 3.03 min, method B |
| **6** | 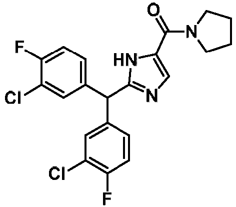 | (2-(bis(3-chloro-4-fluorophenyl)methyl)-1H-imidazol-5-yl)(pyrrolidin-1-yl)methanone | LCMS m/z = 436 [M+H]⁺; retention time: 7.69 min, method C |
| **7** | 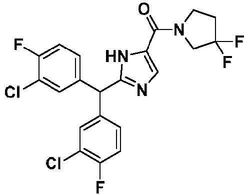 | (2-(bis(3-chloro-4-fluorophenyl)methyl)-1H-imidazol-5-yl)(3,3-difluoro-pyrrolidin-1-yl)methanone | LCMS m/z = 472 [M+H]⁺; retention time: 3.24 min, method B |
| **8** | 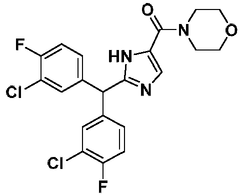 | (2-(bis(3-chloro-4-fluorophenyl)methyl)-1H-imidazol-5-yl)(morpholino)methanone | LCMS m/z = 452 [M+H]⁺; retention time: 7.48 min, method C |
| **9** | 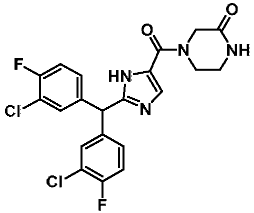 | 4-(2-(bis(3-chloro-4-fluorophenyl)methyl)-1H-imidazole-5-carbonyl)piperazin-2-one | LCMS m/z = 465 [M+H]⁺; retention time: 2.72 min, method B |
| **10** | 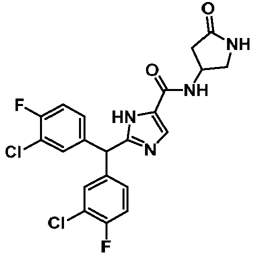 | 2-(bis(3-chloro-4-fluorophenyl)methyl)-N-(5-oxopyrrolidin-3-yl)-1H-imidazole-5-carboxamide | LCMS m/z = 465 [M+H]⁺; retention time: 2.63 min, method B |
| **11** | 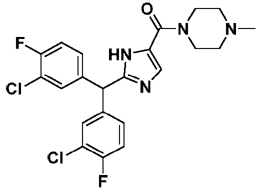 | (2-(bis(3-chloro-4-fluorophenyl)methyl)-1H-imidazol-5-yl)(4-methylpiperazin-1-yl)methanone | LCMS m/z = 465 [M+H]⁺; retention time: 2.83 min, method B |
| **12** | 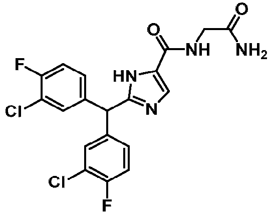 | N-(2-amino-2-oxoethyl)-2-(bis(3-chloro-4-fluorophenyl)methyl)-1H-imidazole-5-carboxamide | LCMS m/z = 439 [M+H]⁺; retention time: 2.66 min, method B |

### Example 13: 2-(bis(3-chloro-4-fluorophenyl)methyl)-1,5,6,7-tetrahydro-4H-imidazo[4,5-c]pyridin-4-one

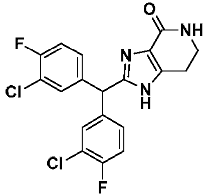

Step 1: To a solution of methyl 2-(bis(3-chloro-4-fluorophenyl)methyl)-5-iodo-1H-imidazole-4-carboxylate (intermediate 10, 0.20 g, 0.373 mmol) in DMSO (3.0 mL) and H₂O (0.5 mL) was added 4-(4,4,5,5-tetramethyl-1,3,2-dioxaborolan-2-yl)isoxazole (0.09 g, 0.448 mmol) at rt and the mixture was degassed with argon for 15 min. Then KF (0.065 g, 1.12 mmol) and PdCl₂(dppf)·DCM (0.028 g, 0.037 mmol) were added to the reaction mixture at rt. The reaction mixture was heated to 130 °C for 16 h in a sealed tube. The reaction mixture was filtered through a celite bed and the filtrate was diluted with ice water and extracted with ethylacetate (3×30 mL). The combined organic layers were washed with brine, dried over Na₂SO₄ and concentrated under reduced pressure. The residue was purified by column chromatography (SiO₂, 0-60% EtOAc/Hex) to yield methyl 2-(bis(3-chloro-4-fluorophenyl)methyl)-5-(cyanomethyl)-1H-imidazole-4-carboxylate (0.07 g, 42%). LCMS m/z = 436 [M+H]⁺.

Step 2: To a solution of methyl 2-(bis(3-chloro-4-fluorophenyl)methyl)-5-(cyanomethyl)-1H-imidazole-4-carboxylate (0.2 g, 0.47 mmol), MeOH (15.0 mL) and aq. NH₃ (1 mL) was added Raney Nickel (0.1 g) at rt. The reaction mixture was heated to 70 °C under 6.2 bar pressure of H₂ gas in an autoclave for 16 h. The reaction mixture was cooled to rt, filtered through a celite bed and the filtrate was concentrated under reduced pressure to get the crude product, which was purified by prep HPLC (reverse-phase) to yield the title compound (0.015 g, 7%). LCMS m/z = 408 [M+H]⁺; ¹H-NMR (400 MHz, DMSO-d₆): 12.83 (s, 1H), 7.55-7.53 (m, 2H), 7.40-7.31 (m, 4H), 7.19 (bs, 1H), 5.61 (s, 1H), 3.40-3.67 (m, 2H), 2.75-2.72 (m, 2H).

### Example 14: 2-(bis(3-chloro-4-fluorophenyl)methyl)-5,6,7,8-tetrahydroimidazo[4,5-c]azepin-4(1H)-one

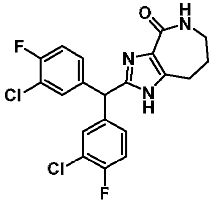

Step 1: To a solution of methyl 2-(bis(3-chloro-4-fluorophenyl)methyl)-5-iodo-1H-imidazole-4-carboxylate (intermediate 10, 0.5 g, 0.93 mmol) in DMF (5.0 mL) was added TEA (1.0 mL, 7.46 mmol) and the mixture was degassed with argon for 10 min. To this mixture were added acrylonitrile (0.5 g, 9.32 mmol), Pd(OAc)₂ (0.042 g, 0.187 mmol) and triphenylphosphine (0.15 g, 0.56 mmol) at rt. The reaction mixture was heated to 100 °C for 16 h in a sealed tube. The reaction mixture was cooled to rt, filtered through a celite bed and the filtrate was concentrated under reduced pressure to get the crude product, which was purified by combi flash column chromatography (SiO₂, 0-60% EtOAc/Hex) to get methyl (E)-2-(bis(3-chloro-4-fluorophenyl)methyl)-5-(2-cyanovinyl)-1H-imidazole-4-carboxylate (0.08 g, 19%). LCMS m/z = 448 [M+H]⁺.

Step 2: To a solution of methyl 2-(bis(3-chloro-4-fluorophenyl)methyl)-5-(cyanomethyl)-1H-imidazole-4-carboxylate (0.08 g, 0.18 mmol) in MeOH (10.0 mL) and ammonical methanol (7M, 1 mL) was added Raney Nickel (0.05 g) at rt. The reaction mixture was hydrogenated under 6.2 bar hydrogen pressure at 90 °C in an autoclave for 16 h. The reaction mixture was cooled to rt, filtered through a celite bed and the filtrate was concentrated under reduced pressure. The resulting crude product was purified by prep HPLC (reverse-phase) to yield the title compound (0.013 g, 17%). LCMS m/z = 422 [M+H]⁺; ¹H-NMR (400 MHz, DMSO-d₆): 12.43 (s, 1H), 7.74 (s, 1H), 7.55-7.53 (m, 2H), 7.39-7.33 (m, 4H), 5.62 (s, 1H), 3.21-3.18 (m, 2 H), 2.80 (t, 2H), 1.90-1.87 (m, 2H).

### Example 15: 2-(bis(3-chloro-4-fluorophenyl)methyl)-1,5-dihydro-4H-imidazo[4,5-c]pyridin-4-one

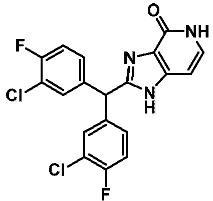

Step 1: To a stirred solution of 2-(bis(3-chloro-4-fluorophenyl)methyl)-N-(tert-butyl)-5-iodo-1H-imidazole-4-carboxamide (intermediate 12, 0.25 g, 0.44 mmol) in toluene (6 mL), ethanol (3 mL) and water (1 mL) was added K₂CO₃ (0.185 g, 1.33 mmol) and the mixture was degassed with argon for 10 min. After degassing, (E)-2-(2-ethoxyvinyl)-4,4,5,5-tetramethyl-1,3,2-dioxaborolane (0.132 g, 0.667 mmol) and Pd(PPh₃)₄ (0.026 g, 0.022 mmol) were added to the reaction mixture at rt. The reaction mixture was heated to 100 °C in a sealed tube for 16 h. The reaction mixture was cooled to rt, filtered through a celite bed and the filtrate was evaporated under reduced pressure to get the crude product, which was purified by column chromatography (SiO₂, 0-40% EtOAc/Hex) to yield 2-[bis(3-chloro-4-fluorophenyl)methyl]-N-tert-butyl-5-[(E)-2-ethoxyethenyl]-1H-imidazole-4-carboxamide (0.1 g, 45%); LCMS m/z = 508 [M+H]⁺.

Step 2: A solution of 2-[bis(3-chloro-4-fluorophenyl)methyl]-N-tert-butyl-5-[(E)-2-ethoxyethenyl]-1H-imidazole-4-carboxamide (0.1 g, 0.197 mmol) in TFA (1 mL) was heated to 120 °C for 1 h under microwave irradiation. The reaction mixture was concentrated under reduced pressure and the resulting residue was purified by reverse-phase prep HPLC to yield the title compound (0.024 g, 30%). LCMS m/z = 404 [M+H]⁺; ¹H-NMR (400 MHz, DMSO-d₆): 12.66 (s, 1H), 10.79 (s, 1H), 7.55 (d, 2H), 7.38-7.31 (m, 4H), 7.04 (d, 1H), 6.46 (d, 1H), 5.76 (s, 1H).

### Example 16: 2-(bis(3-chloro-4-fluorophenyl)methyl)-5-methyl-1H-imidazole-4-carboxamide

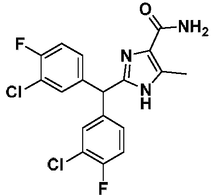

Step 1: To a solution of methyl 2-(bis(3-chloro-4-fluorophenyl)methyl)-5-iodo-1H-imidazole-4-carboxylate (intermediate 10, 0.25 g, 0.479 mmol) in 1,4-dioxane (10.0 mL) was added Cs₂CO₃ (0.32 g, 0.958 mmol) at rt. The mixture was degassed with argon for 10 min followed by the addition of trimethylboroxine (0.19 g, 1.437 mmol) and PdCl₂(dppf)·DCM (0.02 g, 0.024 mmol) at rt. The reaction mixture was heated to 90 °C for 16 h in a sealed tube. The reaction mixture was filtered through a celite bed sintered funnel and the filtrate was concentrated under reduced pressure to get the crude product, which was purified by column chromatography (SiO₂, 0-40% EtOAc/Hex) to yield methyl 2-[bis(3-chloro-4-fluorophenyl)methyl]-5-methyl-1H-imidazole-4-carboxylate (0.18 g, 91%). LCMS m/z = 411 [M+H]⁺.

Step 2: To a solution of methyl 2-[bis(3-chloro-4-fluorophenyl)methyl]-5-methyl-1H-imidazole-4-carboxylate (0.23 g, 0.56 mmol) in THF (1.5 mL) was added NH₃ solution (7M in MeOH, 10.0 mL) at rt. The reaction mixture was heated to 110 °C for 48 h in a sealed tube. The reaction mixture was cooled to rt and the solvent was evaporated under reduced pressure to get the crude product, which was purified by reverse-phase prep HPLC to afford the title compound (0.06 g, 27%). LCMS m/z = 396 [M+H]⁺; ¹H-NMR (400 MHz, DMSO-d₆): 7.57-7.55 (m, 2H), 7.38-7.34 (m, 4H), 7.08 (s, 1H), 6.89 (s, 1H), 5.55 (s, 1H), 3.38 (s, 3H).

### Example 17: 2-((3-chloro-4-fluorophenyl)((5-fluoro-6-methylpyridin-2-yl)amino)methyl)-N-((3S,4R)-4-hydroxypyrrolidin-3-yl)-5-methyl-1H-imidazole-4-carboxamide

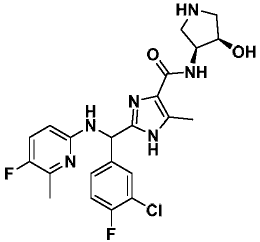

Step 1: To a stirred solution of methyl 2-[(3-chloro-4-fluorophenyl)[(5-fluoro-6-methylpyridin-2-yl)amino]methyl]-5-methyl-1-{[2-(trimethylsilyl)ethoxy]methyl}-1H-imidazole-4-carboxylate (intermediate 26, 6 g, 11.117 mmol) in MeOH, THF and H₂O (1:1:1, 150 mL) was added LiOH·H₂O (1.41 g, 33.52 mmol). The reaction mixture was stirred at rt for 16 h. The reaction mixture was concentrated, diluted with H₂O (50 mL) and acidified with NaHSO₄ solution. The reaction mixture was extracted with ethyl acetate (2 × 100 mL). The combined organic layers were dried over Na₂SO₄ and concentrated under reduced pressure to obtain 2-[(3-chloro-4-fluorophenyl)[(5-fluoro-6-methylpyridin-2-yl)amino]methyl]-5-methyl-1-{[2-(trimethylsilyl)ethoxy]methyl}-1H-imidazole-4-carboxylic acid (5.2 g, 89%). LCMS m/z = 523 [M+H]⁺.

Step 2: To a stirred solution of 2-[(3-chloro-4-fluorophenyl)[(5-fluoro-6-methylpyridin-2-yl)amino]methyl]-5-methyl-1-{[2-(trimethylsilyl)ethoxy]methyl}-1H-imidazole-4-carboxylic acid (0.7 g, 1.34 mmol) in DMF (5.0 mL) were added EDC·HCl (0.4 g, 2.01 mmol), HOBT (0.271 g, 2.01 mmol), DIPEA (0.7 mL, 4.01 mmol) and tert-butyl (3S,4R)-3-amino-4-hydroxypyrrolidine-1-carboxylate (272.02 mg, 1.34 mmol). The reaction mixture was stirred at rt for 16 h. The reaction was mixture diluted with EtOAc (70 mL), washed with H₂O (2 × 30 mL) and brine (30 mL). The organic layer was dried over Na₂SO₄ and concentrated under reduced pressure to obtained the crude product which was purified by column chromatography (SiO₂, 0-100% EtOAc/Hex) to obtained tert-butyl (3S,4R)-3-{2-[(3-chloro-4-fluorophenyl)[(5-fluoro-6-methylpyridin-2-yl)amino]methyl]-5-methyl-1-{[2-(trimethylsilyl)ethoxy]methyl}-1H-imidazole-4-amido}-4-hydroxypyrrolidine-1-carboxylate (0.83 g, 88%). LCMS m/z = 708 [M+H]⁺.

The diastereomeric mixture was separated via chiral SFC (Column: Chiralpak-IG (4.6 × 250 mm), 5 µm, Mobile phase: 80% CO₂ + 20% of 0.2% iPrNH₂ in iPrOH; Flow: 3 mL/min; % of Co-solvent: 20%; ABPR: 100 bar; T: 35 °C) to yield Peak 1, Rt = 3.993 min and Peak 2, Rt = 5.792 min.

Both peaks were individually processed according to the procedure below for Peak 1.

Step 3: To tert-butyl (3S,4R)-3-(2-((R)-(3-chloro-4-fluorophenyl)((5-fluoro-6-methylpyridin-2-yl)amino)methyl)-5-methyl-1-((2-(trimethylsilyl)ethoxy)methyl)-1H-imidazole-4-carboxamido)-4-hydroxypyr-rolidine-1-carboxylate (Peak 1, 0.3 g, 0.424 mmol) was added 50% TFA-DCM (4.0 mL). The reaction mixture was stirred at rt for 16 h. The reaction mixture was concentrated under reduced pressure and purified by prep HPLC to afford the title compound (0.1 g, 49%). LCMS m/z = 477 [M+H]⁺. ¹H-NMR (400 MHz, DMSO-d₆): 12.25 (brs, 1H), 7.61 (d, 1H), 7.45 (d, 1H), 7.38-7.28 (m, 4H), 6.53 (d, 1H), 6.19 (d, 1H), 5.21 (d, 1H), 4.00-3.97 (m, 2H), 3.09-2.99 (m, 2H), 2.64-2.61 (m, 1H), 2.44-2.40 (m, 4H), 2.20 (s, 3H). Column: (R,R) WHELK 01 (4.6 × 250 mm), 5 µm, Mobile phase: 70% CO₂ + 30% of 0.3% iPrNH₂ in iPrOH-MeCN (1:1); Flow: 3 mL/min; % of Co-solvent: 30%; ABPR: 100 bar; T: 35 °C; **Example 17a:** Rt = 5.9 min.

Peak 2 was processed in analogy to yield 50 mg (55%) of the title compound. LCMS m/z = 477. ¹H-NMR (400 MHz, DMSO-d₆): 12.25 (brs,1H), 7.61 (d, 1H), 7.45 (d, 1H), 7.38-7.28 (m, 4H), 6.53 (d, 1H), 6.53 (d, 1H), 6.19 (d, 1H), 5.21 (d, 1H), 4.00-3.97 (m, 2H), 3.09-2.99 (m, 2H), 2.64-2.61 (m, 1H), 2.44-2.40 (m, 4H), 2.20 (s, 3H). Column: (R,R) WHELK 01 (4.6 × 250 mm), 5 µm, Mobile phase: 70% CO₂ + 30% of 0.3% iPrNH₂ in iPrOH-MeCN (1:1); Flow: 3 mL/min; % of Co-solvent: 30%; ABPR: 100 bar; T: 35 °C; **Example 17b;** Rt = 7.29 min.

### Example 18: 2-((3-chloro-4-fluorophenyl)((5-fluoro-6-methylpyridin-2-yl)amino)methyl)-5-methyl-1H-imidazole-4-carboxylic acid

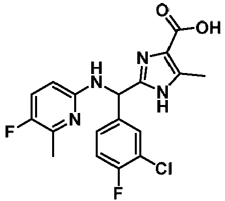

Step 1: To a stirred solution of 2-[(3-chloro-4-fluorophenyl)[(5-fluoro-6-methylpyridin-2-yl)amino]methyl]-5-methyl-1-{[2-(trimethylsilyl)ethoxy]methyl}-1H-imidazole-4-carboxylic acid (0.4 g, 0.765 mmol) in DCM (3 mL) was added TFA (1.8 mL, 22.94 mmol). The reaction mixture was stirred at rt for 16 h. The reaction mixture was concentrated under reduced pressure and purified via prep HPLC to obtain the title compound (0.15 g, 49%). LCMS m/z = 393 [M+H]⁺. The enantiomers were separated via chiral SFC.

Yield: 29%, LCMS m/z = 393 [M+H]⁺. ¹H-NMR (400 MHz, DMSO-d₆, 100 °C): 12.06 (brs, 1H), 7.62 (d, 1H), 7.41 (brs, 1H), 7.31-7.22 (m, 2H), 6.94 (d, 1H), 6.53 (d, 1H), 6.23 (brs, 1H), 2.37 (s, 3H), 2.24 (s, 3H). Chiral HPLC: Column: Chiralpak-IG (4.6 × 250 mm), 5 µm, T= ambient, Mobile phase: Hexane/EtOH/TFA: 95/05/0.1; Flow: 1 mL/min; **Example 18a:** Rt = 10.06 min. **Example 18b:** Rt = 12.940 min.

### Example 19: methyl 2-(bis(4-chloro-2-methoxyphenyl)methyl)-5-methyl-1H-imidazole-4-carboxylate

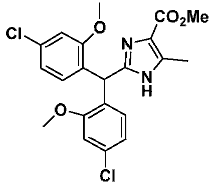

To a stirred solution of methyl 2-(bis(4-chloro-2-methoxyphenyl)methyl)-4-methyl-1-((2-(trimethylsilyl)ethoxy)methyl)-1H-imidazole-5-carboxylate (intermediate 23, 150 mg, 0.265 mmol) in DCM (2 mL) was added TFA (2 mL) at 0 °C. The resulting solution was stirred at rt for 2 h. The reaction mixture was concentrated. The obtained residue was purified by reverse phase prep HPLC to afford the title compound. Yield: 57% (64 mg). LCMS m/z = 435 [M+H]⁺. ¹H-NMR (400 MHz, DMSO-d₆): 12.66-12.26 (m, 1H), 7.05-7.04 (m, 2H), 6.96-6.91 (m, 2H), 6.80-6.76 (m, 2H), 5.98-5.90 (m, 1H), 3.72 (m, 9H), 2.35-2.17 (m, 3H).

### Example 20: 2-(bis(4-chloro-2-methoxyphenyl)methyl)-5-methyl-1H-imidazole-4-carboxylic acid

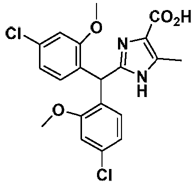

Step 1: To a stirred solution of methyl 2-[bis(4-chloro-2-methoxyphenyl)methyl]-4-methyl-1-{[2-(trimethylsilyl)ethoxy]methyl}-1H-imidazole-5-carboxylate (intermediate 23, 300 mg, 0.53 mmol) in MeOH: water: THF (9 mL, 1:1:1) was added LiOH·H₂O (70 mg, 1.59 mmol) at rt. The resulting solution was stirred at rt for 16 h. The reaction mixture was concentrated. The obtained residue was diluted with water (10 mL) and was acidified using NaHSO₄ solution. The acidic aqueous layer was extracted with ethyl acetate (3 × 20 mL). The combined organic layer were washed with brine, dried over anhydrous sodium sulfate and concentrated under reduced pressure to afford the crude product, which was triturated with pentane to afford 2-(bis(4-chloro-2-methoxyphenyl)methyl)-4-methyl-1-((2-(trimethylsilyl)ethoxy)methyl)-1H-imidazole-5-carboxylic acid. Yield: 85% (250 mg). LCMS m/z = 565 [M+H]⁺.

To a stirred solution 2-(bis(4-chloro-2-methoxyphenyl)methyl)-4-methyl-1-((2-(trimethylsilyl)ethoxy)methyl)-1H-imidazole-5-carboxylic acid (200 mg, 0.363 mmol) in DCM (2 mL) was added TFA (2 mL) at 0 °C. The resulting solution was stirred at rt for 2 h. The reaction mixture was concentrated. The obtained residue was purified by reverese phase prep HPLC to afford the title compound. Yield: 48% (76 mg). LCMS m/z = 435 [M+H]⁺. ¹H-NMR (400 MHz, DMSO-d₆, 100 °C): 12.08-11.85 (m, 1H), 7.02-6.92 (m, 6H), 6.02-5.96 (m, 1H), 3.73 (s, 6H), 2.37-2.29 (m, 3H).

### Example 21: 2-(bis(4-chloro-2-methoxyphenyl)methyl)-N-((3S,4R)-4-hydroxypyrrolidin-3-yl)-5-methyl-1H-imidazole-4-carboxamide

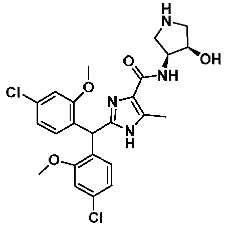

Step 1: To a stirred solution of 2-(bis(4-chloro-2-methoxyphenyl)methyl)-4-methyl-1-((2-(trimethylsilyl)ethoxy)methyl)-1H-imidazole-5-carboxylic acid (see example 20, step 1) (150 mg, 0.27 mmol) in DMF (3 mL) were added EDC·HCl (80 mg, 0.41 mmol), HOBT (55 mg, 0.41 mmol), DIPEA (0.14 mL, 0.82 mmol) and tert-butyl (3R,4S)-3-amino-4-hydroxypyrrolidine-1-carboxylate (83 mg, 0.41 mmol) at 0 °C. The resulting reaction mixture was stirred at rt for 16 h. The reaction mixture was diluted with EtOAc (90 mL) and was washed with cold water (2 × 30 mL) followed by brine (30 mL), dried over anhydrous sodium sulfate and concentrated under reduced pressure to afford tert-butyl (3R,4S)-3-{2-[bis(4-chloro-2-methoxyphenyl)methyl]-5-methyl-1-{[2-(trimethylsilyl)ethoxy]methyl}-1H-imidazole-4-amido}-4-hydroxypyrrolidine-1-carboxylate. Yield: 89% (180 mg). LCMS m/z = 565 [M+H]⁺.

Step 2: To a stirred solution of tert-butyl (3R,4S)-3-{2-[bis(4-chloro-2-methoxyphenyl)methyl]-5-methyl-1-{[2-(trimethylsilyl)ethoxy]methyl}-1H-imidazole-4-amido }-4-hydroxypyrrolidine-1-carboxylate (150 mg, 0.204 mmol) in DCM (2 mL) was added TFA (2 mL) at 0 °C. The resulting solution was stirred at rt for 16 h. The reaction mixture was concentrated. The obtained residue was purified by reverse phase prep HPLC to afford the title compound. Yield: 69% (69 mg). LCMS m/z = 505 [M+H]⁺. ¹H-NMR (400 MHz, DMSO-d₆, 100 °C): 11.59 (m, 1H), 7.37 (m, 1H), 7.02-6.93 (m, 6H), 5.96 (s, 1H), 4.79 (m, 1H, 4.03 (m, 2H), 3.74 (s, 6H), 3.08-3.06 (m, 2H), 2.70-2.67 (m, 1H), 2.56 (m, 1H), 2.39 (s, 3H).

### Example 22: (3-aminoazetidin-1-yl)(2-((3-chloro-4-fluorophenyl)((6-(difluoromethyl)-5-fluoropyridin-2-yl)amino)methyl)-5-(methoxymethyl)-1H-imidazol-4-yl)methanone

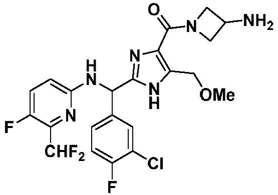

Step 1: To a solution of methyl 2-[(3-chloro-4-fluorophenyl)({[6-(difluoromethyl)-5-fluoropyridin-2-yl]amino})methyl]-4-(methoxymethyl)-1H-imidazole-5-carboxylate (intermediate 30) (1000 mg, 2.11 mmol) in a mixture of THF (5.0 mL), MeOH (5.0 mL) and H₂O (5.0 mL) was added LiOH·H₂O (888 mg, 21.15 mmol) at rt and the reaction mixture was heated to 70 °C for 16 h. The reaction mixture was concentrated under reduced pressure, diluted with water (20 mL) and washed with EtOAc (50 mL). The aqueous part was acidified with sodium hydrogen sulphate monohydrate up to pH~4 and extracted with EtOAc (100 mL). The organic layer was dried over sodium sulfate and concentrated under reduced pressure to get crude 2-[(3-chloro-4-fluorophenyl)({[6-(difluoromethyl)-5-fluoropyridin-2-yl]amino})methyl]-4-(methoxymethyl)-1H-imidazole-5-carboxylic acid. Yield: 760 mg (impure). LCMS m/z = 459 [M+H]⁺.

Step 2: To a solution of 2-((3-chloro-4-fluorophenyl)((6-(difluoromethyl)-5-fluoropyridin-2-yl)amino)methyl)-4-(methoxymethyl)-1H-imidazole-5-caiboxylic acid (400 mg, 0.87 mmol) in DMF (10 mL) were added DIPEA (1.5 mL, 8.72 mmol) and HATU (663 mg, 1.75 mmol) at 0 °C and the mixture was stirred for 15 min followed by the addition of tert-butyl azetidin-3-ylcarbamate·HCl (910 mg, 4.36 mmol). The mixture was then stirred at rt for 16 h. The reaction mixture was diluted with water (20 mL) and extracted with EtOAc (100 mL). The organic layer was dried over sodium sulphate and concentrated under reduced pressure to get the crude product which was purified by column chromatography (SiO₂, 90-95% EtOAc/Hex) to afford tert-butyl (1-(2-((3-chloro-4-fluorophenyl)((6-(difluoromethyl)-5-fluoropyridin-2-yl)amino)methyl)-4-(methoxymethyl)-1H-imidazole-5-carbonyl)azetidin-3-yl)carbamate. Yield: 460 mg (crude). LCMS m/z = 613 [M+H]⁺.

Step 3: To a solution of tert-butyl (1-(2-((3-chloro-4-fluorophenyl)((6-(difluoromethyl)-5-fluoropyridin-2-yl)amino)methyl)-4-(methoxymethyl)-1H-imidazole-5-carbonyl)azetidin-3-yl)carbamate (300 mg, 0.49 mmol) in DCM (30 mL) was added TFA (3.0 mL) at 0 °C and the mixture was then stirred at rt for 3 h. The reaction mixture was concentrated at low temperature under reduced pressure and diluted with methanol (10 mL). Then amberlyst A21 resin (100 mg) was added, the mixture was stirred at rt for 2 h. The reaction mixture was filtered and was washed with MeOH (30 mL). The filtrate was concentrated under reduced pressure to get the crude product which was purified by reverse phase prep HPLC to afford the racemic title compound. Yield: 48% (120 mg). The enantiomers were then separated via SFC. LCMS m/z = 513 [M+H]⁺. ¹H-NMR (400 MHz, DMSO-d₆): 12.58 (bs, 1H), 7.69-7.67 (m, 2H), 7.59-7.54 (m, 1H), 7.44-7.43 (m, 1H), 7.38-7.34 (m, 1H), 6.99-6.72 (m, 2H), 6.20-6.18 (m, 1H), 4.68-4.54 (m, 3H), 4.05-3.97 (m, 2H), 3.67-3.64 (m, 1H), 3.51 (brs, 1H), 3.23 (brs, 3H), 2.14-2.07 (m, 1H).

Chiral SFC: column: ChiralPak IG (4.6 × 150 mm), 5 µm, mobile phase 0.2 % iPrNH in iPrOH; flow: 3 mL/min; % of co-solvent: 30%; ABPR: 1500 psi; T: 35 °C; **Example 22a:** Rt = 2.932 min. **Example 22b:** Rt = 3.705 min.

### Examples 23-25.

The title compound was prepared using an analogous method to that described for intermediate 22.

| Example | Structure | Name/Data |
|---|---|---|
| **23a/23b** | 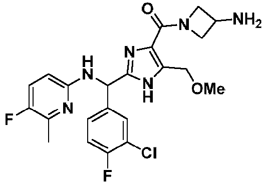 | (3-aminoazetidin-1-yl)(2-((3-chloro-4-fluorophenyl)((5-fluoro-6-methylpyridin-2-yl)amino)methyl)-5-(methoxymethyl)-1H-imidazol-4-yl)methanone, LCMS: m/z [M+H]⁺ = 477. ¹H-NMR (400 MHz, DMSO-d₆): 12.13 (bs, 1H), 7.64-7.62 (m, 1H), 7.42 (s, 1H), 7.31-7.22 (m, 2H), 6.84 (bs, 1H), 6.53-6.51 (m, 1H), 6.22-6.20 (m, 1H), 4.67 (m, 2H), 4.38 (brs, 1H), 3.71 (m, 2H), 3.27 (s, 3H), 2.24 (m, 3H). Chiral SFC: column: ChiralPak IC (4.6 × 250 mm), 5 µm, mobile phase 0.3% iPrNH₂ in iPrOH; flow: 3 mL/min; % of co-solvent: 30%; ABPR: 1500 psi; T: 35 °C; **Example 23a:** Rt = 5.42 min. **Example 23b:** Rt = 6.51 min. |
| **24** | 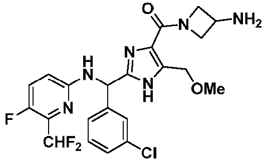 | (3-aminoazetidin-1-yl)(2-((3-chlorophenyl)((6-(difluoromethyl)-5-fluoropyridin-2-yl)amino)methyl)-5-(methoxymethyl)-1H-imidazol-4-yl)methanone, LCMS: m/z [M+H]⁺ = 495. ¹H-NMR (400 MHz, DMSO-d₆): 12.56 (brs, 1H), 7.65-7.63 (m, 1H), 7.58-7.52 (m, 2H), 7.41-7.39 (m, 1H), 7.36-7.32 (m, 1H), 7.31-7.29 (m, 1H), 6.98-6.71 (m, 2H), 6.22-6.20 (m, 1H), 4.68-4.55 (m, 3H), 4.07-3.98 (m, 2H), 3.69-3.63 (m, 1H), 3.53-3.51 (m, 1H), 3.23 (s, 3H), 2.10 (brs, 2H). |
| **25a/25b** | 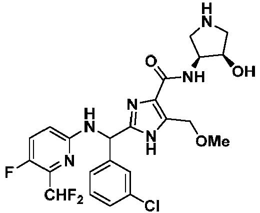 | 2-((3-chlorophenyl)((6-(difluoromethyl)-5-fluoropyridin-2-yl)amino)methyl)-N-((3S,4R)-4-hydroxypyrrolidin-3-yl)-5-(methoxymethyl)-1H-imidazole-4-carboxamide. |
| | | **Example 25a:** LCMS: m/z [M+H]⁺ = 525. ¹H-NMR (400 MHz, DMSO-d₆): 7.53-7.27 (m, 7H), 6.94-6.64 (m, 2H), 6.32-6.30 (m, 1H), 5.11 (bs, 1H), 4.72 (bs, 2H), 4.21-4.16 (m, 2H), 3.30 (s, 3H), 3.24-3.17 (m, 2H), 2.84-2.71 (m, 2H). |
| | | **Example 25b** LCMS: m/z [M+H]⁺ = 525. ¹H-NMR (400 MHz, DMSO-d₆): 7.57-7.27 (m, 7H), 6.93-6.64 (m, 2H), 6.32-6.30 (m, 1H), 5.25 (bs, 1H), 4.72 (brs, 2H), 4.26-4.21 (m, 2H), 3.30-3.22 (m, 5H), 2.83-2.79 (m, 2H). Chiral SFC: column: (R,R) WHELK O-1 (4.6 × 150 mm), 5 µm, mobile phase 0.2% iPrNH₂ in iPrOH:MeCN (1:1); flow: 3 mL/min; %of co-solvent: 40%; ABPR: 1500 psi; T: 35 °C; **Example 25a:** Rt = 3.811 min. **Example 25a:** Rt = 4.465 min. |

### Example 26: N-(3-aminopropyl)-2-((3-chloro-4-fluorophenyl)((5-fluoro-6-methylpyridin-2-yl)amino)-methyl)-5-methyl-1H-imidazole-4-sulfonamide

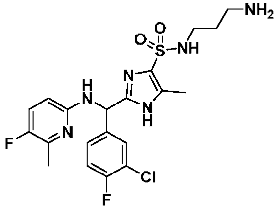

Step 1: To a solution of [4-(benzylsulfanyl)-5-methyl-1-{[2-(trimethylsilyl)ethoxy]methyl}-1H-imidazol-2-yl](3-chloro-4-fluorophenyl)methyl N,N-bis(propan-2-yl)carbamate (intermediate 34, regoisomeric mixture, 20.0 g, 32.242 mmol) in THF (300 mL) was added 5-fluoro-6-methylpyridin-2-amine (8.13 g, 64.485 mmol) and boron trifluoride etherate (12.0 mL, 96.727 mmol) followed by TFA (15.0 mL, 193.455 mmol) at 0 °C. The resulting mixture was stirred at 60 °C for 3 h. The reaction mixture was quenched with saturated NaHCO₃ solution, extracted with EtOAc (1 L) and washed with H₂O (500 mL) and brine (500 mL). The organic extract was dried over anhydrous Na₂SO₄, filtered and concentrated under reduced pressure to get the crude product, which was purified by column chromatography (SiO₂, 0-50% EtOAc/Hex) to afford N-{[4-(benzylsulfanyl)-5-methyl-1-{[2-(trimethylsilyl)ethoxy]methyl}-1H-imidazol-2-yl](4-chloro-3-fluorophenyl)methyl}-5-fluoro-6-methylpyridin-2-amine (regoisomeric mixture, 17.0 g, 87%). LCMS m/z = 601 [M+H]⁺.

Step 2: To a stirred solution of N-{[4-(benzylsulfanyl)-5-methyl-1-{[2-(trimethylsilyl)ethoxy]methyl}-1H-imidazol-2-yl](3-chloro-4-fluorophenyl)methyl}-5-fluoro-6-methylpyridin-2-amine (1.0 g, 1.66 mmol) in AcOH (10.0 mL) and water (1.7 mL) was added NCS (0.6 g, 4.99 mmol) portionwise at 0 °C. The reaction mixture was stirred at rt for 5 h. The reaction mixture was diluted with H₂O (10 mL) and extracted with ethyl acetate (2 × 10 mL). Th organic layer was dried over anhyd. Na₂SO₄ and concentrated under reduced pressure to obtain 2-[(3-chloro-4-fluorophenyl)[(5-fluoro-6-methylpyridin-2-yl)amino]methyl]-5-methyl-1-{[2-(trimethylsilyl)ethoxy]methyl}-1H-imidazole-4-sulfonyl chloride (0.9 g, 93%). LCMS m/z = 577 [M+H]⁺.

Step 3: To a stirred solution of 2-[(3-chloro-4-fluorophenyl)[(5-fluoro-6-methylpyridin-2-yl)amino]methyl]-5-methyl-1-{[2-(trimethylsilyl)ethoxy]methyl}-1H-imidazole-4-sulfonyl chloride (1.0 g, 1.73 mmol) in THF (10.0 mL) were added TEA (1.42 mL, 10.38 mmol) and tert-butyl N-(3-aminopropyl)carbamate (0.30 g, 1.73 mmol) dropwise at 0 °C. The reaction mixture was stirred at rt for 16 h. The reaction mixture was diluted with H₂O (5 mL) and extracted with ethyl acetate (2 × 15 mL). The combined organic layers were dried over anhyd. Na₂SO₄ and concentrated under reduced pressure to get the crude product, which was purified by column chromatography (SiO₂, 0-40% EtOAc/Hex) to afford tert-butyl N-(3-{2-[(3-chloro-4-fluorophenyl)[(5-fluoro-6-methylpyridin-2-yl)amino]methyl]-5-methyl-1-{[2-(trimethylsilyl)ethoxy]methyl}-1H-imidazole-4-sulfon-amido}propyl)carbamate (0.6 g, 48%). LCMS m/z = 715 [M+H]⁺. The enantiomers were separated by chiral SFC. Chiral SFC Column: (R,R) WHELK O-1 (30 mm x 250 mm), 5 µm, Mobile phase: 70% CO₂ + 30% of 0.3% iPrNH in iPrOH; Flow: 3 mL/min; % of Co-solvent: 30%; ABPR: 100 bar; T: 35 °C; T: 35 °C; Rt Peak 1 = 2.14 min, Rt Peak 2 = 2.42 min.

Both enantiomers were processed individually as described for Peak 1 below.

Step 4: To a stirred solution of tert-butyl 3-((2-((3-chloro-4-fluorophenyl)((5-fluoro-6-methylpyridin-2-yl)amino)methyl)-5-methyl-1-((2-(trimethylsilyl)ethoxy)methyl)-1H-imidazole)-4-sulfonamido)propyl)carbamate (Peak 1, 0.12 g , 0.168 mmol) in DCM (1.0 mL) was added TFA (1.0 mL) and the reaction mixture was stirred at rt for 16 h. The reaction mixture was concentrated under reduced pressure to get the crude product, which was purified by prep-HPLC to afford N-(3-aminopropyl)-2-((3-chloro-4-fluorophenyl)((5-fluoro-6-methylpyridin-2-yl)amino)methyl)-5-methyl-1H-imidazole-4-sulfonamide (0.04 g, 49%). LCMS m/z = 485 [M+H]⁺. ¹H-NMR (400 MHz, DMSO-d₆): 7.62 (d, 1H), 7.40-7.38 (m, 2H), 7.35-7.31 (m, 2H), 6.51 (d, 1H), 6.19 (d, 1H), 2.81-2.78 (m, 2H), 2.64-2.60 (m, 2H), 2.32 (s, 3H), 2.20 (s, 3H), 1.54-1.50 (m, 2H). Chiral HPLC: Column name: CHIRALPAK IC (250 × 4.6) mm, 5 µm, ambient temperature, flow rate = 1.0 mL/min, mobile phase = 80% Hexane, 20% EtOH and 0.1% iPrNH₂ **Example 26a:** Rt = 12.33 min.

Conversion of Peak 2 yielded **Example 26b:** LCMS m/z = 485 [M+H]⁺. ¹H-NMR (400 MHz, DMSO-d₆): 7.62 (d, 1H), 7.40-7.28 (m, 4H), 6.54-6.51(m, 1H), 6.19 (d, 1H), 2.80-2.77 (m, 2H), 2.57-2.55 (m, 2H), 2.32 (s, 3H), 2.20 (s, 3H), 1.47-1.44 (m, 2H). Chiral HPLC: Column name: CHIRALPAK IC (250 × 4.6) mm, 5 µm, ambient temperature, flow rate = 1.0 mL/min, mobile phase = 80% Hexane, 20% EtOH and 0.1% iPrNH₂ **Example 26b:** Rt = 11.11 min.

### Example 27

The title compound was prepared using an analogous method to that described for example 26. In step 4, different conditions were used: 4 M HCl in 1,4 dioxane, DCM, 0 to rt, 16 h.

| Example | Structure | Name/Data |
|---|---|---|
| **27a/27b** | 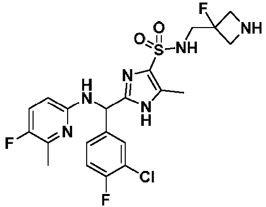 | 2-((3-chloro-4-fluorophenyl)((5-fluoro-6-methylpyridin-2-yl)amino)methyl)-N-((3-fluoroazetidin-3-yl)methyl)-5-methyl-1H-imidazole-4-sulfonamide, LCMS m/z = 511 [M+H]⁺. ¹H-NMR (400 MHz, DMSO-d6): 7.53-7.51 (m, 1H), 7.34-7.33 (m, 1H), 7.22-7.15 (m, 2H), 6.44-6.42 (m, 1H), 6.14 (s, 1H), 3.72-3.6 (m, 4H), 3.26 (s, 1H), 2.42 (s, 3H), 2.24-2.23 (d, 3H). Chiral SFC: Column: (R,R) WHELK O-1 (4.6 × 250 mm), 5 µm, Mobile phase: 70% CO₂ + 30% of 0.2 iPrNH₂ in iPrOH; Flow: 3 mL/min; % of Co-solvent: 30%; ABPR: 100 bar; T: 35 °C; **Example 27a:** Rt = 5.89 min, **Example 27b:** Rt = 6.39 min. |

### Example 28: 2-((3-chloro-4-fluorophenyl)((5-fluoro-6-methylpyridin-2-yl)amino)methyl)-N-(2-(dimethylamino)ethyl)-5-methyl-1H-imidazole-4-sulfonamide

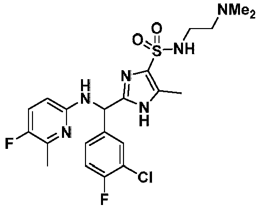

Step 1-3 were carried out in analogy to the synthesis of example 26 using N1,N1-dimethylethane-1,2-diamine in step 3.

Step 4: Both enantiomers were processed individually. To a stirred solution of 2-((3-chloro-4-fluorophenyl)((5-fluoro-6-methylpyridin-2-yl)amino)methyl)-N-(2-(dimethylamino)ethyl)-5-methyl-1-((2-(trimethylsilyl)ethoxy)methyl)-1H-imidazole-4-sulfonamide (Peak 1 from SFC, 280 mg, 0.45 mmol) in DCM (3 mL) was added 4 (M) HCl in dioxane (4 mL) and the mixture was stirred at rt for 16 h. The mixture was concentrated, the obtained residue was dissolved in THF (2 mL) followed by the addition of 25% NH₃ in water (3 mL) at 0°C and the mixture was stirred at rt for 5 h. The reaction mixture was diluted with brine (10 mL) and extracted with ethyl acetate (2 × 20 mL). The combined organic layers were dried over anhydrous Na₂SO₄ and concentrated under reduced pressure to afford the crude product, which was purified by reverse phase prep-HPLC to afford the title compound. Yield: 31% (70 mg).

LCMS m/z = 499 [M+H]⁺. ¹H-NMR (400 MHz, DMSO-d₆): 7.62 (d, 1H), 7.42-7.28 (m, 4H), 6.87 (brs, 1H), 6.53-6.51 (m, 1H), 6.20 (d, 1H), 2.82-2.78 (m, 2H), 2.33 (s, 3H), 2.20 (s, 3H), 2.17- 2.14 (m, 2H), 1.98 (s, 6H). Chiral HPLC Column: CHIRALPAK IC (250 × 4.6 mm, 5µm), Mobile phase: Hexane/EtOH/iPrNH₂: 80/20/0.1, Flow rate: 1.0 mL/min; **Example 28a:** Rt = 6.79 min, **Example 28b:** Rt = 7.36 min.

### Example 29: N-(azetidin-3-ylmethyl)-2-((3-chloro-4-fluorophenyl)((trans)-3-(trifluoromethyl)cyclobutoxy)methyl)-5-methyl-1H-imidazole-4-sulfonamide

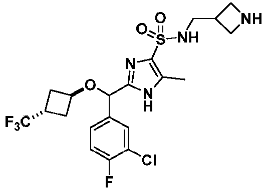

Step 1: To a stirred solution of (4-(benzylthio)-5-methyl-1-((2-(trimethylsilyl)ethoxy)methyl)-1H-imidazol-2-yl)(3,4-dichlorophenyl)methyl diisopropylcarbamate (intermediate 34, 700 mg, 1.099 mmol) in THF (11 mL) was added (trans)-3-(trifluoromethyl)cyclobutan-1-ol (462 mg, 3.298 mmol) followed by BF₃·Et₂O (0.4 mL, 3.298 mmol) and TFA (0.5 mL, 6.596 mmol) at 0 °C. The resulting reaction mixture was stirred at 80 °C for 2 h. The reaction mixture was quenched using cold sat. NaHCO₃ solution (500 mL) and extracted with DCM (2 × 500 mL). The combined organic layers were washed with water (500 mL) followed by brine (250 mL), dried over anhydrous Na₂SO₄ and concentrated under reduced pressure to afford the crude product, which was purified by column chromatography (SiO₂, 5% EtOAc/Hex) to afford 4-(benzylthio)-2-((3,4-dichlorophenyl)((trans)-3-(trifluoromethyl)cyclobutoxy)methyl)-5-methyl-1-((2-(trimethylsilyl)ethoxy)methyl)-1H-imidazole. Yield: 35% (240 g). LCMS m/z = 631 [M+H]⁺.

Step 2: To a stirred solution of 4-(benzylthio)-2-((3,4-dichlorophenyl)((trans)-3-(trifluoromethyl)cyclobutoxy)methyl)-5-methyl-1-((2-(trimethylsilyl)ethoxy)methyl)-1H-imidazole (390 mg, 0.617 mmol) in acetic acid (5 mL) was added NCS (330 mg , 2.47 mmol, 4 equiv.) at 0 °C. The reaction mixture was stirred at rt for 8 h. The reaction mixture was diluted with ethyl acetate (50 mL) and washed with cold water (2 × 20 mL). The combined organic layers were washed with brine (20 mL), dried over anhydrous Na₂SO₄ and concentrated under reduced pressure to afford 2-((3,4-dichlorophenyl)((trans)-3-(trifluoromethyl)cyclobutoxy)methyl)-5-methyl-1H-imidazole-4-sulfonyl chloride. Yield: 290 mg, crude.

Step 3: To a stirred solution 2-((3,4-dichlorophenyl)((trans)-3-(trifluoromethyl)cyclobutoxy)methyl)-5-methyl-1H-imidazole-4-sulfonyl chloride (290 mg, 0.607 mmol) in THF (4 mL) was added TEA (0.5 mL, 3.642 mmol) at 0 °C followed by tert-butyl 3-(aminomethyl)azetidine-1-carboxylate (1.4 g, 7.285 mmol) at the same temperature. The reaction mixture was stirred at rt for 16 h. The reaction mixture was diluted with cold water (30 mL) and extracted with ethyl acetate (2 × 40 mL). The combined organic layers were washed with brine (30 mL), dried over anhydrous Na₂SO₄ and concentrated under reduced pressure to afford the crude product, which was purified by column chromatography to afford tert-butyl 3-(((2-((3,4-dichlorophenyl)((trans)-3-(trifluoromethyl)cyclobutoxy)methyl)-5-methyl-1H-imidazole)-4-sulfonamido)methyl)azetidine-1-catboxylate. Yield: 34% over two steps (130 mg, 0.207 mmol). LCMS m/z = 627 [M+H]⁺.

The enantiomers were separated using chiral SFC.

Chiral SFC Column: (R,R)-WHELK-01 (4.6 × 250 mm), 5 µm, Mobile phase: 70% CO₂ + 30% of 0.2% iPrNH₂ in iPrOH; Flow: 3 mL/min; % of Co-solvent: 30% of 0.2% iPrNH₂ in iPrOH; ABPR: 100 bar; T: 35 °C; Rt Peak 1 = 2.74 min, Rt Peak 2 = 4.28 min.

Both enantiomers were processed individually as described for Peak 1 below.

To a stirred solution of tert-butyl 3-(((2-((3,4-dichlorophenyl)((trans)-3-(trifluoromethyl)cyclobutoxy)methyl)-5-methyl-1H-imidazole)-4-sulfonamido)methyl)azetidine-1-carboxylate (150 mg, 0.239 mmol) in DCM (4 mL) was added TFA (0.7 mL) at 0 °C. The resulting reaction mixture was stirred at rt for 3 h. The reaction mixture was concentrated to afford the crude product, which was purified by reverse-phase prep-HPLC to afford N-(azetidin-3-ylmethyl)-2-((3,4-dichlorophenyl)((1r,3r)-3-(trifluoromethyl)cyclobutoxy)methyl)-5-methyl-1H-imidazole-4-sulfonamide. Yield: 41% (51 mg).

LCMS m/z = 527 [M+H]⁺. ¹H-NMR (400 MHz, CD₃OD): 7.60 (s, 1H), 7.52-7.50 (d, 1H), 7.30-7.28 (d, 1H), 5.44 (s, 1H), 4.22-4.20 (m, 1H), 3.74-3.48 (m, 4H), 3.09-2.64 (m, 4H), 2.42-2.31 (m, 7H). Chiral SFC Colunm: (R,R) WHELK O-1 (4.6 × 250 mm), 5 µm, Mobile phase: 60% CO₂ + 40% of 0.2% iPrNH₂ in iPrOH; Flow: 3 mL/min; % of Co-solvent: 40% of 0.2% iPrNH₂ in iPrOH; ABPR: 100 bar; T: 35 °C; **Example 29a:** Rt = 2.32 min, **Example 29b:** Rt = 4.28 min (from conversion of Peak 2).

### Example 30: 2-((3-chloro-4-fluorophenyl)((5-fluoro-6-methoxypyridin-2-yl)amino)methyl)-5-methyl-1H-imidazole-4-carboxylic acid

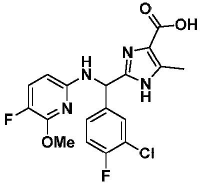

Step 1: To a mixture of (3-chloro-4-fluorophenyl)(4-iodo-5-methyl-1-((2-(trimethylsilyl)ethoxy)methyl)-1H-imidazol-2-yl)methyl diisopropylcarbamate (intermediate 27, 2 g, 3.2 mmol) and 5-fluoro-6-methoxypyridin-2-amine (0.45 g, 3.2 mmol) in THF (20 mL) were added BF₃·Et₂O (0.8 mL, 6.41 mmol) and TFA (1.23 mL, 16.02 mmol) at 0 °C and the mixtire was stirred at 90 °C for 2 h. The reaction mixture was quenched with NaHCO₃ solution (50 mL) and extracted with EtOAc (2 × 50 mL). The combined organic layers were washed with water (100 mL) and brine (100 mL), dried over anhydrous Na₂SO₄ and concentrated under reduced pressure to get the crude product which was purified by column chromatography (SiO₂, 20-50% EtOAc/Hex) to get N-((3-chloro-4-fluorophenyl)(4-iodo-5-methyl-1-((2-(trimethylsilyl)ethoxy)methyl)-1H-imidazol-2-yl)methyl)-5-fluoro-6-methoxypyridin-2-amine. Yield: 50% (1 g). LCMS m/z = 621 [M+H]⁺.

Step 2: To a solution of N-((3-chloro-4-fluorophenyl)(4-iodo-5-methyl-1-((2-(trimethylsilyl)ethoxy)methyl)-1H-imidazol-2-yl)methyl)-5-fluoro-6-methoxypyridin-2-amine (1 g, 1.61 mmol) in MeOH (100 mL) was added TEA (2.24 mL , 16.08 mmol) and the mixture was degassed with argon for 10 minutes, followed by the addition of Pd(OAc)₂ (140 mg, 0.64 mmol) and the mixture was heated to 80 °C under 120 psi of CO pressure for 16 h in an autoclave. The reaction mixture was filtered through a celite bed, washed with EtOAc (100 mL) and the filtrate was concentrated under reduced pressure to get crude the product which was purified by column chromatography (SiO₂, 0-60% EtOAc/Hex) to yield methyl 2-((3-chloro-4-fluorophenyl)((5-fluoro-6-methoxypyridin-2-yl)amino)methyl)-5-methyl-1-((2-trimethylsilyl) ethoxy) methyl)-1H-imidazole-4-carboxylate. Yield: 62% (550 mg). LCMS m/z = 553 [M+H]⁺.

Step 3: To a solution of methyl 2-((3-chloro-4-fluorophenyl)((5-fluoro-6-methoxypyridin-2-yl)amino)methyl)-5-methyl-1-((2-(trimethylsilyl)ethoxy)methyl)-1H-imidazole-4-carboxylate (500 mg, 0.9 mmol) in THF (10 mL) was added TBAF (1.0 M in THF, 5.5 mL, 5.42 mmol) and the resulting mixture was heated to 70 °C for 16 h. The reaction mixture was quenched with aq. NH₄Cl solution (50 mL) and extracted with EtOAc (2 × 50 mL). The combined organic layers were washed with brine (100 mL), dried over Na₂SO₄ and concentrated under reduced pressure to get the crude product which was purified by column chromatography (SiO₂, 0-100% EtOAc/Hex) to afford methyl 2-((3-chloro-4-fluorophenyl)((5-fluoro-6-methoxypyridin-2-yl)amino)methyl)-5-methyl-1H-imidazole-4-carboxylate. Yield: 65% (250 mg). LCMS m/z = 423 [M+H]⁺.

The enantiomers were separated by chiral SFC.

Chiral SFC: Colum name: (R,R)-WHELK-01, mobile phase: 0.3 iPrNH₂ in iPrOH, flow rate: 3 mL/min; % of co-solvent: 30%; ABPR: 100 psi; T: 35 °C; Rt Peak 1= 2.38 min, Rt Peak 2= 2.72 min.

Both enantiomers were processed individually as described for Peak 1 below.

Step 4: To a solution of methyl 2-((3-chloro-4-fluorophenyl)((5-fluoro-6-methoxypyridin-2-yl)amino)methyl)-5-methyl-1H-imidazole-4-carboxylate (Peak 1, 100 mg, 0.24 mmol) in a mixture of THF (10 mL) and water (5 mL) was added LiOH·H₂O (298 mg, 7.1 mmol) and the mixture was heated to 70° C for 16 h. The reaction mixture was evaporated to dryness inder reduced pressure, diluted with water (25 mL) and washed with EtOAc (75 mL). The aqueous part was acidified with sodium bisulfate and extracted with 10% MeOH in DCM (3 × 20 mL). The combined organic layers were dried over Na₂SO₄ and concentrated under reduced pressure to get the crude product which was purified by reverse phase prep-HPLC to obtain the title compound. Compound racemized under reaction conditions.

LCMS m/z = 409 [M+H]⁺. ¹H-NMR (400 MHz, CD₃OD): 12.61 (bs, 1H), 12.39 (bs, 1H), 7.68 (d, 1H), 7.46-7.42 (m, 2H), 7.38-7.30 (m, 2H), 6.20-6.09 (m, 2H), 3.75 (s, 3H), 2.33-2.32 (m, 3H).

### Example 31: 2-((3-chloro-4-fluorophenyl)(((trans)-4-(trifluoromethyl)cyclohexyl)oxy)methyl)-5-methyl-1H-imidazole-4-carboxylic acid

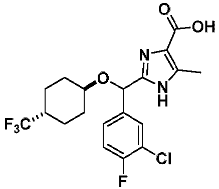

Step 1: To a solution of methyl 2-[(3-chloro-4-fluorophenyl) ({ [(trans)-4-(trifluoromethyl) cyclohexyl]oxy})methyl]-5-methyl-1-{[2-(trimethylsilyl)ethoxy]methyl}-1H-imidazole-4-carboxylate (intermediate 35, 0.8 g, 1.38 mmol) in DCM (2 mL) was added 4M HCl in 1,4-dioxane (10 mL) at 0 °C and the mixture was stirred at rt for 24 h. The reaction mixture was concentrated under reduced pressure. The reaction mixture was neutralized with saturated NaHCO₃ solution (300 mL) and extracted with ethyl acetate (2 × 300 mL). The combined organic layers was washed with water (2 × 300 mL) followed by brine (200 mL), dried over anhydrous Na₂SO₄ and concentrated under reduced pressure to afford methyl 2-((3-chloro-4-fluorophenyl)(((trans)-4-(trifluoromethyl)cyclohexyl)oxy)methyl)-5-methyl-1H-imidazole-4-carboxylate. Yield: 96% (0.6 g). LCMS m/z = 449 [M+H]⁺.

The enantiomers were separated via chiral SFC. Both enantiomers were processed individually as described for Peak 1 below.

Step 2: To a solution of methyl 2-((3-chloro-4-fluorophenyl)(((trans)-4-(trifluoromethyl)cyclohexyl)oxy)methyl)-5-methyl-1H-imidazole-4-carboxylate (Peak 1, 0.2 g, 0.45 mmol) in a mixture of THF-MeOH (18 mL, 9:1) and water (6 mL) was added LiOH·H₂O (0.10 g, 4.45 mmol, 10.0 equiv.) at 0 °C and the mixture was then stirred at rt for 72 h. The volatiles were evaporated under reduced pressure and the residue was purified by reverse phase prep HPLC to afford the title compound. Yield: 18% (0.035 g)

LCMS m/z = 435 [M+H]⁺. ¹H-NMR (400 MHz, CD₃OD): 12.35 (bs, 1H),7.62 (s, 1H), 7.42 -7.38 (m, 2H), 5.66 (s,1H), 2.36-2.33 (m, 3H), 2.24-2.22 (m, 1H), 2.05 (bs, 2H), 1.85 (d, 2H), 1.34-1.17 (m, 4H). Chiral HPLC Column: Chiralpak IG (4.6 x 250 mm), 5 µm; mobile phase: hexane/EtOH/TFA: 95/05/0.1; Flow: 1.0 mL/min; **Example 31a:** Rt = 8.64 min, **Example 31b:** Rt = 9.34 min (from coversion of Peak 2).

### Example 32: 2-((3-chloro-4-fluorophenyl)(((trans)-4-(trifluoromethyl)cyclohexyl)oxy)methyl)-N-((3S,4R)-4-hydroxypyrrolidin-3-yl)-5-methyl-1H-imidazole-4-carboxamide

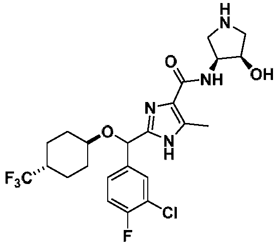

Step 1: To a solution of methyl 2-((3-chloro-4-fluorophenyl)(((trans)-4-(trifluoromethyl)cyclohexyl)oxy)methyl)-5-methyl-1-((2-(trimethylsilyl)ethoxy)methyl)-1H-imidazole-4-carboxylate (intermediate 35, 240 mg, 0.41 mmol) in THF (5 mL), H₂O (2 mL), MeOH (1 mL) was added LiOH·H₂O (160 mg, 4.14 mmol) and the mixture was stirred at rt for 16 h. The reaction mixture was acidified with saturated sodium bisulphate monohydrate solution to pH ~3 and extracted with EtOAc (50 mL). The organic part was dried over Na₂SO₄, filtered and concentrated under reduced pressure to afford 2-((3-chloro-4-fluorophenyl)(((trans)-4-(trifluoromethyl)cyclohexyl)oxy)methyl)-5-methyl-1-((2-(trimethylsilyl)ethoxy)methyl)-1H-imidazole-4-carboxylic acid, Yield: 85% (200 mg). LCMS m/z = 565 [M+H]⁺.

Step 2: To a mixture of 2-((3-chloro-4-fluorophenyl)(((trans)-4-(trifluoromethyl)cyclohexyl)oxy)methyl)-5-methyl-1-((2-(trimethylsilyl)ethoxy)methyl)-1H-imidazole-4-carboxylic acid (200 mg, 0.35 mmol) and tert-butyl (3S,4R)-3-amino-4-hydroxypyrrolidine-1-carboxylate (85 mg, 0.42 mmol) in DMF (5 mL) were added DIPEA (0.13 mL, 1.77 mmol), EDCI (80 mg, 0.42 mmol) and HOBT (60 mg, 0.46 mmol) and the mixture was stirred at rt for 16 h. The reaction mixture was diluted with water (60 mL) and extracted with EtOAc (60 mL). The organic layer was washed with brine (50 mL), dried over Na₂SO₄ and concentrated under reduced pressure to get the crude prudct which was purified by column chromatography (SiO₂, 50% EtOAc/Hex) to afford tert-butyl (3S,4R)-3-(2-((3-chloro-4-fluorophenyl)(((trans)-4-(trifluoromethyl)cyclohexyl)oxy)methyl)-5-methyl-1-((2-(trimethylsilyl)ethoxy)methyl)-1H-imidazole-4-carboxamido)-4-hydroxypyrrolidine-1-carboxylate. Yield: 64% (170 mg). LCMS m/z = 749 [M+H]⁺.

Step 3: To a solution tert-butyl (3S,4R)-3-(2-((3-chloro-4-fluorophenyl)(((trans)-4-(trifluoromethyl)cyclohexyl)oxy)methyl)-5-methyl-1-((2-(trimethylsilyl)ethoxy)methyl)-1H-imidazole-4-carboxamido)-4-hydroxy-pyrrolidine-1-carboxylate (200 mg, 0.27 mmol) in THF (10 mL) was added a 1 M solution of TBAF in THF (0.8 mL, 0.8 mmol) and the mixture was stirred at 60 °C for 16 h. The reaction mixture was quenched with water (30 mL) and extracted with ethyl acetate (2 × 30 mL). The combined organic layers were washed with water (40 mL) and brine (40 mL), dried over anhydrous Na₂SO₄, filtered and concentrated under reduced pressure to afford the crude product which was purified by flash chromatography (SiO₂, 40-50% EtOAc/Hex) to afford tert-butyl (3S,4R)-3-(2-((3-chloro-4-fluorophenyl)(((trans)-4-(trifluoromethyl)cyclohexyl)oxy)methyl)-5-methyl-1H-imidazole-4-carboxamido)-4-hydroxypyrrolidine-1-carboxylate. Yield: 84% (140 mg, 0.22 mmol). LCMS m/z = 619 [M+H]⁺.

The diastereomers were separated using chiral SFC.

Both diastereomers were processed individually as described for Peak 1 below.

Step 4: A solution of tert-butyl (3S,4R)-3-(2-((3-chloro-4-fluorophenyl)(((trans)-4-(trifluoromethyl)cyclohexyl)oxy)methyl)-5-methyl-1H-imidazole-4-carboxamido)-4-hydroxypyrrolidine-1-carboxylate (55 mg, 0.09 mmol) in TFA (1 mL) was stirred at rt for 2 h. The reaction mixture was concentrated under reduced pressure and was diluted with MeOH (10 mL). Amberlyst A21 resin (200 mg) was added and the mixture was stirred at rt for 2 h. The reaction mixture was filtered, washed with MeOH (10 mL) and the filtrate was concentrated under reduced pressure to get the crude product which was purified by reverse phase prep HPLC to get the title compound. Yield: 39% (18 mg).

LCMS m/z = 519 [M+H]⁺. ¹H-NMR (400 MHz, CD₃OD): 12.24 (bs, 1H), 7.61-7.60 (m, 1H), 7.47-7.35 (m, 3H), 5.67 (s, 1H), 5.24-5.23 (m, 1H), 4.01-3.93 (m, 2H), 3.10-3.01 (m, 2H), 2.68-2.61 (m, 1H), 2.40 (s, 3H), 2.24-2.12 (m, 1H), 2.09-1.95 (m, 2H), 1.86-1.83 (m, 2H), 1.25-1.18 (m, 4H). Chiral SFC: column: (R,R)-WHELK-01 (4.6 × 150 mm), 5 µm, mobile phase 0.2% iPrNH₂in iPrOH; flow: 3 g/min; % of co-solvent: 25%; ABPR: 1500 psi; T: 35 °C; **Example 32a:** Rt = 2.41 min.

LCMS m/z = 519 [M+H]⁺. ¹H-NMR (400 MHz, CD₃OD): 11.90 (bs, 1H), 7.59-7.58 (m, 1H), 7.45-7.43 (m, 1H), 7.38-7.30 (m, 2H), 5.64 (s, 1H), 4.83 (bs, 1H), 4.05-4.01 (m, 2H), 3.43-3.40 (m, 1H), 3.39-3.37 (m, 2H), 2.71-2.67 (m, 1H), 2.57-2.55 (m, 1H), 2.43 (s, 3H), 2.33-2.22 (m, 1H), 2.20-2.11 (m, 2H), 2.08-2.04 (m, 2H), 1.39-1.29 (m, 4H). Chiral SFC: column: (R,R)-WHELK-01 (4.6 × 150 mm), 5 µm, mobile phase 0.2% iPrNH₂ in iPrOH; flow: 3 g/min; % of co-solvent: 25%; ABPR: 1500 psi; T: 35 °C; **Example 32b:** Rt = 3.09 min.

### Example 33: N-((4-((azetidin-3-ylmethyl)sulfonyl)-5-methyl-1H-imidazol-2-yl)(3-chloro-4-fluorophenyl)methyl)-5-fluoro-6-methylpyridin-2-amine

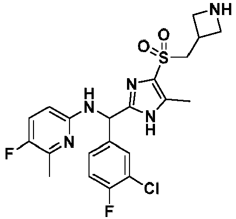

Step 1: A mixture of (3-chloro-4-fluorophenyl)(4-iodo-5-methyl-1-{[2-(trimethylsilyl)ethoxy]methyl}-1H-imidazol-2-yl)methyl N,N-bis(propan-2-yl)carbamate (intermediate 34, 2 g, 3.21 mmol), tert-butyl 3-(mercaptomethyl)azetidine-1-carboxylate (1.96 g, 9.62 mmol) and K₂CO₃ (1.33 g, 9.62 mmol) in 1,4-dioxane (25 mL) was degassed with argon for 10 min followed by the addition of Xantphos (0.28 g, 0.48 mmol) and Pd₂(dba)₃ (0.29 g, 0.32 mmol) at rt. The reaction mixture was heated to 110 °C for 16 h in a sealed tube. The mixture was filtered, the filter was washed with EtOAc (100 mL) and the filtrate was concentrated under reduced pressure to get the crude product which was purified by column chromatography (SiO₂, 0-50% EtOAc/Hex) to afford tert-butyl 3-(((2-((3-chloro-4-fluorophenyl)((diisopropylcarbamoyl)oxy)methyl)-5-methyl-1-((2-(trimethylsilyl)ethoxy)methyl)-1H-imidazol-4-yl)thio)methyl)azetidine-1-carboxylate. Yield: 40% (0.9 g). LCMS m/z = 700 [M+H]⁺.

Step 2: To a solution of tert-butyl 3-(((2-((3-chloro-4-fluorophenyl)((diisopropylcarbamoyl)oxy)methyl)-5-methyl-1-((2-(trimethylsilyl)ethoxy)methyl)-1H-imidazol-4-yl)thio)methyl)azetidine-1-carboxylate (600 mg, 0.86 mmol) in THF (8 mL) and water (2 mL) was added oxone (1054.78 mg, 3.43 mmol) at rt and the mixture was stirred for 16 h. The reaction was extracted with EtOAc (2 × 150 mL), the combined organic layers were washed with brine (100 mL). dried over Na₂SO₄ and concentrated under reduced pressure to afford the crude product which was purified by column chromatography (SiO₂, 0-80% EtOAc/Hex) to afford tert-butyl 3-(((2-((3-chloro-4-fluorophenyl)((diisopropylcarbamoyl)oxy)methyl)-5-methyl-1-((2-(trimethylsilyl)ethoxy)methyl)-1H-imidazol-4-yl)sulfonyl)methyl)azetidine-1-carboxylate. Yield: 40% (0.25 g). LCMS m/z = 731 [M+H]⁺.

Step 3: To a mixture of tert-butyl 3-(((2-((3-chloro-4-fluorophenyl)((diisopropylcarbamoyl)oxy)methyl)-5-methyl-1-((2-(trimethylsilyl)ethoxy)methyl)-1H-imidazol-4-yl)sulfonyl)methyl)azetidine-1-carboxylate (200 mg, 0.27 mmol) and 5-fluoro-6-methylpyridin-2-amine (103.47 mg, 0.82 mmol) in DCM (5 mL) were added BF₃·Et₂O (0.1 mL, 0.82 mmol) and TFA (0.19 mL, 2.46 mmol) at 0 °C and the mixture was stirred at rt for 16 h. The reaction mixture was concentrated under reduced pressure, diluted with ice-cooled water (20 mL), neutralized with sat. NaHCO₃ solution (50 mL) and extracted with EtOAc (150 mL). The organic layer was dried over sodium sulfate and concentrated under reduced pressure to get the crude product which was purified by reverse phase prep HPLC to afford the title compound. Yield: 15 % (20 mg). LCMS m/z = 482 [M+H]⁺. ¹H-NMR (400 MHz, DMSO-d₆): 7.62 (d, 1H), 7.40-7.35 (m, 3H), 7.30 (t, 1H), 6.55-6.52 (m, 1H), 6.19 (d, 1H), 3.44-3.31 (m, 1H), 3.21-3.16 (m, 2H), 2.84-2.80 (m, 1H), 2.33 (s, 3H), 2.20 (s, 3H).

### Example 34: 2-(bis(4-chloro-2-methylphenyl)methyl)-5-methyl-1H-imidazole-4-carboxylic acid

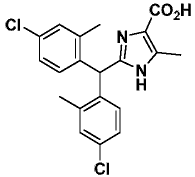

Step 1: To a solution of 2-(bis(4-chloro-2-methylphenyl)methyl)-4-iodo-5-methyl-1H-imidazole (intermediate 22, 0.16 g, 0.35 mmol) in methanol (10 mL) was added TEA (0.15 mL, 1.05 mmol) and the mixture was degassed with nitrogen for 10 minutes, followed by the addition of PdCl₂(dppf)·DCM (0.025 g, 0.04 mmol). The mixture was heated to 90 °C under 150 psi of CO for 16 h in an autoclave. The reaction mixture was filtered through a celite pad, the pad was washed with EtOAc, and the filtrate was concentrated under reduced pressure. The resulting residue was purified by column chromatography (SiO₂, 0-50% EtOAc/Hex) to afford methyl 2-(bis(4-chloro-2-methylphenyl) methyl)-5-methyl-1H-imidazole-4-carboxylate. Yield: 71% (0.1 g). LCMS m/z = 403 [M+H]⁺.

Step 2: To a solution of methyl 2-(bis(4-chloro-2-methylphenyl)methyl)-5-methyl-1H-imidazole-4-carboxylate (0.1 g, 0.25 mmol) in a mixture of THF-MeOH (8 mL, 3:1) and water (6 mL) was added LiOH·H₂O (0.05 g, 1.24 mmol) at rt. The reaction mixture was stirred at rt for 16 h. The reaction mixture was concentrated under reduced pressure, and the resulting residue was purified by reverse phase prep HPLC obtain the title compound. Yield: 26% (0.025 g) LCMS m/z = 389 [M+H]⁺. ¹H-NMR (400 MHz, DMSO-d₆): 12.55 (bs, 1H), 12.22 (bs, 1H), 7.29 (s, 2H), 7.22-7.20 (m, 2H), 6.81 (bs, 2H), 5.67 (bs, 1H), 2.33 (s, 3H), 2.12 (s, 6H).

The prophetic examples 35-103 can be synthesized according to the general schemes and by analogy to the synthetic procedures described above.

### LCMS Methods

### Method A:

Waters Acquity H Class UPLC attached with Waters SQD 2 mass spectrometer; Ionisation method: Electro spray
Capillary (kV) 3.50, Cone (V) 25.00, Source Temperature (°C) 150, Desolvation Temperature (°C) 400, Cone Gas Flow (L/Hr) -50, Desolvation Gas Flow (L/Hr) -750; Mass range:100 to 900 Da; DAD Wavelength range (nm): 200 to 400; Solvent A: 5 mM NH₄OAc in water and Solvent B: 5 mM NH₄OAc in ACN: Water (90:10); Flow rate: 1.20 ml/min; (mobile phase: 95% [5 mM NH₄OAc in water] and 5% [5 mM NH₄OAc in ACN: Water (90:10)] held for 0.75 min, then 85% [5 mM NH₄OAc in water] and 15% [5 mM NH₄OAc in ACN: Water (90:10)] in 1.25 min further 70% [5 mM NH₄OAc in water] and 30% [5 mM NH₄OAc in ACN: Water (90:10)] in 2.50 min, and finally 2% [5 mM NH₄OAc in water] and 98% [5 mM NH₄OAc in ACN: Water (90:10)] in 3.75 min, held this mobile phase composition up to 4.25 min and finally back to initial condition in 4.50 min and held this composition up to 5.10 min).

| TIME | Flow Rate (ml/min) | %A (5 mM NH₄OAc in water) | % B (5 mM NH₄OAc in ACN: Water (90:10)) |
|---|---|---|---|
| 0.00 | 1.20 | 95 | 5 |
| 0.75 | 1.20 | 95 | 5 |
| 1.25 | 1.20 | 85 | 15 |
| 2.50 | 1.20 | 70 | 30 |
| 3.75 | 1.20 | 2 | 98 |
| 4.25 | 1.20 | 2 | 98 |
| 4.50 | 1.20 | 95 | 5 |
| 5.10 | 1.20 | 95 | 5 |

Column Used: Xbridge C18 column (3.5 µm, 50 x 3 mm); Column Temperature: 40°C.

### Method B

Waters Acquity H Class UPLC attached with Waters SQD 2 mass spectrometer; Ionisation method: Electro spray; Capillary (kV) 3.50, Cone (V) 25.00, Source Temperature (°C) 150, Desolvation Temperature (°C) 400, Cone Gas Flow (L/Hr) -50, Desolvation Gas Flow (L/Hr) -750;Mass range:100 to 900 Da; DAD Wavelength range (nm): 200 to 400; Solvent A: 5 mM NH₄OAc in water and Solvent B: 5 mM NH₄OAc in ACN: Water (90:10); Flow rate: 1.00 ml/min; (mobile phase: from 98% [5 mM NH₄OAc in water] and 2% [5 mM NH₄OAc in ACN: Water (90:10)] to 85% [5 mM NH₄OAc in water] and 15% [5 mM NH₄OAc in ACN: Water (90:10)] in 1.00 min, then 60% [5 mM NH₄OAc in water] and 40% [5 mM NH₄OAc in ACN: Water (90:10)] in 6.50 min, and finally 2% [5 mM NH₄OAc in water] and 98% [5 mM NH₄OAc in ACN: Water (90:10)] in 8.00 min, held this mobile phase composition up to 10.00 min and finally back to initial condition in 11.00 min and held this composition up to 12.00 min).

| TIME | Flow Rate (ml/min) | %A (5 mM NH₄OAc in water) | % B (5 mM NH₄OAc in ACN: Water (90:10) ) |
|---|---|---|---|
| 0.00 | 1.00 | 98 | 2 |
| 1.00 | 1.00 | 85 | 15 |
| 6.50 | 1.00 | 60 | 40 |
| 8.00 | 1.00 | 2 | 98 |
| 10.00 | 1.00 | 2 | 98 |
| 11.00 | 1.00 | 98 | 2 |
| 12.00 | 1.00 | 98 | 2 |

Column Used: Xbridge C18 column (3.5 µm, 50 x 3 mm); Column Temperature: 40°C.

### Method B (K91-5-min)

Waters Acquity H Class UPLC attached with Waters SQD 2 mass spectrometer; Ionisation method: Electro spray; Capillary (kV) 3.50, Cone (V) 25.00, Source Temperature (°C) 150, Desolvation Temperature (°C) 400, Cone Gas Flow (L/Hr) -50, Desolvation Gas Flow (L/Hr) -750; Mass range:100 to 900 Da; DAD Wavelength range (nm): 200 to 400; Solvent A: 5 mM NH₄OAc in water and Solvent B: 5 mM NH₄OAc in ACN: Water (90:10); Flow rate: 1.20 ml/min; (mobile phase: 95% [5 mM NH₄OAc in water] and 5% [5 mM NH₄OAc in ACN: Water (90:10)] held for 0.75 min, then 85% [5 mM NH₄OAc in water] and 15% [5 mM NH₄OAc in ACN: Water (90:10)] in 1.25 min further 70% [5 mM NH₄OAc in water] and 30% [5 mM NH₄OAc in ACN: Water (90:10)] in 2.50 min, and finally 2% [5 mM NH₄OAc in water] and 98% [5 mM NH₄OAc in ACN: Water (90:10)] in 3.75 min, held this mobile phase composition up to 4.25 min and finally back to initial condition in 4.50 min and held this composition up to 5.10 min).

| TIME | Flow Rate (ml/min) | %A (5 mM NH4OAc in water) | % B (5 mM NH4OAc in ACN: Water (90:10) |
|---|---|---|---|
| 0.00 | 1.20 | 95 | 5 |
| 0.75 | 1.20 | 95 | 5 |
| 1.25 | 1.20 | 85 | 15 |
| 2.50 | 1.20 | 70 | 30 |
| 3.75 | 1.20 | 2 | 98 |
| 4.25 | 1.20 | 2 | 98 |
| 4.50 | 1.20 | 95 | 5 |
| 5.10 | 1.20 | 95 | 5 |

Column Used: Xbridge C18 column (3.5 µm, 50 x 3 mm); Column Temperature: 40°C.

### Method C (K91-12-min)

Waters Acquity H Class UPLC attached with Waters SQD 2 mass spectrometer; Ionisation method: Electro spray; Capillary (kV) 3.50, Cone (V) 25.00, Source Temperature (°C) 150, Desolvation Temperature (°C) 400, Cone Gas Flow (L/Hr) -50, Desolvation Gas Flow (L/Hr) -750; Mass range:100 to 900 Da; DAD Wavelength range (nm): 200 to 400; Solvent A: 5 mM NH₄OAc in water and Solvent B: 5 mM NH₄OAc in ACN: Water (90:10); Flow rate: 1.00 ml/min; (mobile phase: from 98% [5 mM NH₄OAc in water] and 2% [5 mM NH₄OAc in ACN: Water (90:10)] to 85% [5 mM NH₄OAc in water] and 15% [5 mM NH₄OAc in ACN: Water (90:10)] in 1.00 min, then 60% [5 mM NH₄OAc in water] and 40% [5 mM NH₄OAc in ACN: Water (90:10)] in 6.50 min, and finally 2% [5 mM NH₄OAc in water] and 98% [5 mM NH₄OAc in ACN: Water (90:10)] in 8.00 min, held this mobile phase composition up to 10.00 min and finally back to initial condition in 11.00 min and held this composition up to 12.00 min).

| TIME | Flow Rate (ml/min) | %A (5 mM NH₄OAc in water) | % B (5 mM NH₄OAc in ACN: Water (90:10) |
|---|---|---|---|
| 0.00 | 1.00 | 98 | 2 |
| 1.00 | 1.00 | 85 | 15 |
| 6.50 | 1.00 | 60 | 40 |
| 8.00 | 1.00 | 2 | 98 |
| 10.00 | 1.00 | 2 | 98 |
| 11.00 | 1.00 | 98 | 2 |
| 12.00 | 1.00 | 98 | 2 |

Column Used: Xbridge C18 column (3.5 µm, 50 x 3 mm); Column Temperature: 40 °C.

### Electrophysiology: Voltage-Clamp recordings

The following Na_{V}1.8 recombinant cell line was used for recordings: HEK-Na_{V}1.8 (NM_006514.1) with b3 (NM_018400.3).

Sodium currents were measured using the patch clamp technique in the whole-cell configuration using the Qube384 (Sophion A/S, Copenhagen, Denmark) automated voltage clamp platform. "Multi hole" plates were used for the cell line expressing Na_{V}1.8. Appropriate filters (for minimum seal resistance and minimum current size) and series resistance compensation (for high quality sodium channel recordings) were applied. Data was collected at ambient room temperature or 19 °C.

The recording extracellular solution contained (in mM): NaCl 145 mM, KCl 4 mM, CaCl₂ 2 mM, MgCl₂ 1 mM, HEPES 10 mM, Glucose 10 mM, pH 7.4 (NaOH). The intracellular recording solution contained (in mM): CsF 120 mM, CsCl 20 mM, NaCl 10 mM, EGTA 10 mM, HEPES 10 mM, pH 7.2 (CsOH). Currents were recorded at 25 kHz sampling frequency and filtered at 5 kHz. Series resistance compensation was applied at 65%.

Vehicle (VEH) is the control condition where cells are exposed to 0.3 % DMSO without compound. All runs include VEH controls being exposed to the same voltage protocols to assess non-compound related phenomena such as run down and then used to isolate compound dependent effects on currents.

### To check for state-dependent inhibition, the following voltage-sequence was applied every 20 seconds:

From a resting membrane potential of -120 mV, the first test pulse (P1; 20 ms to -10 mV) was applied to check for channels in the Resting State followed by a brief recovery (100 ms to -120 mV), then holding the membrane voltage to V_{1/2} (4 seconds at voltage to obtain half the channel at Rest and half Inactivated) with a subsequent second test pulse (P2; 20 ms to -10 mV) to check for channels in the Inactivated State, followed by another brief recovery (20 ms to -120 mV) and a final third test pulse (P3; 20 ms to -10 mV) to check for recovered channels.

### To check for frequency-dependent inhibition, a 10 Hz protocol and a 20 Hz protocol were applied; namely,

From a resting membrane potential of -120 mV, 40-pulses (10 ms to -10 mV) was applied at 10 Hz (at 100 ms between pulses) and then at 20 Hz (at 50 ms between pulses).

Each parameter was recorded (P1, P2, P3, P40 from 10Hz and P40 from 20Hz) during a control period (lasting ~ 5 minutes) when establishing the baseline and during compound period (lasting ~ 12 minutes) when test compound (or vehicle) was applied. For each parameter, the value at the end of the compound period was normalized to the vehicle baseline; as follows $Normalized Inhibition \left({Norm}_{CPD}\right) = \frac{CPD {Value}_{end CPD period}}{VEH {Value}_{end Control Period}}$

To adjust for any variance in the Na⁺ current signal during the compound period (owing to cumulative inactivation independent of compound or shifts in biophysics over time), a dedicated segment of the recording wells in each 384 plate were dedicated to having only vehicle exposure. These vehicle-only recordings were used to correct for any apparent "run-up" or "run-down" in the experiment. $Normalized Inhibition \left({Norm}_{VEH}\right) = \frac{VEH {Value}_{end CPD period}}{VEH {Value}_{end Control Period}}$

The adjusted inhibition was calculated as follows: $% {Inhibition}_{corrected} = 100 \times \frac{{Norm}_{CPD} - {Norm}_{VEH}}{100 - {Norm}_{VEH}}$

Percent inhibition was determined and IC₅₀ values were calculated using a 4 parameter logistic model within XLFit Software (IDBS, Boston MA): $% {Inhibition}_{corrected} = A + \frac{\left(B-A\right)}{{\left(1+\left(\frac{x}{C}\right.\right)}^{D}}$ where A and B are the maximal and minimum inhibition respectively, C is the IC₅₀ concentration and D is the (Hill) slope.

The potency data of the example compounds are summarized in the table below (category **A:** human NaV1.8 IC₅₀ ≤ 0.1 µM; category **B:** 0.1 µM < human NaV1.8 IC₅₀ ≤ 1 µM; category **C:** 1 µM < human NaV1.8 IC₅₀ ≤ 10 µM; "n.d.": not determined):

| Example | Potency category |
|---|---|
| 1 | A |
| 2 | B |
| 3 | B |
| 4 | B |
| 5 | C |
| 6 | C |
| 7 | C |
| 8 | B |
| 9 | B |
| 10 | B |
| 11 | B |
| 12 | C |
| 13 | A |
| 14 | A |
| 15 | A |
| 16 | A |
| 17a | A |
| 17b | A |
| 18a | A |
| 18b | A |
| 19 | A |
| 20 | A |
| 21 | A |
| 22a | A |
| 22b | A |
| 23a | A |
| 23b | A |
| 24 | A |
| 25a | A |
| 25b | A |
| 26a | A |
| 26b | A |
| 27a | A |
| 27b | A |
| 28a | A |
| 28b | A |
| 29a | A |
| 29b | A |
| 30 | A |
| 31a | A |
| 31b | A |
| 32a | A |
| 32b | A |
| 33 | A |
| 34 | A |

## Claims

1. A compound according to general formula (I) 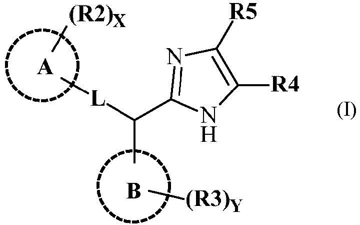 wherein
**A** and **B** independently from one another represent phenyl, 5 to 10-membered heteroaryl, C₃₋₇-cycloalkyl, or 4- to 10-membered heterocycloalkyl;
**R2** and **R3** independently from one another represent F, Cl, CN, C₁₋₄-alkyl, C₃₋₇-cycloalkyl, OH, OC₁₋₄-alkyl, NH₂, NHC₁₋₄-alkyl or N(C₁₋₄-alkyl)₂;
**X** and **Y** independently from one another represent 0, 1, 2, 3 or 4;
**L** represents bond, O, NH or C₁₋₃-alkylene;
**R4** represents H, Cl, CN, C₁₋₄-alkyl or C₃₋₇-cycloalkyl;
**R5** represents S(O)₂N**R6R7**, C(O)N**R6R7**, C(O)O**R6**, C(O)OH, C(O)NH₂, C(O)C₁₋₄-alkyl, S(O)₂-(4- to 7-membered heterocycloalkyl) or S(O)₂C₁₋₃-alkylene-(4- to 7-membered heterocycloalkyl);
**R6** represents C₁₋₄-alkyl, C₃₋₇-cycloalkyl, 4- to 7-membered heterocycloalkyl or C₁₋₃-alkylene-(4- to 7-membered heterocycloalkyl); and
**R7** represents H or C₁₋₄-alkyl; or
**R4** and **R6** together with the atoms to which they are attached form 4- to 7-membered heterocycloalkyl or 5 to 10-membered heteroaryl; or
**R6** and **R7** together with the nitrogen atom to which they are attached form 4- to 7-membered heterocycloalkyl;
with the proviso that when **R5** represents S(O)₂N**R6R7**, **R4** represents C₁₋₄-alkyl and/or **L** represents O or NH;
wherein C₁₋₄-alkyl and C₁₋₃-alkylene in each case independently from one another is linear or branched, saturated or unsaturated;
wherein C₁₋₄-alkyl, C₁₋₃-alkylene, C₃₋₇cycloalkyl, 4- to 7-membered heterocycloalkyl and 4- to 10-membered heterocycloalkyl in each case independently from one another are unsubstituted or mono- or polysubstituted with one or more substituents selected from F, Cl, CN, C₁₋₄-alkyl, C₁₋₃-alkylene-NH₂, CH₂F, CHF₂, CF₃, C(O)C₁₋₄-alkyl, C(O)OH, C(O)OC₁₋₄-alkyl, C(O)NH₂, C(O)NHC₁₋₄-alkyl, C(O)N(C₁₋₄-alkyl)₂, OH, =O, OC₁₋₄-alkyl, OCH₂F, OCHF₂, OCF₃, NH₂, NHC₁₋₄-alkyl, N(C₁₋₄-alkyl)₂ and NHC(O)C₁₋₄-alkyl;
wherein phenyl and 5 to 10-membered heteroaryl in each case independently from one another are unsubstituted or mono- or polysubstituted with one or more substituents selected from F, Cl, CN, C₁₋₄-alkyl, CH₂F, CHF₂, CF₃, OH, OCH₂F, OCHF₂, OCF₃, OC₁₋₄-alkyl, NH₂, NHC₁₋₄-alkyl and N(C₁₋₄-alkyl)₂;
in the form of the free compound or a physiologically acceptable salt thereof.

2. The compound according to claim 1, wherein **A** represents phenyl, **L** represents bond and **B** represents phenyl; or A represents C₃₋₇-cycloalkyl, **L** represents **O** and **B** represents phenyl.

3. The compound according to claim 1 or 2, wherein **A** represents pyridyl, **L** represents NH and **B** represents phenyl.

4. The compound according to any of the preceding claims, wherein **R5** represents C(O)N**R6R7**, C(O)O**R6**, C(O)NH₂, C(O)OH or C(O)C₁₋₄-alkyl.

5. The compound according to any of the preceding claims, wherein **R5** represents S(O)₂N**R6R7**.

6. The compound according to any of the preceding claims, wherein **R6** represents C₁₋₄-alkyl; preferably CH₃, CH₂C(O)NH₂, CH₂CH₂NH₂, CH₂CH₂NHCH₃, CH₂CH₂N(CH₃)₂, CH₂CH₂CH₂NH₂, CH₂CF₂CH₂NH₂, CH₂CH₂CH₂NHCH₃ or CH₂CH₂CH₂N(CH₃)₂.

7. The compound according to any of the preceding claims, wherein **R6** represents C₃₋₇-cycloalkyl; preferably cyclopropyl or cyclobutyl; in each case unsubstituted or monosubstituted with NH₂;

8. The compound according to any of the preceding claims, wherein **R6** represents 4- to 7-membered heterocycloalkyl; preferably azetidinyl, pyrrolidinyl or piperidinyl; in each case unsubstituted or with 1 or 2 substituents independently from one another selected from F, CH₃, CH₂OH, CH₂OCH₃, NH₂, OH and =O;

9. The compound according to any of the preceding claims, wherein **R6** represents C₁₋₃-alkylene-(4- to 7-membered heterocycloalkyl); preferably -CH₂-azetidinyl or -CH₂-pyrrolidinyl; in each case unsubstituted or monosubstituted with F, CH₃ or CH₂CH₃;

10. The compound according to any of the preceding claims, wherein **R6** and **R7** together with the nitrogen atom to which they are attached form azetidinyl, pyrrolidinyl, morpholinyl or piperazinyl; in each case unsubstituted or substituted with 1 or 2 substituents independently from one another selected from F, CH₂OH, CH₂OCH₃, NH₂, NHCH₃, N(CH₃)₂ and =O;

11. The compound according to any of the preceding claims, wherein **R5** represents C(O)N**R**6**R7** and wherein **R4** and **R6** together with the nitrogen atom to which they are attached form a ring and mean CH₂CH₂ or CH₂CH₂CH₂.

12. The compound according to any of the preceding claims, wherein **R7** represents H or CH₃.

13. The compound according to any of the preceding claims, which is according to general formula (II), (III) or (IV) 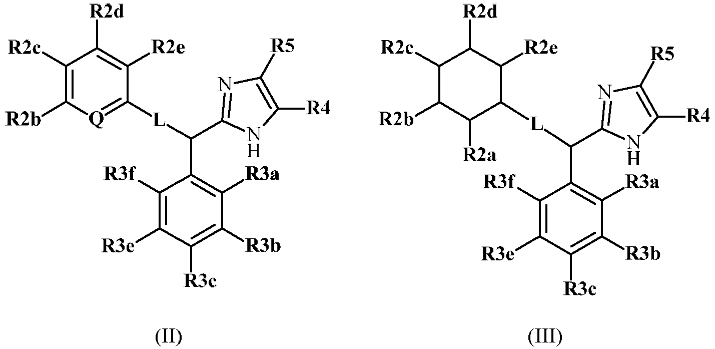 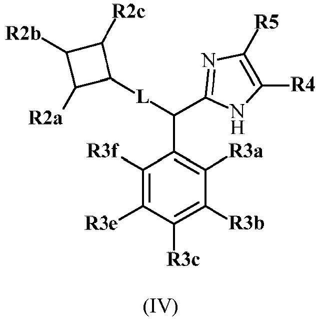 wherein
**L, R4** and **R5** are defined as in any of the preceding claims;
Q represents N or C**R2a;**
**R2a, R2b, R2c, R2d and R2e** independently from one another represent H, F, Cl, CH₃, CH₂F, CHF₂, CF₃ or OCH₃; with the proviso that not more than 4 of **R2a, R2b, R2c, R2d, and R2e** are other than H;
**R3a, R3b, R3c, R3d and R3e** independently from one another represent H, F, Cl, CH₃, CH₂F, CHF₂, CF₃ or OCH₃; with the proviso that not more than 4 of **R3a, R3b, R3c, R3d** and **R3e** are other than H.

14. The compound according to any of the preceding claims, which is selected from the group consisting of
1 1-(2-(bis(3-chloro-4-fluorophenyl)methyl)-1H-imidazol-4-yl)ethan-1-one
2 2-(bis(3-chloro-4-fluorophenyl)methyl)-1H-imidazole-4-carboxamide
3 2-(bis(3-chloro-4-fluorophenyl)methyl)-N-methyl-1H-imidazole-4-carboxamide
4 2-(bis(3-chloro-4-fluorophenyl)methyl)-N,N-dimethyl-1H-imidazole-5-carboxamide
5 2-(bis(3-chloro-4-fluorophenyl)methyl)-N-cyclopropyl-1H-imidazole-5-carboxamide
6 (2-(bis(3-chloro-4-fluorophenyl)methyl)-1H-imidazol-5-yl)(pyrrolidin-1-yl)methanone
7 (2-(bis(3-chloro-4-fluorophenyl)methyl)-1H-imidazol-5-yl)(3,3-difluoropyrrolidin-1-yl)meth-anone
8 (2-(bis(3-chloro-4-fluorophenyl)methyl)-1H-imidazol-5-yl)(morpholino)methanone
9 4-(2-(bis(3-chloro-4-fluorophenyl)methyl)-1H-imidazole-5-carbonyl)piperazin-2-one
10 2-(bis(3-chloro-4-fluorophenyl)methyl)-N-(5-oxopyrrolidin-3-yl)-1H-imidazole-5-carboxamide
11 (2-(bis(3-chloro-4-fluorophenyl)methyl)-1H-imidazol-5-yl)(4-methylpiperazin-1-yl)methanone
12 N-(2-amino-2-oxoethyl)-2-(bis(3-chloro-4-fluorophenyl)methyl)-1H-imidazole-5-carboxamide
13 2-(bis(3-chloro-4-fluorophenyl)methyl)-1,5,6,7-tetrahydro-4H-imidazo[4,5-c]pyridin-4-one
14 2-(bis(3-chloro-4-fluorophenyl)methyl)-5,6,7,8-tetrahydroimidazo[4,5-c]azepin-4(1H)-one
15 2-(bis(3-chloro-4-fluorophenyl)methyl)-1,5-dihydro-4H-imidazo[4,5-c]pyridin-4-one
16 2-(bis(3-chloro-4-fluorophenyl)methyl)-5-methyl-1H-imidazole-4-carboxamide
17 2-((3-chloro-4-fluorophenyl)((5-fluoro-6-methylpyridin-2-yl)amino)methyl)-N-(4-hydroxypyr-rolidin-3-yl)-5-methyl-1H-imidazole-4-carboxamide
18 2-(3-chloro-4-fluorophenyl)((5-fluoro-6-methylpyridin-2-yl)amino)methyl)-5-methyl-1H-imidazole-4-carboxylic acid
19 methyl2-(bis(4-chloro-2-methoxyphenyl)methyl)-5-methyl-1H-imidazole-4-carboxylate
20 2-(bis(4-chloro-2-methoxyphenyl)methyl)-5-methyl-1H-imidazole-4-carboxylic acid
21 2-(bis(4-chloro-2-methoxyphenyl)methyl)-N-(4-hydroxypyrrolidin-3-yl)-5-methyl-1H-imidazole-4-carboxamide
22 (3-aminoazetidin-1-yl)(2-((3-chloro-4-fluorophenyl)((6-(difluoromethyl)-5-fluoropyridin-2-yl)amino)methyl)-5-(methoxymethyl)-1H-imidazol-4-yl)methanone
23 (3-aminoazetidin-1-yl)(2-((3-chloro-4-fluorophenyl)((5-fluoro-6-methylpyridin-2-yl)amino)me-thyl)-5-(methoxymethyl)-1H-imidazol-4-yl)methanone
24 (3-aminoazetidin-1-yl)(2-((3-chlorophenyl)((6-(difluoromethyl)-5-fluoropyiidin-2-yl)amino)me-thyl)-5-(methoxymethyl)-1H-imidazol-4-yl)methanone
25 2-((3-chlorophenyl)((6-(difluoromethyl)-5-fluoropyridin-2-yl)amino)methyl)-N-(4-hydroxypyrrolidin-3-yl)-5-(methoxymethyl)-1H-imidazole-4-carboxamide
26 N-(3-aminopropyl)-2-((3-chloro-4-fluorophenyl)((5-fluoro-6-methylpyridin-2-yl)amino)methyl)-5-methyl-1H-imidazole-4-sulfonamide
27 2-((3-chloro-4-fluorophenyl)((5-fluoro-6-methylpyridin-2-yl)amino)methyl)-N-((3-fluoroazetidin-3-yl)methyl)-5-methyl-1H-imidazole-4-sulfonamide
28 2-((3-chloro-4-fluorophenyl)((5-fluoro-6-methylpyridin-2-yl)amino)methyl)-N-(2-(dimethylamino)ethyl)-5-methyl-1H-imidazole-4-sulfonamide
29 N-(azetidin-3-ylmethyl)-2-((3-chloro-4-fluorophenyl)((trans)-3-(trifluoromethyl)cyclobutoxy)methyl)-5-methyl-1H-imidazole-4-sulfonamide
30 2-((3-chloro-4-fluorophenyl)((5-fluoro-6-methoxypyridin-2-yl)amino)methyl)-5-methyl-1H-imidazole-4-carboxylic acid
31 2-((3-chloro-4-fluorophenyl)(((trans)-4-(trifluoromethyl)cyclohexyl)oxy)methyl)-5-methyl-1H-imidazole-4-carboxylic acid
32 2-((3-chloro-4-fluorophenyl)(((trans)-4-(trifluoromethyl)cyclohexyl)oxy)methyl)-N-(4-hydroxy-pyrrolidin-3-yl)-5-methyl-1H-imidazole-4-carboxamide
33 N-((4-((azetidin-3-ylmethyl)sulfonyl)-5-methyl-1H-imidazol-2-yl)(3-chloro-4-fluorophenyl)methyl)-5-fluoro-6-methylpyridin-2-amine
34 2-(bis(4-chloro-2-methylphenyl)methyl)-5-methyl-1H-imidazole-4-carboxylic acid
35 2-((3-chloro-4-fluorophenyl)((5-fluoro-6-methylpyridin-2-yl)amino)methyl)-5-(methoxymethyl)-1H-imidazole-4-carboxylic acid
36 2-((3-chloro-4-fluorophenyl)((6-(difluoromethyl)-5-fluoropyridin-2-yl)amino)methyl)-5-(methoxymethyl)-1H-imidazole-4-carboxylic acid
37 methyl 2-((3-chloro-4-fluorophenyl)((5-fluoro-6-methylpyridin-2-yl)amino)methyl)-4-methyl-1H-imidazole-5-carboxylate
38 2-((3-chloro-4-fluorophenyl)((5-fluoro-6-methoxypyridin-2-yl)amino)methyl)-5-(methoxymethyl)-1H-imidazole-4-carboxylic acid
39 2-(((6-(difluoromethyl)-5-fluoropyridin-2-yl)amino)(3,4-difluorophenyl)methyl)-5-(methoxymethyl)-1H-imidazole-4-carboxylic acid
40 2-(bis(3-chloro-4-fluorophenyl)methyl)-5-methyl-1H-imidazole-4-carboxylic acid
41 methyl 2-(bis(3-chloro-4-fluorophenyl)methyl)-4-methyl-1H-imidazole-5-carboxylate
42 2-((3,4-difluorophenyl)((5-fluoro-6-methylpyridin-2-yl)amino)methyl)-5-methyl-1H-imidazole-4-carboxylic acid
43 methyl 2-((3-chlorophenyl)((6-(difluoromethyl)-5-fluoropyridin-2-yl)amino)methyl)-5-(methox-ymethyl)-1H-imidazole-4-carboxylate
44 2-((3,4-dichlorophenyl)((6-(difluoromethyl)-5-fluoropyridin-2-yl)amino)methyl)-5-methyl-1H-imidazole-4-carboxylic acid
45 2-((3-chlorophenyl)((5-fluoro-6-methylpyridin-2-yl)amino)methyl)-5-methyl-1H-imidazole-4-carboxylic acid
46 2-((3-chloro-4-fluorophenyl)((6-(difluoromethyl)-5-fluoropyridin-2-yl)amino)methyl)-5-methyl-1H-imidazole-4-carboxylic acid
47 2-((3-chlorophenyl)((6-(difluoromethyl)-5-fluoropyridin-2-yl)amino)methyl)-5-(methoxymethyl)-1H-imidazole-4-carboxylic acid
48 methyl 2-(bis(4-chloro-2-methoxyphenyl)methyl)-5-(methoxymethyl)-1H-imidazole-4-carbox-ylate
49 2-((3-chloro-4-fluorophenyl)((4-(trifluoromethyl)cyclohexyl)oxy)methyl)-5-(methoxymethyl)-1H-imidazole-4-carboxylic acid
50 2-(bis(4-chloro-2-methoxyphenyl)methyl)-5-(methoxymethyl)-1H-imidazole-4-carboxylic acid
51 2-(bis(4,5-difluoro-2-methoxyphenyl)methyl)-5-methyl-1H-imidazole-4-carboxylic acid
52 2-(bis(4,5-difluoro-2-methylphenyl)methyl)-5-methyl-1H-imidazole-4-carboxylic acid
53 2-(bis(4-fluoro-2-methylphenyl)methyl)-5-methyl-1H-imidazole-4-carboxylic acid
54 2-((3-chloro-4-fluorophenyl)((5-fluoro-6-methylpyridin-2-yl)amino)methyl)-4-(hydroxymethyl)-N-(1-methylazetidin-3-yl)-1H-imidazole-5-carboxamide
55 2-((3-chloro-4-fluorophenyl)((5-fluoro-6-methylpyridin-2-yl)amino)methyl)-5-(methoxymethyl)-N-(1-methylazetidin-3-yl)-1H-imidazole-4-carboxamide
56 (2-((3-chloro-4-fluorophenyl)((5-fluoro-6-methylpyridin-2-yl)amino)methyl)-5-methyl-1H-im-idazol-4-yl)(2-(hydroxymethyl)piperazin-1-yl)methanone
57 (3,6-diazabicyclo[3.1.1]heptan-3-yl)(2-((3-chloro-4-fluorophenyl)((5-fluoro-6-methylpyridin-2-yl)amino)methyl)-5-methyl-1H-imidazol-4-yl)methanone
58 (2-((3-chloro-4-fluorophenyl)((5-fluoro-6-methylpyridin-2-yl)amino)methyl)-5-methyl-1H-imidazol-4-yl)(2,6-diazaspiro[3.3]heptan-2-yl)methanone
59 2-((3-chloro-4-fluorophenyl)((5-fluoro-6-methylpyridin-2-yl)amino)methyl)-N-(4-hydroxypyrrolidin-3-yl)-5-methyl-1H-imidazole-4-carboxamide
60 (3-aminoazetidin-1-yl)(2-((3-chloro-4-fluorophenyl)((5-fluoro-6-methylpyridin-2-yl)amino)me-thyl)-5-methyl-1H-imidazol-4-yl)methanone
61 (3-aminopyrrolidin-1-yl)(2-((3-chloro-4-fluorophenyl)((5-fluoro-6-methylpyridin-2-yl)amino)methyl)-5-methyl-1H-imidazol-4-yl)methanone
62 (3-amino-3-(methoxymethyl)azetidin-1-yl)(2-((3-chloro-4-fluorophenyl)((5-fluoro-6-methylpyr-idin-2-yl)amino)methyl)-4-methyl-1H-imidazol-5-yl)methanone
63 (2-((3-chloro-4-fluorophenyl)((5-fluoro-6-methylpyridin-2-yl)amino)methyl)-5-methyl-1H-im-idazol-4-yl)(3-(hydroxymethyl)piperazin-1-yl)methanone
64 (2-((3-chloro-4-fluorophenyl)((5-fluoro-6-methylpyridin-2-yl)amino)methyl)-5-methyl-1H-im-idazol-4-yl)(piperazin-1-yl)methanone
65 2-((3-chloro-4-fluorophenyl)((5-fluoro-6-methylpyridin-2-yl)amino)methyl)-N-(3-(hydroxymethyl)pyrrolidin-3-yl)-5-methyl-1H-imidazole-4-carboxamide
66 2-((3-chloro-4-fluorophenyl)((5-fluoro-6-methylpyridin-2-yl)amino)methyl)-N-(3-(methoxymethyl)azetidin-3-yl)-4-methyl-1H-imidazole-5-carboxamide
67 (3-amino-3-(hydroxymethyl)azetidin-1-yl)(2-((3-chloro-4-fluorophenyl)((5-fluoro-6-methylpyri-din-2-yl)amino)methyl)-5-methyl-1H-imidazol-4-yl)methanone
68 (3-amino-3-(hydroxymethyl)pyrrolidin-1-yl)(2-((3-chloro-4-fluorophenyl)((5-fluoro-6-methylpyridin-2-yl)amino)methyl)-5-methyl-1H-imidazol-4-yl)methanone
69 (3-aminopyrrolidin-1-yl)(2-((3-chloro-4-fluorophenyl)((5-fluoro-6-methylpyridin-2-yl)amino)methyl)-5-(methoxymethyl)-1H-imidazol-4-yl)methanone
70 (2,2-bis(hydroxymethyl)piperazin-1-yl)(2-((3-chloro-4-fluorophenyl)((5-fluoro-6-methylpyridin-2-yl)amino)methyl)-5-methyl-1H-imidazol-4-yl)methanone
71 (2-((3-chloro-4-fluorophenyl)((5-fluoro-6-methylpyridin-2-yl)amino)methyl)-5-(methoxyme-thyl)-1H-imidazol-4-yl)(3-(dimethylamino)azetidin-1-yl)methanone
72 (2-((3-chloro-4-fluorophenyl)((5-fluoro-6-methylpyridin-2-yl)amino)methyl)-4-(methoxyme-thyl)-1H-imidazol-5-yl)(3-(methylamino)azetidin-1-yl)methanone
73 2-((3-chloro-4-fluorophenyl)((5-fluoro-6-methylpyridin-2-yl)amino)methyl)-N-(4-hydroxypyrrolidin-3-yl)-5-(methoxymethyl)-1H-imidazole-4-carboxamide
74 (3-aminoazetidin-1-yl)(2-(((6-(difluoromethyl)-5-fluoropyridin-2-yl)amino)(3,4-difluoro-phenyl)methyl)-5-(methoxymethyl)-1H-imidazol-4-yl)methanone
76 N-(3-aminocyclobutyl)-2-((3-chloro-4-fluorophenyl)((5-fluoro-6-methylpyridin-2-yl)amino)methyl)-4-methyl-1H-imidazole-5-sulfonamide
77 N-((5-((3-aminoazetidin-1-yl)sulfonyl)-4-methyl-1H-imidazol-2-yl)(3-chloro-4-fluoro-phenyl)methyl)-5-fluoro-6-methylpyridin-2-amine
78 2-((3-chloro-4-fluorophenyl)((5-fluoro-6-methylpyridin-2-yl)amino)methyl)-N-(3-(hydroxymethyl)azetidin-3-yl)-5-methyl-1H-imidazole-4-carboxamide
79 N-(3-amino-2,2-difluoropropyl)-2-((3-chloro-4-fluorophenyl)((5-fluoro-6-methylpyridin-2-yl)amino)methyl)-4-methyl-1H-imidazole-5-sulfonamide
80 2-((3-chloro-4-fluorophenyl)((5-fluoro-6-methylpyridin-2-yl)amino)methyl)-4-methyl-N-(pyrrolidin-2-ylmethyl)-1H-imidazole-5-sulfonamide
81 2-((3-chloro-4-fluorophenyl)((5-fluoro-6-methylpyridin-2-yl)amino)methyl)-N-(3-(dimethylamino)propyl)-4-methyl-1H-imidazole-5-sulfonamide
82 N-(azetidin-3-ylmethyl)-2-((3-chloro-4-fluorophenyl)((5-fluoro-6-methylpyridin-2-yl)amino)methyl)-5-(methoxymethyl)-1H-imidazole-4-carboxamide
83 2-((3-chloro-4-fluorophenyl)((5-fluoro-6-methylpyridin-2-yl)amino)methyl)-4-methyl-N-(piperidin-3-yl)-1H-imidazole-5-sulfonamide
84 2-((3-chloro-4-fluorophenyl)((5-fluoro-6-methylpyridin-2-yl)amino)methyl)-4-methyl-N-(1-methylazetidin-3-yl)-1H-imidazole-5-sulfonamide
85 2-((3-chloro-4-fluorophenyl)((5-fluoro-6-methylpyridin-2-yl)amino)methyl)-4-methyl-N-(2-(1-methylpyrrolidin-2-yl)ethyl)-1H-imidazole-5-sulfonamide
86 2-((3-chloro-4-fluorophenyl)((5-fluoro-6-methylpyridin-2-yl)amino)methyl)-4-methyl-N-(2-(methylamino)ethyl)-1H-imidazole-5-sulfonamide
87 2-((3-chloro-4-fluorophenyl)((4-(trifluoromethyl)cyclohexyl)oxy)methyl)-N-(4-hydroxy-1-methylpyrrolidin-3-yl)-5-(methoxymethyl)-1H-imidazole-4-carboxamide
88 2-((3-chloro-4-fluorophenyl)((5-fluoro-6-methylpyridin-2-yl)amino)methyl)-N-(4-hydroxypyrrolidin-3-yl)-4-methyl-1H-imidazole-5-sulfonamide
89 2-((3-chloro-4-fluorophenyl)((5-fluoro-6-methylpyridin-2-yl)amino)methyl)-4-methyl-N-(pyrrolidin-3-yl)-1H-imidazole-5-sulfonamide
91 N-(azetidin-2-ylmethyl)-2-((3-chloro-4-fluorophenyl)((5-fluoro-6-methylpyridin-2-yl)amino)methyl)-4-methyl-1H-imidazole-5-sulfonamide
92 N-(2-aminoethyl)-2-((3-chloro-4-fluorophenyl)((5-fluoro-6-methylpyridin-2-yl)amino)methyl)-4-methyl-1H-imidazole-5-sulfonamide
93 2-((3-chloro-4-fluorophenyl)((5-fluoro-6-methylpyridin-2-yl)amino)methyl)-4-methyl-N-((1-methylazetidin-3-yl)methyl)-1H-imidazole-5-sulfonamide
94 2-((3-chloro-4-fluorophenyl)((5-fluoro-6-methylpyridin-2-yl)amino)methyl)-N-(4-fluoropyrroli-din-3-yl)-5-(methoxymethyl)-1H-imidazole-4-carboxamide
95 (3-aminoazetidin-1-yl)(2-((3-chloro-4-fluorophenyl)((5-fluoro-6-methoxypyridin-2-yl)amino)methyl)-5-(methoxymethyl)-1H-imidazol-4-yl)methanone
96 N-(azetidin-3-yl)-2-((3-chloro-4-fluorophenyl)((5-fluoro-6-methylpyridin-2-yl)amino)methyl)-4-methyl-1H-imidazole-5-sulfonamide
97 2-((3-chloro-4-fluorophenyl)((5-fluoro-6-methylpyridin-2-yl)amino)methyl)-N-((1-ethylpyrrolidin-2-yl)methyl)-4-methyl-1H-imidazole-5-sulfonamide
98 2-((3-chloro-4-fluorophenyl)((4-(trifluoromethyl)cyclohexyl)oxy)methyl)-N-(4-hydroxypyrrolidin-3-yl)-5-(methoxymethyl)-1H-imidazole-4-carboxamide
99 N-(azetidin-3-ylmethyl)-2-((3-chloro-4-fluorophenyl)((4-(trifluoromethyl)cyclohexyl)oxy)methyl)-5-(methoxymethyl)-1H-imidazole-4-carboxamide
100 2-((3-chloro-4-fluorophenyl)((5-fluoro-6-methylpyridin-2-yl)amino)methyl)-4-methyl-N-((1-methylazetidin-2-yl)methyl)-1H-imidazole-5-sulfonamide
101 2-((3-chloro-4-fluorophenyl)((5-fluoro-6-methylpyridin-2-yl)amino)methyl)-N-(4-fluoro-1-methylpyrrolidin-3-yl)-5-(methoxymethyl)-1H-imidazole-4-carboxamide
102 N-(azetidin-3-ylmethyl)-2-((3-chloro-4-fluorophenyl)((5-fluoro-6-methylpyridin-2-yl)amino)methyl)-5-methyl-1H-imidazole-4-carboxamide
103 2-((3-chloro-4-fluorophenyl)((5-fluoro-6-methylpyridin-2-yl)amino)methyl)-N-(4-fluoropyrrolidin-3-yl)-5-methyl-1H-imidazole-4-carboxamide
and the physiologically acceptable salts thereof.

15. A medicament comprising a compound according to any of the preceding claims.
